# EUROPÄISCHE PATENTANMELDUNG

(11) **EP 1 176 152 A2**
(43) Veröffentlichungstag der Anmeldung: **30.01.2002**
(21) Anmeldenummer: 01116617.0
(22) Anmeldetag: 12.07.2001
(51) Int. Cl.: C07K 14/435, G01N 33/68

(54) **Ligandenbindedomäne des Ultraspiracle (USP)-Proteins**

(30) Priorität: 25.07.2000 DE 10036461
(71) Anmelder: BAYER AG, 51368 Leverkusen (DE)
(72) Erfinder: Franken, Eva-Maria, Dr., 42799 Leichlingen (DE); Janssen, Martina, Dr., 53639 Königswinter (DE); Schindler, Michael, Prof. Dr., 51467 Bergisch Gladbach (DE); Tietjen, Klaus, Dr., 40764 Langenfeld (DE); Moras, Dino, 67450 Lampertingen (FR); Wurtz, Jean-Marie, 67410 Drusenheim (FR); Rochel-Guibertau, Natacha, 67000 Strasbourg (FR); Billas-Massobrio, Isabelle, 67000 Strasbourg (FR)

(57) **Zusammenfassung**

Die Erfindung betrifft die räumliche Struktur der Ligandenbindedomäne des Ultraspiracle-Proteins, die Verwendung dieser Struktur zur Erzeugung von Proteinmodellen dieses Proteins in verschiedenen Konformationen und verwandter Proteine, sowie Verfahren zum Auffinden von Liganden des Ultraspriracle-Proteins und verwandter Proteine.

## Beschreibung

Die Erfindung betrifft die räumliche Struktur der Ligandenbindedomäne des Ultraspiracle-Proteins, die Verwendung dieser Struktur zur Erzeugung von Proteinmodellen dieses Proteins in verschiedenen Konformationen und verwandter Proteine, sowie Verfahren zum Auffinden von Liganden des Ultraspriracle Proteins und verwandter Proteine.

Das Ultraspiracle-Protein (im Folgenden als USP bezeichnet) ist das Insektenorthologe des Vertebraten Retinoid X Rezeptors (RXR). Wie RXR gehört es zur Familie der Kernrezeptoren (Nuclear Receptors, NR). Diese Kernrezeptoren befinden sich im Zellinneren. Sie binden als Homo- oder Heterodimere an responsive Elemente auf der DNA und regulieren die Expression von Genen. Um aktiv zu sein, müssen sie spezielle kleine, oft hydrophobe Liganden (z.B. Steroide, Retinoide, Vitamin D) binden. Kernrezeptoren besitzen eine modulare Struktur mit funktionellen Domänen für Transaktivierung, DNA-Bindung und Ligandenbindung. Während die DNA-Bindedomäne der Kernrezeptoren hoch konserviert ist, zeigen die Ligandenbindedomänen nur moderate Homologien untereinander. Die räumlichen Strukturen verschiedener Ligandenbindedomänen wurden bereits bestimmt (Zusammenfassung in 2) und geben einen Einblick in den der Aktivierung zugrunde liegenden Mechanismus, der starke Konformationsänderungen der Ligandenbindedomänen beinhaltet. Die Bindung von Agonisten führt über die Verdrängung gebundener Co-Repressoren und Bindung von Co-Aktivatoren zur Aktivierung, während die Bindung von Antagonisten die Wechselwirkung mit dem Co-Aktivator verhindert.

Von Kernrezeptoren aus Insekten liegen noch keine räumlichen Strukturen vor. In Insekten wird z.B. die Entwicklung von der Larve zum adulten Insekt über Kernrezeptoren unter Beteiligung des Steroidhormons Ecdyson sowie des Isoprenoids Juvenilhormon gesteuert (3, 4, 5, 6). Eine Schlüsselrolle spielt hierbei der Ecdysonrezeptor, ein Kernrezeptor, der aus zwei verschiedenen Untereinheiten, EcR und USP, zusammengesetzt ist (7, 8, 9). Seit langem ist das Hormon Ecdyson (in seiner aktiven Form 20-Hydroxyecdyson) als Ligand für die EcR-Untereinheit bekannt.

Der Ecdysonrezeptor stellt ein wichtiges Insektizid-Target dar. Wird er außerhalb des in der Insektenentwicklung dafür vorgesehenen zeitlichen Fensters aktiviert, so führt dies zu schweren Störungen bis hin zum Absterben des Insekts. Auf diesem Mechanismus beruht die insektizide Wirkung von Ecdysonagonisten (10, 11). Nichtsteroidale Liganden der EcR-Untereinheit, die spezifisch auf Lepidopteren wirken, finden bereits kommerzielle Anwendung als Insektizide (12).

Bei USP handelt es sich um einen Orphanrezeptor, für den bisher kein Ligand bekannt ist. Zwar wurde verschiedentlich vermutet, dass USP einen Rezeptor für Juvenilhormone darstellt, doch wurde dafür nie ein wirklicher experimenteller Beleg gefunden (9). Es wurde sogar vermutet, dass USP überhaupt keinen Liganden besitzt, wie dies für einige andere aus Tieren bekannte Kernrezeptoren beschrieben ist.

Aufgabe der vorliegenden Erfindung war es daher, die räumliche Struktur der Ligandenbindedomäne (im Folgenden als LBD bezeichnet) des USP zur Verfügung zu stellen und die mögliche Ligandenbindetasche zu beschreiben.

Die Aufgabe wurde gelöst durch die Bereitstellung einer USP-LBD in kristalliner Form und die erfolgreiche Durchführung der Röntgenstrukturanalyse der so erhaltenen Kristalle.

Bei der erfindungsgemäßen kristallinen LBD handelt es sich bevorzugt um eine LBD des USP von Heliothis virescens. Besonders bevorzugt weist die erfindungsgemäße LBD eine Aminosäuresequenz gemäß SEQ ID NO: 1 auf.

Gegenstand der vorliegenden Erfindung ist auch ein kristalliner Komplex einer USP-LBD mit einem Liganden.

Die erfindungsgemäße LBD weist vorzugsweise die in Tabelle 1 definierten Strukturkoordinaten auf. Die dreidimensionale Struktur wurde mit Hilfe von Proteinkristallen, die einer Röntgenstrukturanalyse zugänglich sind, bei hoher Auflösung mittels molekularem Ersatz gelöst und vollständig verfeinert. Gegenstand der vorliegenden Erfindung ist somit auch die anhand dieser Strukturkoordinaten ermittelbare dreidimensionale Struktur der USP-LBD.

In der hierin beschriebenen erfindungsgemäßen dreidimensionalen Struktur der USP-LBD wurde eine Ligandenbindetasche identifiziert, in welche - wie in den anderen bekannten Strukturen von Kernrezeptoren - die Liganden binden. Dieses ist der erste wirkliche Beweis dafür, dass USP eine funktionelle Ligandenbindetasche besitzt.

Gegenstand der vorliegenden Erfindung ist weiterhin eine USP-LBD, die eine Ligandenbindungstasche, welche durch die Aminosäuren LEU230, VAL238, PRO239, PHE242, LEU249, LEU291, ILE294, MET323, LEU331, GLN338, ALA339, VAL341, PHE345, SER431, HIS434, LEU435, PHE438 und LEU440 gemäß SEQ ID NO: 2 und Tabelle 1 definiert ist, umfasst.

Weiterhin ist eine USP-LBD Gegenstand der vorliegenden Erfindung, die eine Ligandenbindungstasche, welche durch die Aminosäuren LEU230, VAL238, PRO239, PHE242, PRO245, VAL246, LEU249, CYS250, GLY253, ASN287, LEU290, LEU291, ILE294, MET323, LEU325, LEU331, SER335, ALA336, GLN338, ALA339, VAL341, ILE344, PHE345, VAL348, SER431, HIS434, LEU435, PHE438 und LEU440 gemäß SEQ ID NO: 2 und Tabelle 1 definiert ist, umfasst.

Weiterhin ist eine USP-LBD Gegenstand der vorliegenden Erfindung, die eine Ligandenbindungstasche umfasst, die durch die oben beschriebenen Aminosäuren definiert ist, und in der eine oder mehrere dieser Aminosäuren mutiert sind. Vorzugsweise handelt es sich dabei um konservative Mutationen, in der ein Austausch durch eine Aminosäure mit ähnlichen physikalischen Eigenschaften erfolgt.

Solche konservativen Substitutionen umfassen Variationen, bei denen eine Aminosäure durch eine andere Aminosäure aus den folgenden Gruppen ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, His |
| Met | Leu, Tyr, Ile |
| Phe | Met, Leu, Tyr |
| Ser | Thr |
| Thr | Ser |
| Trp | Tyr, Phe |
| Tyr | Trp, Phe |
| Val | Ile, Leu |

Die hierin beschriebene dreidimensionale Struktur einer USP-LBD ist von großer Bedeutung für die Suche nach Liganden mit praktischer Nutzanwendung. Solche Liganden können z.B. als Insektizide mit neuem Wirkmechanismus Verwendung finden. Die Ecdyson/Juvenilhormon gesteuerte Entwicklung ist nur bei Invertebraten zu finden und kommt in Vertebraten nicht vor; sie stellt also einen für den Anwender und die Umwelt sicheren insektiziden Mechanismus dar.

Unter Einsatz der erfindungsgemäßen dreidimensionalen Struktur der USP-LBD können mit Hilfe etablierter automatisierter Computerprotokolle Datenbanken durchgemustert werden, die die Strukturen einer großen Zahl von Verbindungen enthalten (Virtual Screening). Für das virtuelle Screenen können zum Beispiel Algorithmen wie FLEXX (13) oder GOLD (14) verwendet werden. Durch diese Vorgehensweise können Verbindungen identifiziert werden, deren dreidimensionale Struktur es ermöglicht, in die Bindetasche zu gelangen und dort zu binden, beispielsweise durch Bildung von Wasserstoffbrücken, durch hydrophobe Wechselwirkung, durch elektrostatische Wechselwirkungen, durch van-der-Waals-Wechselwirkungen oder durch Dipol-Wechselwirkungen. Die so identifizierten Verbindungen können synthetisiert werden und dann z.B. als Insektizide oder als Effektoren in Expressionssystemen (Gene Switch) auf der Basis des USP Verwendung finden.

Eine weitere Anwendung der erfindungsgemäßen dreidimensionalen Struktur der USP-LBD liegt in der Generierung neuer Liganden. Hierzu werden unter Einsatz der Struktur und mit Hilfe etablierter de novo Designprogramme am Computer Strukturformeln für neue Liganden generiert, die in die Bindetasche gelangen können und dort binden können, beispielsweise durch Bildung von Wasserstoffbrücken, durch hydrophobe Wechselwirkung, durch elektrostatische Wechselwirkungen, durch van-der-Waals-Wechselwirkungen oder durch Dipol-Wechselwirkungen. Beispiele für mögliche de novo Designprogramme sind LUDI (15), LEGEND (16) oder GROW (17). Solcherart generierte Verbindungen können synthetisiert und dann ebenfalls z.B. als Insektizide oder als Effektoren in Expressionssystemen (Gene Switch) auf der Basis des USP genutzt werden.

Die erfindungsgemäße dreidimensionale Struktur der USP-LBD ermöglich es auch, die dreidimensionale Struktur einer USP-LBD aus anderen Organismen durch Modelling-Methoden vorherzusagen. Solche Proteinmodelle können in der gleichen Weise verwendet werden, wie die hier gelöste dreidimensionale Struktur. Durch Vergleich der Unterschiede in den Aminosäuresequenzen ist es möglich, Unterschiede in den Ligandenbindetaschen verschiedener Organismen vorherzusagen. Dies ist nützlich, wenn für spezifische Organismen spezifische Liganden gesucht werden, oder wenn im umgekehrten Fall gerade unspezifische Liganden gesucht werden. Darüberhinaus kann die erfindungsgemäße dreidimensionale Struktur zur Erstellung von Proteinmodellen anderer in der Sequenz verwandter Kernrezeptoren dienen.

Insbesondere sind die folgenden Gegenstände und Verfahren von der vorliegenden Erfindung umfasst:

Ein Computer-lesbares Datenspeichermedium, das ein Datenspeichermaterial umfasst, auf welchem die Strukturkoordinaten einer LBD gemäß der vorliegenden Erfindung gespeichert sind.

Ein Computer-lesbares Datenspeichermedium in einer Form, die es ermöglicht, eine dreidimensionale Abbildung einer LBD gemäß der vorliegenden Erfindung auf einem Computer-Bildschirm zu erzeugen.

Ein Verfahren zum Erstellen von Proteinmodellen von USP-LBDs, gekennzeichnet durch Computer-unterstütztes Erstellen einer dreidimensionalen Abbildung einer LBD gemäß der vorliegenden Erfindung.

Ein Verfahren zum Erstellen von Proteinmodellen von USP-LBDs in einer agonistischen Konformation, gekennzeichnet durch Computer-unterstütztes Erstellen einer dreidimensionalen Abbildung einer LBD gemäß der vorliegenden Erfindung in einer agonistischen Konformation.

Ein Verfahren zum Erstellen von Proteinmodellen von Kernrezeptoren, die Homologien zu USP-LBDs aufweisen, gekennzeichnet durch Computer-unterstütztes Erstellen einer dreidimensionalen Abbildung einer LBD gemäß der vorliegenden Erfindung mit einer mutierten Aminosäuresequenz.

Ein Verfahren zum Erstellen von Proteinmodellen von Kernrezeptoren, die Homologien zu USP-LBDs aufweisen, in einer agonistischen Konformation, gekennzeichnet durch Computer-unterstütztes Erstellen einer dreidimensionalen Abbildung einer LBD gemäß der vorliegenden Erfindung mit einer mutierten Aminosäuresequenz in einer agonistischen Konformation.

Ein Verfahren zum Auffinden von Liganden des USP, gekennzeichnet durch die folgenden Schritte:
(a) Computer-unterstütztes Erstellen einer dreidimensionalen Abbildung einer LBD gemäß der vorliegenden Erfindung, und
(b) Computer-unterstütztes Durchsuchen (virtuelles Screenen) von Datenbanken, die Strukturdaten von chemischen Verbindungen enthalten, nach solchen Strukturen, die die Fähigkeit besitzen, spezifische Wechselwirkungen mit einer LBD gemäß der vorliegenden Erfindung einzugehen.

Ein Verfahren zum Auffinden von Liganden des USP, gekennzeichnet durch die folgenden Schritte:
(a) Computer-unterstütztes Erstellen einer dreidimensionalen Abbildung einer LBD gemäß der vorliegenden Erfindung, und
(b) Computer-unterstütztes Modellieren von chemischen Verbindungen mit Strukturen, die die Fähigkeit besitzen, spezifische Wechselwirkungen mit einer LBD gemäß der vorliegenden Erfindung einzugehen.

Ein Verfahren zum Auffinden von Liganden von USP-LBDs in einer agonistischen Konformation, gekennzeichnet durch die folgenden Schritte:
(a) Computer-unterstütztes Erstellen einer dreidimensionalen Abbildung einer LBD gemäß der vorliegenden Erfindung in einer agonistischen Konformation, und
(b) Computer-unterstütztes Durchsuchen (virtuelles Screenen) von Datenbanken, die Strukturdaten von chemischen Verbindungen enthalten, nach solchen Strukturen, die die Fähigkeit besitzen, spezifische Wechselwirkungen mit einer LBD in einer agonistischen Konformation einzugehen.

Ein Verfahren zum Auffinden von Liganden von USP-LBDs in einer agonistischen Konformation, gekennzeichnet durch die folgenden Schritte:
(a) Computer-unterstütztes Erstellen einer dreidimensionalen Abbildung einer LBD gemäß der vorliegenden Erfindung in einer agonistischen Konformation, und
(b) Computer-unterstütztes Modellieren von chemischen Verbindungen mit Strukturen, die die Fähigkeit besitzen, spezifische Wechselwirkungen mit einer LBD in einer agonistischen Konformation einzugehen.

Ein Verfahren zum Auffinden von Wirkstoffen für den Pflanzenschutz, insbesondere von chemischen Verbindungen, welche durch Bindung an eine LBD gemäß der vorliegenden Erfindung zur Aktivierung oder Hemmung von USP führen, umfassend die folgenden Schritte:
(a) Durchführen eines oben genannten Verfahren zum Auffinden von Liganden des USP,
(b) Synthetisieren der als Liganden identifizierten Verbindung(en), und
(c) Detektieren der biologischen Aktivität der im Schritt (b) synthetisierten Verbindung durch Transaktivierungstests, Verdrängungstests oder Biotests.

Ein Verfahren zum Auffinden von Wirkstoffen für den Pflanzenschutz, insbesondere von chemischen Verbindungen, welche durch Bindung an eine LBD gemäß der vorliegenden Erfindung in einer agonistischen Konformation zur Aktivierung oder Hemmung von USP führen, umfassend die folgenden Schritte:
(a) Durchführen eines oben genannten Verfahren zum Auffinden von Liganden von USP-LBDs in einer agonistischen Konformation,
(b) Synthetisieren der als Liganden identifizierten Verbindung(en), und
(c) Detektieren der biologischen Aktivität der im Schritt (b) synthetisierten Verbindung durch Transaktivierungstests, Verdrängungstests oder Biotests.

Ein Verfahren zum Auffinden von Effektoren für Systeme zur induzierbaren Expression von Zielgenen mittels USP, umfassend die folgenden Schritte:
(a) Durchführen eines oben genannten Verfahren zum Auffinden von Liganden des USP,
(b) Synthetisieren der als Liganden identifizierten Verbindung(en),
(c) Applizieren einer im Schritt (b) synthetisierten Verbindung auf Wirtszellen oder Wirtsorganismen, welche ein auf USP basierendes Expressionssystem enthalten, und
(d) Detektieren einer Induktion oder Hemmung des Expressionssystems.

Die Verwendung einer LBD gemäß der vorliegenden Erfindung oder eines Computer-lesbares Datenspeichermediums gemäß der vorliegenden Erfindung zum Auffinden von Wirkstoffen für den Pflanzenschutz oder von Effektoren zur kontrollierten Expression von Zielgenen in Wirtszellen oder ganzen Wirtsorganismen.

Die vorliegende Erfindung wird anhand der folgenden Beispiele genauer beschrieben.

### Beispiele

### Beispiel 1

### Proteinexpression und Aufreinigung

Die USP-LBD von Heliothis virescens (AS Val-205 bis Met 466) wurde als N-terminales Fusionsprotein mit einem His-Tag in einem pET-15b-Expressionsvektor cloniert und im E. coli Stamm BL21(DE3) überexprimiert. Zellen wurden in 2x LB Medium bei 37°C kultiviert und 2 Stunden mit 0,8 mM Isopropyl-β-D-Thiogalactopyranosid bei 24°C induziert. Der Proteinextrakt wurde über eine Kobalt-Chelat-Säule mit nachfolgender Gelfiltration über eine Superdex 200 16/60-Säule aufgereinigt. Dann wurde der His-Tag durch Thrombinverdau entfernt und das Protein durch Gelfiltration abgetrennt. Eine homogene, monomere Proteinspezies lag in der Lösung vor und wurde mittels SDS- und nativer Polyacrylamidgelektrophorese sowie durch denaturierende und native Elektrospray-Ionisations-Massenspektrometrie bestätigt.

### Kristallisierung

Die Kristallisierung erfolgte per Gasdiffusion an hängenden Tropfen. Die eingesetzte Proteinkonzentration betrug 3-9 mg/ml. Kristalle von einer Größe von 200 x 200 x 400 mm³ bildeten sich innerhalb von 10 Tagen aus einer Lösung, die 10% Polyethylenglycol (PEG) 4000, 50 mM Tris (pH 7,5), 100 mM NaCl und 5 mM Dithiothreitol enthielt und gegen eine Lösung aus 20 % Polyethylenglycol (PEG) 4000 und 100 mM Tris (pH 7,5) im Reservoir äquilibriert wurde. Die Kristalle gehören zur tetragonalen P4₃22 Raumgruppe mit einem Monomer je asymmetrische Einheit. Die Parameter der Einheitszelle sind a=58,21 Å, b=58,21 Å, c=144,69 Å und α=β=γ=90°. Der Lösemittelgehalt liegt bei 32% und der im Wilson Plot abgeschätzte B-Faktor beträgt 27 Å².

### Datensammlung, Strukturbestimmung und Verfeinerung

Kristalle wurden kurz in eine Lösung aus 10 % Glycerin getaucht und in flüssigem Stickstoff schockgefroren. Das native Datenset wurde mit einem einzelnen Kristall erstellt an Meßstand ID14-EH2 am ESRF (Grenoble, Frankreich). Die Daten wurden mit Hilfe von HKL-Programmen (18) prozessiert. Die Kristallstruktur wurde durch die Methode des Molekularen Ersatzes (19) mittels einer partiellen hRXRα-Struktur (20) als Suchmodell gelöst. Eine schlechte Lösung wurde mit einer Korrelation von 24.8 % und R_{free}=54.5 % nach einer Verfeinerung als starrer Körper erzielt. Die Phasing Power des Modells war gering und benötigte zahlreiche manuelle Aufbauzyklen mit O (21). Die wARP Methode (22) wurde benutzt, um die Richtigkeit der partiell aufgebauten Strukturen zu überprüfen. Die Verfeinerung erfolgte mit CNS (23) unter Nutzung einer Maximum Likelihood-Zielfunktion und Solvens-Korrektur. Zyklen manueller Modellbildung und Least-square Minimierung mit darauf folgendem simulierten Annealing und individueller anisotropischer B-Faktor-Verfeinerung führten zum endgültigen Modell. Lösungsmittelmoleküle wurden in einer Fₒ - F_{c} Karte bei einer Oberfläche von 3σ konturiert. Das endgültige Modell, verfeinert bis zu einer Auflösung von 1,65 Å beinhaltet 246 Aminosäurereste, 259 Wassermoleküle und ein Ligandenmolekül. Ein großer Teil der Verbindungsschleife zwischen Helix H5 und dem Beginn des β-Faltblatts (Aminosäurereste 306-315) sowie der C-terminale Fortsatz von H12 (Aminosäurereste 459-466) konnten aufgrund der schlechten Elektronendichte in diesen Regionen nicht dargestellt werden. Die Qualität des endgültigen Modells wurde mit Procheck (24) überprüft.

Die Charakterisierung der Kristalle der USP-LBD durch "Electrospray time-of-flight mass-spectrometrie" (ESI TOF-MS) unter natürlichen Bedingungen zeigt, dass eine heterogene Massenverteilung um 740 ± 50 Da zu dem Peak des reinen Proteins (30,2 kDa) hinzukommt. Dies war ein Hinweis darauf, dass ein Ligand in der LBD gebunden vorliegt. Die Gegenwart eines Liganden wurde durch der Elektronendichte bestätigt. Zur Charakterisierung des Liganden wurden verschiedene komplementäre Techniken verwendet. Der Ligand, der sich in der Bindetasche der USP-LBD befindet, konnte als Phospholipid-Molekül charakterisiert werden. Ein Phosphatidylglycerin oder ein Phosphatidylethanolamin oder ein Phosphatidylcholin würden zu den kristallographischen Daten passen und sind konsistent mit den Ergebnissen aus Massenspektroskopie und chemischer Analyse. Diese amphiphilen Moleküle haben eine Kopfgruppe bestehend aus einer Phosphorylglycerin bzw. einer Phosphorylethanolamin Gruppe und einem Schwanz aus zwei unterschiedlichen Fettsäuren, die durch Ester-Bindungen an das Glycerin-3-phosphate gebunden sind. Eine detaillierte Beschreibung des Liganden und seiner Wechselwirkungen mit den Resten der USP-LBD werden weiter unter gegeben.

### Beispiel 2

### Architektur der USP-LBD von Heliothis virescens

Generell zeigt die Architektur der USP-LBD eine kanonische NR Faltung mit 11 α-Helices (H1, H3-H12) und zwei kurzen β-Strands (s1-s2). Diese Struktur wurde mit zwei anderen Kristallstrukturen verglichen, die wesentliche Eigenschaften von NRs besitzen und mit dem USP von Heliothis virescens eng verwandt sind: die Bindetaschen von Agonist-gebundenem RXRα (hRXRα/9-cis RA) und Antagonistgebundenem Maus-RXRα (msRXRα/Ölsäure). Die Überlagerung der USP-LBD mit der Struktur der Holo-RXRα -LBD wurde mit Hilfe eines least square Fits [Methode der kleinsten Quadrate; LSQ] vorgenommen. Insgesamt lassen sich die Sekundärstrukturelemente der USP-LBD mit denen der Holo-RXRα-LBD recht gut überlagern. Die mittlere quadratische Abweichung (root mean square deviation, r.m.s.d.) beträgt 1,22 Å für 183 von 246 überlagerten Cα-Atomen. Sieben Helices lassen sich ziemlich gut überlagern (r.m.s.d. 1,13, 0,88, 0,57, 1,18, 0,67, 0,69, 0,75 Å für H4, H5, H7-H11). Der C-Terminus von H1 ist um etwa 2 Å zur Helix H3 gebogen und sein r.m.s.d. beträgt 1,63 Å. H3, H6 und das β-Faltblatt zeigen größere Abweichungen. Die Struktur der USP-LBD zeigt, dass die Aktivierungshelix H12 eine Konformation annimmt, die ähnlich zu derjenigen des Antagonisten-RXRα ist. Die antagonistische AF-2 Konformation der USP-LBD wird weiter unten diskutiert.

Die verbindende Schleife L1-3 der meisten NRs verhält sich normalerweise wie eine sehr flexible Region. Bei hRXRα zeigen die Kristallstrukturen sowohl der Apo- als auch der Holo-Konformationen substantielle Unterschiede in den Bereichen, die die Helices H1 and H3 verbinden. In der Holo-LDB-Struktur besteht L1-3 aus einer ausgedehnten Schleife, die über das β-Faltblatt und eine Ω-Schleife hinwegreicht. Die Apo-Form enthält in diesem Bereich eine zusätzliche Helix, die sich in der Holo-Form entfaltet. Während des Übergangs von der Apo- zur Holo-Form bewegt sich L1-3 substantiell. Insbesondere orientiert sich die Ω-Schleife zur entgegengesetzten Seite des Proteinzentrums. Wie es aus dem Vergleich der beiden Strukturen vorgeschlagen wurde, könnte L1-3 als molekulare Feder wirken, die die konformationellen Änderungen begleitet, die mit der Ligandenbindung verknüpft sind. Für die ligandengebundene RARγ-LBD ist die Konformation von L1-3 ähnlich zu derjenigen der Holo-RXRα. Interessanterweise folgt L1-3 für ER-LBDs einem von der Holo-RXRα verschiedenen Weg. Er führt zwischen Helix H3 und dem β-Faltblatt, eng gepackt an das Proteinzentrum.

Bei der USP-LBD nimmt L1-3 keine der Konformationen an, die sonst bei anderen NRs gefunden werden. Sein Verlauf (Val-220 to Pro-239) wurde aus den Elektronendichtekarten unzweideutig abgeleitet. Nur wenige Reste am Beginn der Schleife, Asp-222, Pro-223 and Ser-224 wurden wegen der schlechten Elektronendichte der Seitenketten als Alanine behandelt. Daher sind die Temperaturfaktoren dieser Reste höher (60-64 Å²) als die der anderen Aminosäuren von L1-3 (36 Å² im Mittel über L1-3). Die ersten Reste von L1-3 bilden einen Pfad, der Helix H3 im Bereich von Gln-256 bis Val-262 kreuzt. Die nächsten Reste (Glu-226 to Pro-234) formen eine ausgedehnte Schleife, die entlang von H3 verläuft, und schließlich bilden die letzten fünf Reste von L1-3 (Asp-235 to Pro-239) eine Schleife, die ziemlich ähnlich zu der Ω-Schleife ist, die man in den LBDs von RXRα und RARγ beobachtet. L1-3 nimmt eine recht gespannte Konformation ein, die es ermöglicht, direkte Kontakte mit den Resten der Helices H3, H11 and H12 herzustellen und deren aktuelle Positionen zu stabilisieren. Das ist insofern wichtig, als dass diese Helices diejenigen Strukturelemente sind, die infolge der Ligandenbindung den größten konformationellen Änderungen unterworfen sind.

Die besondere Konformation von L1-3 beruht nicht auf Kristallpackungseffekten. In dem Bereich der Schleife L1-3 der RXRα-LBD, erfolgt die Wechselwirkung der USP-LBD mit seinem symmetrieäquivalenten Molekül über die β-Faltblätter. Diese Wechselwirkung findet höchstwahrscheinlich deshalb statt, weil L1-3 diesen Bereich nicht schon besetzt, wenn sich das Protein in Lösung befindet. Wenn aufgrund von Packungseffekten L1-3 gezwungen würde, zu schwingen und sich von einer Konformation wegzubewegen, die ähnlich zu der aktuellen Konformation von RXRα ist, hätten sich mehrere Sekundärstrukturelemente dramatisch von dieser hypothetischen Konformation bis zu ihrer endgültigen Position zu bewegen. Es ist sehr unwahrscheinlich, dass diese drastische Reorganisation der ganzen LBD stattfindet, besonders deshalb, weil L1-3 in einem Bereich der LBD liegt, in dem L1-3 sehr spezifische Wechselwirkungen zu benachbarten Sekundärstrukturelementen ausbildet.

Direkt mit der Schleife L1-3 verbunden, unterscheidet sich Helix H3 sowohl in der Länge als auch in der Position des N- und C-terminalen Teils von ihren Pendants in RXRα. In USP von Heliothis virescens beginnt H3 bei Pro-240 und ist dehalb eine Windung länger als H3 in der ligandengebundenen RXRα (Start bei RXRα-Pro-264). Die Reste von H3 im Mittelteil der Helix nehmen fast identische Positionen ein, verglichen mit den Positionen der entsprechenden Reste in den Apo- und Holo-RXRα-LBDs. Allerdings sind sowohl N- als auch C-terminale Bereiche zur Außenseite des Proteinzentrums gebogen. Der N-terminale Bereich von H3 (Pro-240 to Cys-250) ist substantiell in Richtung von H11 verschoben. Er ist um etwa 24° im Vergleich zum gleichen Bereich in der Holo-RXRα gekippt (etwa 7.2 Å zwischen USP-Pro-245 und Holo-RXRα-Pro-264). Diese Position liegt zwischen denen der N-terminal Regionen in der Apo-RXRα und der Holo-RXRα-LBD-Struktur. Der nach außen gebogene C-Terminus von H3 (um ca. 10°) hat Auswirkungen auf die Anordnung der benachbarten Schleifen L3-4 und L8-9. Die Schleife L3-4, die ein Teil des Signaturbereich von NRs ist, wird um etwa 1,8 Å lateral verschoben und in die Richtung von L8-9 gebogen, die Schleife L8-9 selbst wird um etwa 1,5 Å nach außen verschoben.

### Beispiel 3

### Die Ligandenbindetasche

Die Ligandenbindetasche des USP von Heliothis virescens wird von Resten aus der Schleife L1-3, den Helices H3, H5, H6 und H7, dem β-Faltblatt und der Schleife L11-12 gebildet. Wie oben beschrieben, ist der N-terminale Teil der Helix H3, verglichen mit seinem Gegenstück in RXRα, deutlich nach außen verschoben. Auch zwei andere Sekundärstrukturen, die zur Bindetasche beitragen, unterscheiden sich von denen in RXRα: 1) Helix 6 hat sich um etwa 1,9 Ä nach innen bewegt, und 2) die Krümmung des β-Faltblatts zeigt auf H1. Die damit einhergehende Verschiebung der drei Strukturelemente führt zu einer Aufweitung der Ligandenbindetasche verglichen mit derjenigen der RXRα-LBD. Der Rand der Bindetasche wird von der Ω-Schleife von L1-3, dem N-Terminus von H3 und H6 gebildet, wohingegen bei RXRα die Schleife L11-12 und H6 den Eingang der Tasche bilden. Die Bindetasche ist an ihrem Eingang etwa 13,5 Å weit (Abstand zwischen Lys-241 in H3 und Gln-338 in H6). Dieser Eingang ist viel breiter als bei RXRα (7,1 Å von Pro-264 in H3 bis zu Ala-340 in H6). Die Topologie der Ligandenbindetasche ist recht ungewöhnlich mit einer Spalte zwischen H3 und H6. In RXRα und anderen NRs bildet dieser Bereich feste Kontakte zu der verbindenden Schleife L1-3 aus. Das Volumen der Höhle der USP-LBD ist um den Faktor 2,5 größer als das der hRXRα-LBD (1256 Å³ bei USP verglichen mit 489 Å³ bei hRXRα).

### Beispiel 4

### Der mutmaßliche Ligand von USP in der Kristallstruktur

Die Ligandenbindetasche des USP von Heliothis virescens enthält unerwarteterweise ein Molekül, das zusammen mit der USP-LBD co-gereinigt und und co-kristallisiert wurde. Der Fit der Elektronendichte passt gut zu der massenspektroskopischen und analytisch-chemischen Charakterisierung der Moleküle. In ähnlicher Weise zeigen neue kristallographische Untersuchungen der heterodimeren RARα/RXRα-LBD eine E.coli-endogene Ölsäure (C18) oder eine ähnliche Verbindung (Stearin-(C18)- oder Palmitin-(C16)säure) in der RXRα-Untereinheit. Obwohl dieses Molekül nicht der natürliche Ligand von Vertebraten-NR ist, induziert und stabilisiert er eine antagonistische AF-2 Konformation, die höchstwahrscheinlich sehr ähnlich zu der tatsächlichen Antagonist-gebundenen RXRα ist.

Im vorliegenden Fall wurde der beste Fit der Elektronendichte mit der Annahme eines Phospholipids erhalten, dessen erster Schwanz aus einer Fettsäure mit einer Länge von 18 Kohlenstoff-Atomen an C1 besteht, und einer 16 Kohlenstoff-Atom langen zweiten Kette an C2. Die längere der beiden Fettsäuren hat eine recht verdrehte Gestalt mit zwei größeren Knicken, wohingegen die andere Fettsäure innerhalb der Tasche eine eher normale Form einnimt. Der Schwanz des Phospholipids ist innerhalb der Ligandenbindetasche verborgen. Der Glycerin-Teil und die beiden Fettsäuren bilden van-der-Waals-Kontakte mit den Resten in L1-3 (Leu-230, Val-238), H3 (Phe-242, Leu-249), H5 (Leu-291), L6-7 (Ala-339), H7 (Phe-345), H11 (Ser-431, His-434, Phe-438) and L11-12 (Leu-440). Die Kopfgruppe des Phospholipids liegt vorne am Eingang der Tasche zwischen H3 und H6. Beim Phosphatidylglycerin bildet die Carbonylgruppe des Phosphorylglycerins und beim Phosphatidylethanolamin die Aminogruppe des Ethanolamins eine starke Wasserstoffbrückenbindung mit Gln-338 (H6) aus. Zusätzlich wird ein Sauerstoff der Phosphatgruppe über eine Wasserstoffbrücke mit einem Rest L1-3 (Cγ of Pro-239) gebunden.

Vermutlich stellt das hier gefundene Phospholipid keinen natürlichen Liganden des USP dar. Es wurde jedoch eindeutig gezeigt, dass Liganden von USP existieren.

Die Reste, die mit dem Liganden in Wechselwirkung treten, sind innerhalb der Lepidopteren-USPs streng konserviert, mit der Ausnahme von Ser-431, das in msUSP durch ein Cysteine ersetzt wird. Im Gegensatz dazu wechselwirken von den 16 Resten der RXRα-LBD, die mit 9-cis RA wechselwirken, nur 3 der entsprechenden USP-Reste mit dem Phospholipid (Leu-249, Ser-431 and His-434). Der Grund für dieses Verhalten liegt hauptsächlich in der unterschiedlichen Position der Liganden in den entsprechenden Taschen. Die 9-*cis* RA ist tief im Innernen der Tasche vergraben, wo seine Carboxylatgruppe eine Salzbrücke zu Arg-316 aus der Helix H5 der hRXRα aufbaut. Im Gegensatz dazu dringt das Phospholipid nicht tief in das Innere der Tasche ein. Beispielsweise liegt der Schwanz der längeren Fettsäure ungefähr bei Atom C9 von 9-*cis* RA in der hRXRα-LBD, wohingegen der Schwanz der anderen Fettsäure fast bis zum β-ionon Ring von 9-*cis* RA reicht. Als Folge davon beteiligt sich Arg-297 nicht an der Verankerung des Liganden, wie es bei der agonistischen RXRα-, RARγ- und anderen NR-LBDs beabachtet wird. Trotzdem nimmt es fast die gleiche Position wie Arg-316 der Holo-RXRα ein und nicht die dem Lösungsmittel ausgesetzte Position der Apo-RXRα-Konformation. Anstatt mit dem Liganden in Wechselwirkuung zu treten, bildet Arg-297 Wasserstoffbrückenbindungen zur Backbone-Carbonylgruppe von Leu-325 (β-Faltblatt) aus und beteiligt sich unter Wasser-Beteiligung an einem Wasserstoffbrücken-Netzwerk mit Leu-290 (H5) und der Seitenkette von Gln-256 (H3). An diesen Wechselwirkungen sind insbesondere zwei Wassermoleküle beteiligt, die räumlich etwa bei den beiden Sauerstoff-Atomen der Carboxylatgruppe von 9-*cis* RA liegen.

### Beispiel 5

### Die antagonistische Konformation der USP-LBD

Die AF-2 Domäne in der Struktur der USP-LBD weist eine durch den Liganden in der Ligandenbindetasche erzeugte antagonistische Konformation auf. H12 nimmt die gleiche Konformation ein, die im Fall anderer Antagonisten-gebundener Kernrezeptoren wie RXRα/Ölsäure, RARα/BMS614 und ER gefunden wurde. In all diesen Fällen wurde beobachtet, dass die Furche, in der H12 liegt, der Bindestelle für die helikale Kernrezeptor-Box von Kernrezeptor-Koaktivatoren entspricht. Diese helikale Kernrezeptor-Box zeichnet sich durch die Konsensus-Sequenz LXXLL aus, wie für die Ligandenbindedomäne von PPARγ, TRβ und ERα gezeigt wurde. Im USP von Heliothis virescens liegen Ile-450, Ala-453 und Leu-454 von H12 in etwa der gleichen Lage, wie der erste, zweite und dritte Leucin-Rest des LXXLL Bindungsmotives (IXXAL anstelle LXXLL). Wie in anderen antagonistischen Konformationen von Kernrezeptoren ist H12 in eine Furche aus Resten von H3 und H4 sowie von L3-4 (Val-261, Arg-265, Met-275, Glu-276, Ile-279, Ile-282, Lys-283) eingepackt. In der USP-LBD ist jedoch auch L1-3 an der Furchentopologie beteiligt und hat mit den Resten Phe-227, Gln-228 und Phe-229 van-der-Waals-Kontakte mit H12.

Die Länge von H12 in der USP-LBD ist identisch mit der von H12 in der Antagonisten-gebundenen Form der RXRα-LBD. Jedoch findet sich das Strukturprinzip, das in einem anderen Fall einer antagonistischen Konformation einer Kernrezeptor Ligandenbindetasche beobachtet wurde, bei der USP-LBD nicht vollständig. Und zwar wurde dort gefunden, dass H11 sich aufwindet und so H12 erlaubt, sich an die Bindungsfurche des Kernrezeptor-Kooaktivator-Bindungsmotifs LXXLL zu binden. H11 befindet sich in der Verlängerung von H10 und überlagert sich sich sehr gut mit H11 in der Holo-RXRα-LBD Struktur, außer dass die H11 der USP-LBD um zwei Reste kürzer ist. Es folgt eine 6 Reste lange Region, die H11 und H12 verbindet (His-439 to Thr-444). Diese Aminosäuren der Schleife L11-12 überspannen in einer gestreckten Konformation einen 12 Å langen Strang. Der C-Terminus von H11 enthält drei Phenylalanine, die man auch in RXRα findet. In Apo-RXRα zeigen die beiden ersten Phenylalanine zur hydrophoben Ligandenbindetasche und das dritte Phenylalanin ist dem Lösungsmittel zugewendet. In der Agonisten-gebundenen Form vertauschen die Phenylalanine ihre Rolle. In der USP-LBD ist die Situation ähnlich zur Agonisten-gebundenen Form der RXRα-LBD: Phe-436 und Phe-437 sind zum Lösungsmittel gewendet, während Phe-438 zur Ligandenbindetasche beiträgt. Die Seitenkette von Phe-438 ist im Vergleich zum Gegenstück in RXRα leicht gedreht, und berührt den Liganden auf Höhe seiner kürzeren Fettsäure. In der Antagonisten-gebundenen Form von RXRα entspricht der erste Rest des Phenylalanin-Tripletts dem Ende von H11. Dieser Rest befindet sich in etwa in der Position des C-α Atoms von Phe-437. Die beiden anderen Phenylalaninreste, die bereits Teil von L11-12 sind, sind in der Ligandenbindetasche einwärts zum Proteininneren orientiert. In einer Überlagerung des USP von Heliothis virescens und der Antagonisten-gebundenen RXRα-LBD kollidieren diese beiden Reste mit dem Phospholipidliganden.

### Beispiel 6

### Die Verbindungsregion L1-3 interagiert mit H3 und L11-12 und verhindert eine agonistische Konformation

Die Bindung des Phospholipids in der Ligandenbindetasche der USP-LBD erzeugt wahrscheinlich bedeutende strukturelle Umlagerungen in der USP-LBD. Der Vergleich mit den Apo- und Holo-RXRα-LBD-Strukturen lässt vermuten, dass auch bei der USP-LBD die molekularen Mechanismen, die zur Liganden-gebundenen LBD-Konformation führen, die Verlagerung von H3 und H11 beinhalten. Jedoch spielt im USP von Heliothis virescens anders als in allen anderen bisher bekannten Kernrezeptor-LBDs das strukturelle Element L1-3 eine wesentliche Rolle.

Die Schleife L1-3 interagiert mit H3, H11, L11-12 und H12. Diese Strukturelemente werden durch die Ligandenbindung am stärksten beeinflusst. L1-3 stabilisiert den N-Terminus von H3 durch ein Wasserstoffbrücken-Netzwerk mit Arg-243 und Asn-254 von H3. Der Guanidinium-Teil des Arg-243 ist mit starken Wasserstoffbrücken an die Backbone-Carbonyle von Gly-233, Ser-236 und Val-238 verankert (Abstände von 2,61, 2,97 und 2,78 Å) und zeigt einen van-der-Waals-Kontakt zur Seitenkette von Val-232. Darüberhinaus ist die Backbone-Amidgruppe des Arg-243 durch eine Wasserstoffbrücke an die Carbonylgruppe von Pro-239 (3,20 Å) gebunden. Die Seitenkette von Asn-254 bildet Wasserstoffbrücken zur Carbonylgruppe von Leu-230 (2,83 Å), zur Amidgruppe von Phe-229 (3,10 Å) und über ein Wassermolekül zur Seitenkette von Gln-228. Außerdem steht es in van-der-Waals-Kontakt zur Carbonylgruppe von Phe-227. Die Backbone-Carbonylgruppe von Asn-254 bildet eine starke Wasserstoffbrücke zur Seitenkette von Glu-226 (2,74 Å).

L1-3 (Gln-228 to Arg-231, Asp-235 und Ser-236) steht auch in Kontakt zum N-terminalen Bereich von H11 und zu L11-12. Die Backbone-Carbonylgruppe von Gln-228 bildet eine Wasserstoffbrücke zu Ala-442 (3,20 Å), und die Backbone-Carbonylgruppe von Phe-229 bildet eine starke Wasserstoffbrücke zur Amidgruppe von Ala-442 (2,88 Å). Darüberhinaus stabilisiert Arg-231 mit starken Wechselwirkungen die Schleife L11-12: die Backbone-Amidgruppe bildet eine starke Wasserstoffbrücke zur Carbonylgruppe von Leu-440 (2,90 Å), während die Seitenkette eine starke Wasserstoffbrücke zur Carbonylgruppe von His-439 (3,00 Å) bildet und van-der-Waals-Kontakte mit Val-441 und Ala-442 zeigt. Andere Wechselwirkungen betreffen das Backbone-Carbonyl von Asp-235 mit der Seitenkette von His-439 und einer Wasser-vermittelten Wechselwirkung mit Val-441. Die Hydroxylgruppe von Ser-236 bildet einen van-der-Waals-Kontakt mit der Seitenkette von Leu-440.

Es ist wichtig festzustellen, dass es bei allen Resten, die an der Wechselwirkung von L1-3 mit H3 und mit L11-12 beteiligt sind, eine hohe Sequenzkonservierung gibt. Die Hauptinteraktionspartner von H3, Arg-243 und Asn-254, sind in allen Lepidopteren-USPs strikt konserviert. Ebenso sind alle Interaktionspartner in L1-3 (Glu-226, Phe-227, Gln-228, Phe-229, Leu-230, Val-232, Gly-233, Ser-236, Val-238, Pro-239) in allen Lepidopteren-USPs strikt konserviert; mit Ausnahme von Phe-227 und Phe-229, die im USP von Bombyx mori durch Leucin und Isoleucin ersetzt sind. Auch im Fall der Wechselwirkungen von L1-3 mit L11-12 sind die beteiligten Reste (L1-3: Gln-228 bis Arg-231, Asp-235 und Ser-236; L11-12: His-439 bis Ala-442) außer Phe-229 und Asp-235 in allen Lepidopteren-USPs strikt konserviert. Das ist ein starker Hinweis auf darauf, dass Wechselwirkungsmuster von L1-3 mit H3 und von L1-3 mit L11-12 in allen Lepidopteren-USPs ähnlich sind.

In der Überlagerung des USP von Heliothis virescens mit den Holo-RXRα-LBDs kann man beobachten, dass einige Reste aus L1-3 im USP von Heliothis virescens (Asn-237, Ser-236 und Phe-229) etwa an der gleichen Stelle liegen, wie Reste aus L11-12 von Holo-RXRα (Asp-444, Thr-445 und Phe-450). Dieser Vergleich erlaubt die aufschlussreiche Folgerung, dass L1-3 in seiner aktuellen Konformation die Existenz einer agonistischen Konformation ausschließt, weil diese an der Schleife L11-12 behindert wäre. Es handelt sich jedenfalls um kein Kristallisationsartefakt und spiegelt die besondere Rolle dieses Strukturelements in den Lepidopteren-USPs wieder.

Die sterische Verhinderung der agonistischen Position von H12 ist hier ein konstitutiver Bestandteil der Rezeptorstruktur und nicht, wie bei anderen Kernrezeptor-LBDs, die mit voll antagonistischen Liganden besetzt sind, die Folge der Sperrigkeit des Liganden.

Es lässt sich vorhersagen, dass durch Ligandenbindung von Agonisten eine Konformationsänderung der USP-LBD erzeugt wird, die L1-3, H12 sowie die anderen beschriebenen Reste der LBD in eine antagonistische Position umspringen lässt.

Es handelt sich jedenfalls nicht um ein Kristallisationsartefakt und spiegelt die besondere Rolle dieses Strukturelements in den Lepidopteren-USPs wieder.

### Beispiel 7

### Agonistische Konformation der USP-LBD von Heliothis virescens durch Homologie-Modelling, basierend auf dem RXRα/9-cis-RA-Komplex

Um ein 3D-Modell der USP-LBD von Heliothis virescens zu erstellen, wurden die fehlende Reste der Schleife zwischen den Helices H1 und H3 (L1-3) aus der hRXRα-Kristallstruktur so ergänzt, daß ein kontinuierlicher Backbone entsteht. Die so entstandene Struktur ist das experimentelle hRXRα-Referenzmodell.

In der Einheitszelle von hRXRα wurden zwei hRXRα-Monomere beobachtet, in jedem dieser Monomere war die Ll-3-Region schlecht aufgelöst. Durch Überlagerung der beiden unabhängig voneinander verfeinerten Strukturen ergaben sich Hinweise darauf, wie diese Schleife modelliert werden sollte. Es wurde ein vollständiges 3D-Modell von hRXRα erstellt, das auf Kristallstruktur basiert, und in dem die Residuen in L1-3 ergänzt sind. Die Wasserstoffatome wurden mit Hilfe der Hbuild-Option des Programms Charmm ergänzt.

Die L1-3-Region wurde durch eine Powell-Minimierung des Programms Charmm relaxiert (1000 Optimierungsschritte, Dielektrizitätskonstante : 4, Gradiententoleranz: 10⁻⁶, Schrittweite 0.02, Cutoff für nichtbindende Wechelwirkungen: 15 Å).

Diese optimierte Struktur wurde als Vorbild für das Homologie-Modell der USP-LBD von Heliothis virescens benutzt.

Die Aminosäuresequenzen der USP-LBD von Heliothis virescens und hRXRα wurden entsprechend der Tabelle 2 zugeordnet.

Mit Hilfe des Softwarepaketes Modeller und dessen Standardeinstellungen wurde anhand der Zuordnung ein 3D-Modell erzeugt. Die USP-LBD-Sequenz weist einige wenige Insertionen in der Schleife 1-3 und in der Schleife vor dem ersten β-Faltblatt auf. Um vernünftige Konformationen für diese beiden Regionen zu erstellen, wurde die Option lego-loop des Software-Paketes O verwendet. Anschließend wurde die USP-Modellstruktur einer Powell-Minimierung unterworfen (2000 Optimierungsschritte, Dielektrizitätskonstante: 4, Gradiententoleranz: 10⁻⁶, Schrittweite 0.02, Cutoff für nichtbindende Wechelwirkungen: 15 Å). Die Qualität der so erhaltenen Struktur wurde mit dem Programm PROCHECK analysiert. Danach liegen 97% der Residuen in erlaubten Bereichen und weniger als 2% der Residuen in verbotenen Bereichen. Letztere liegen in den vorher beschriebenen modifizierten Regionen.

### Beispiel 8

### Vergleich zwischen der agonistischen USP-LBD-Struktur, die aus dem hRXRα/9-cis RA-Komplex erhalten wurde, und der USP-LBD-Kristallstruktur

Die größten Unterschiede zwischen diesen beiden Strukturen liegen in der Position der Aktivierungshelix (H12) und dem Verlauf der Schleife zwischen den Helices H1 und H3. Die Aktivierungshelix H12 befindet sich in der experimentellen Struktur in der antagonistischen Position, während sie in der Modellstruktur eine agonistische Konformation einnimmt, die die Ligandenbindenische verschließt. In der experimentellen Struktur liegt die Schleife L1-3 über der Helix H3 und stabilisiert die antagonistische Position von H12 durch hydrophobe Kontakte. Im Gegensatz dazu ist diese Schleife im agonistischen Homologie-Modell ziemlich weit von der zentralen AF2-AD Helix entfernt. Die Schleife L1-3 ist von der Helix H3 durch das β-Faltblatt getrennt.

Weiterhin unterscheidet sich die Größe der Ligandenbindenische zwischen beiden Strukturen beträchtlich. Die Gegenwart des großen Fettsäurerestes in der USP-LBD-Kristallstruktur bewirkt eine große Kavität durch Verschiebung der Helices H3, H6 und H11. In der USP agonistischen Konformation sind diese Helices dicht gepackt und erzeugen eine kleinere Ligandenbindenische.

Die Regionen der C-terminalen Enden von H3, H4, H5, H8 und H9 sind starr und lassen sich in den beiden Strukturen sehr gut überlagern. Im Gegensatz dazu sind die Schleife L1-3 und die C-Termini von H3, H6 und H11 in beiden Strukturen gegeneinander verschoben. Diese Segmente bilden die beweglichste Region der Ligandenbindedomäne von Kernrezeptoren. Wahrscheinlich ist diese Bewegung für jeden Rezeptor und die durch einen Liganden erzeugte Verschiebung spezifisch.

### Eräuterungen zum Sequenzprotokoll

SEQ ID NO: 1 zeigt die Aminosäuresequenz der USP-LBD von Heliothis virescens.

SEQ ID NO: 2 zeigt die Aminosäuresequenz des USP von Heliothis virescens.

### Erläuterungen zu den Tabellen

Die Tabelle 1 zeigt die Strukturkoordinaten der LBD des USP von Heliothis virescens.

Die Tabelle 2 zeigt die Aminosäuresequenz-Zuordnung für hRXRα und USP von Heliothis virescens sowie weiterer Kernrezeptor-LBDs für die Bildung eines Homologie-Modells der agonistischen Konformation von USP.

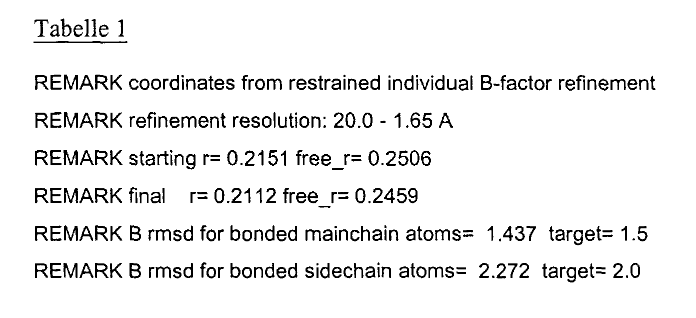

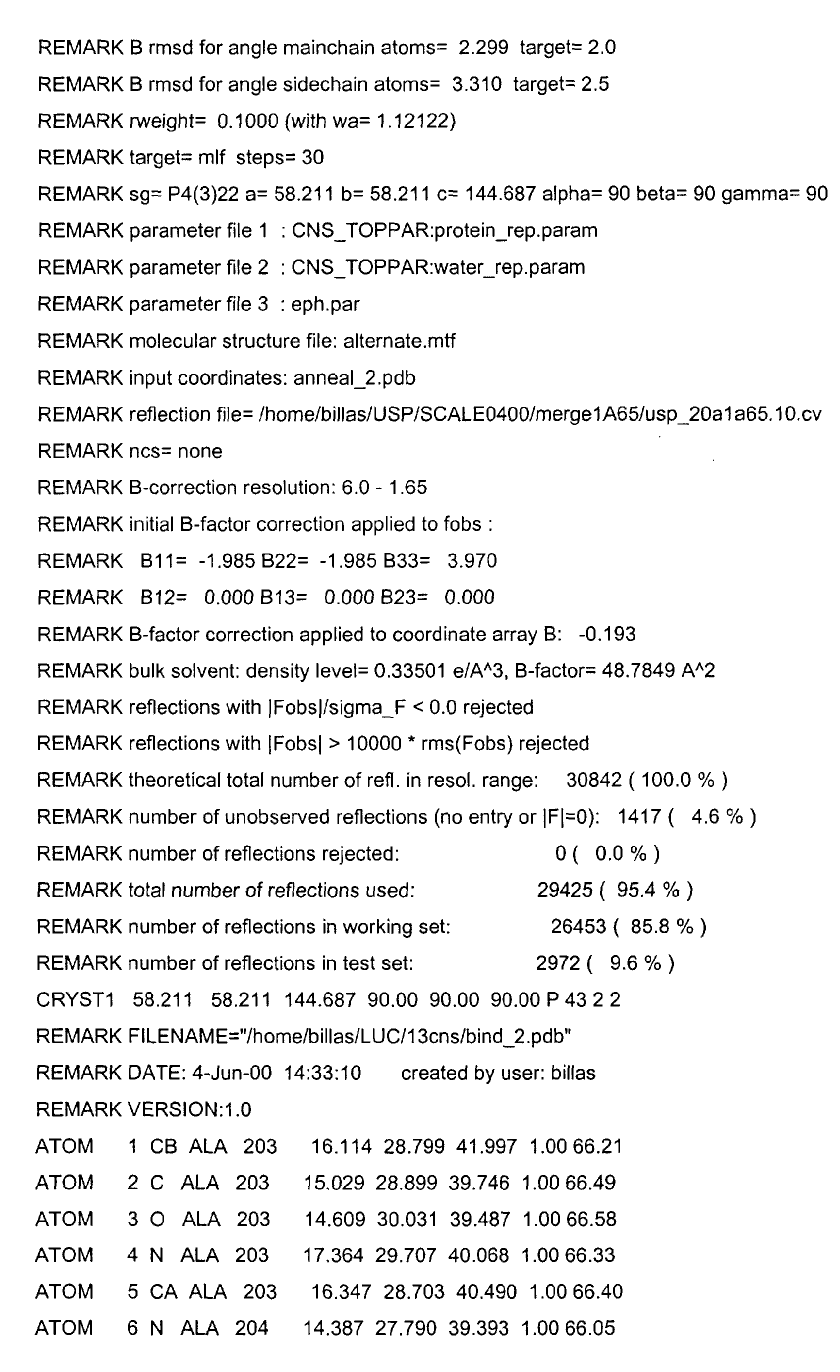

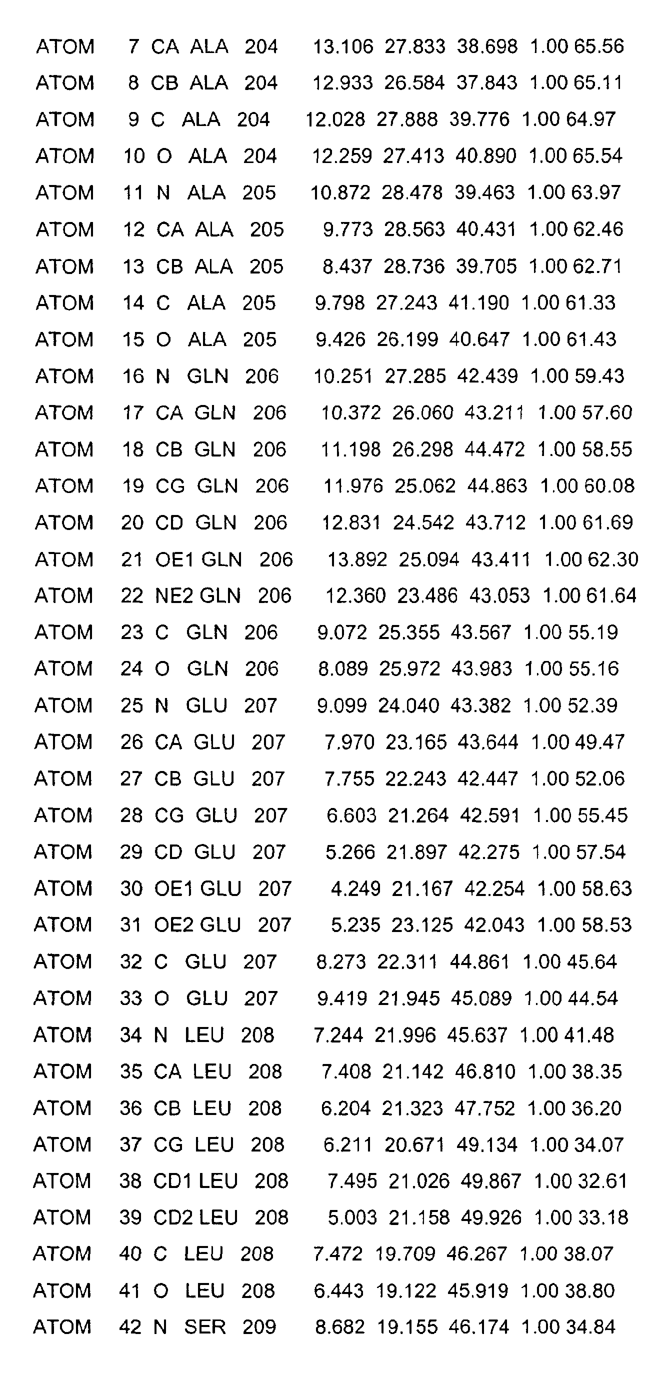

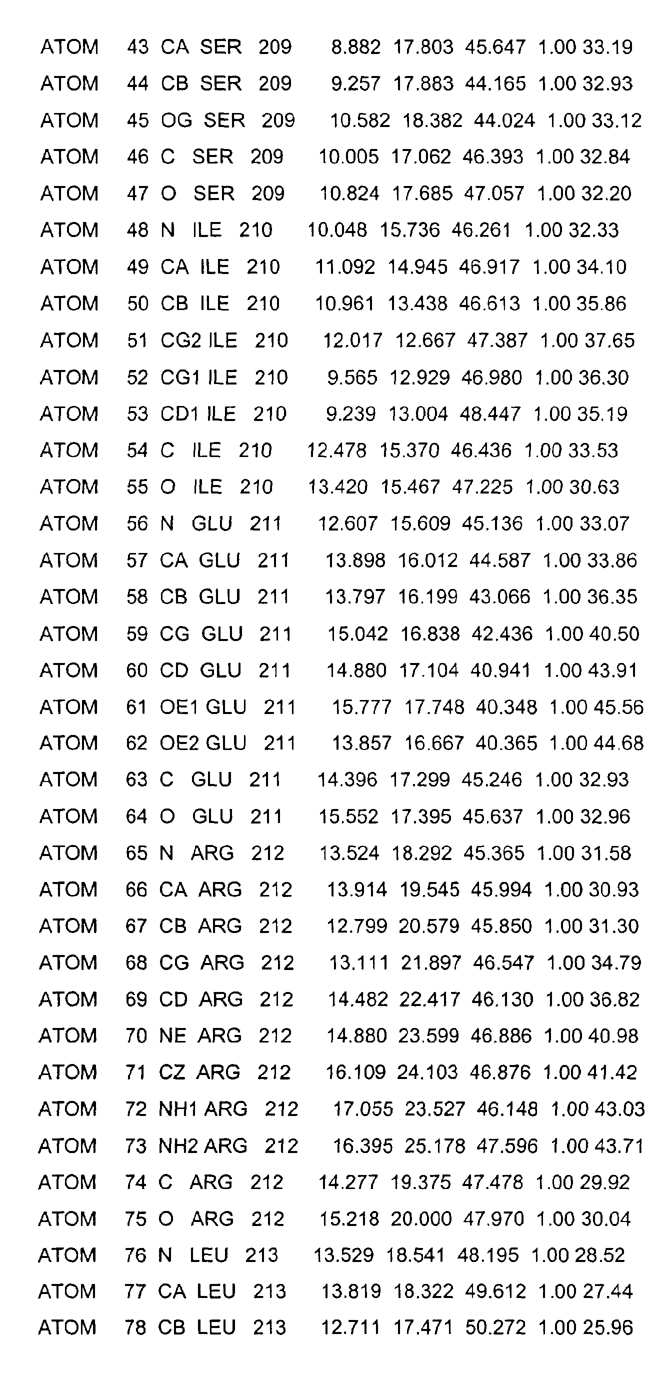

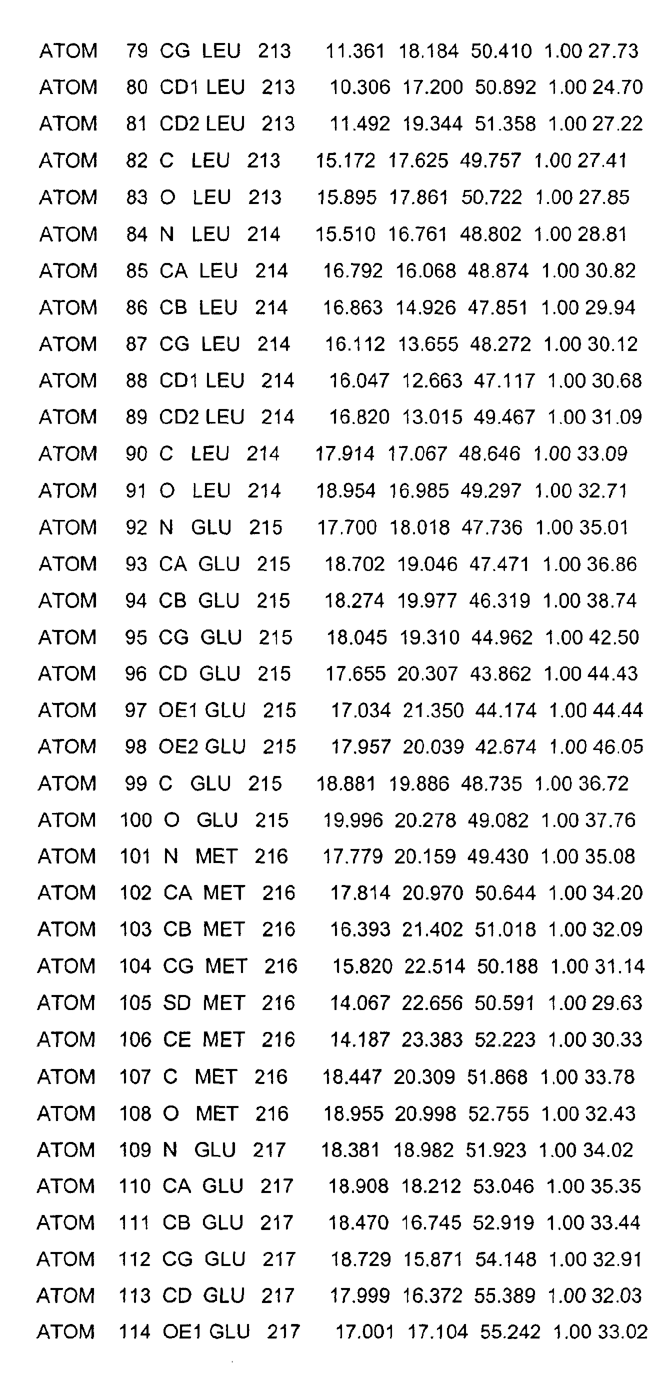

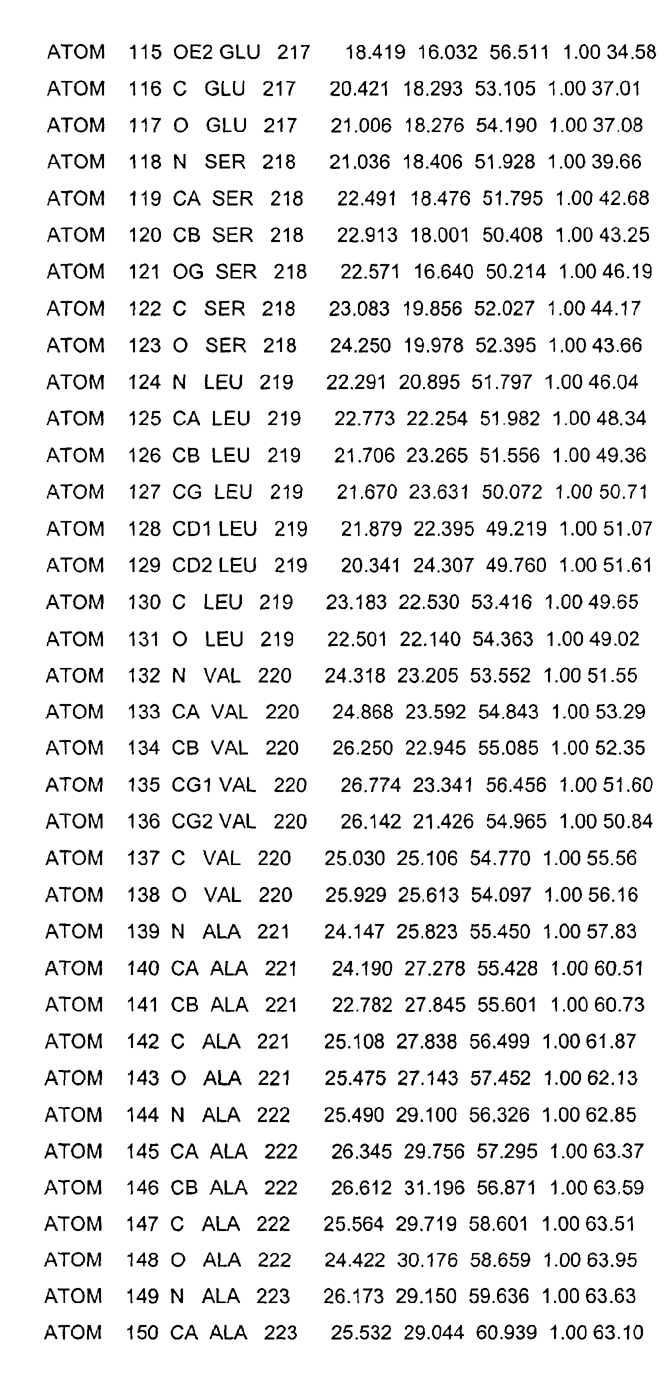

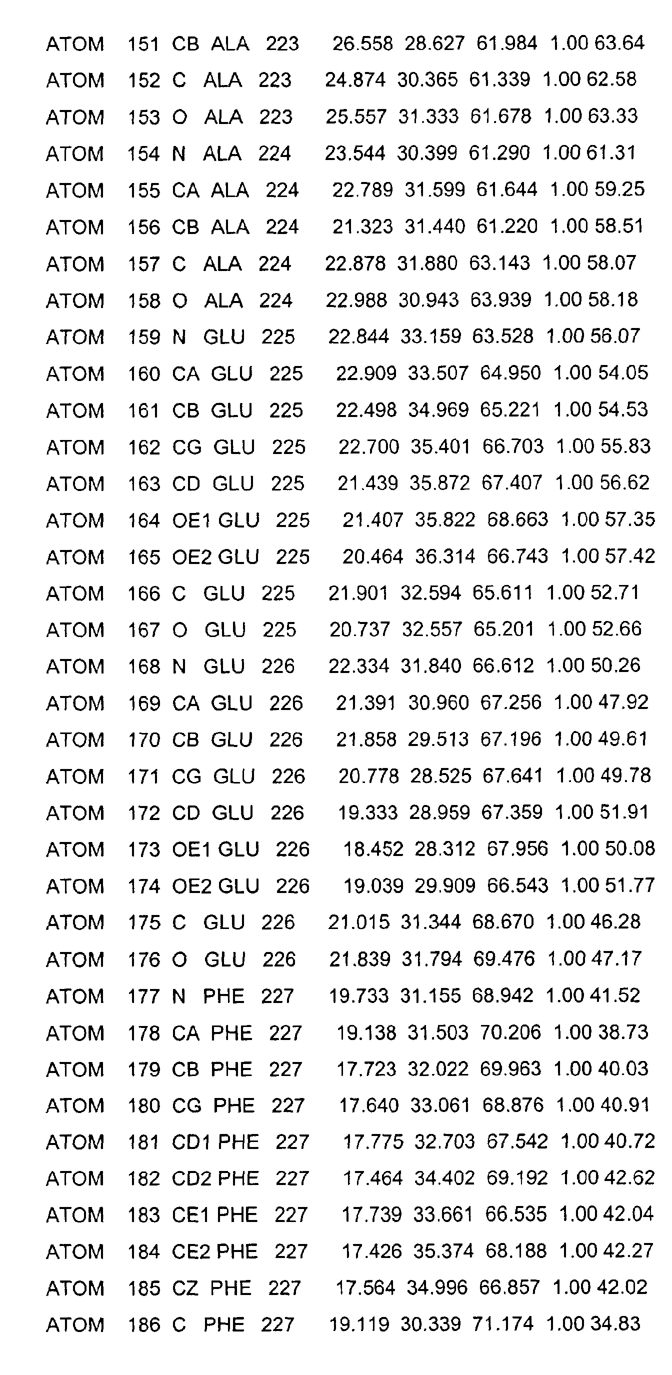

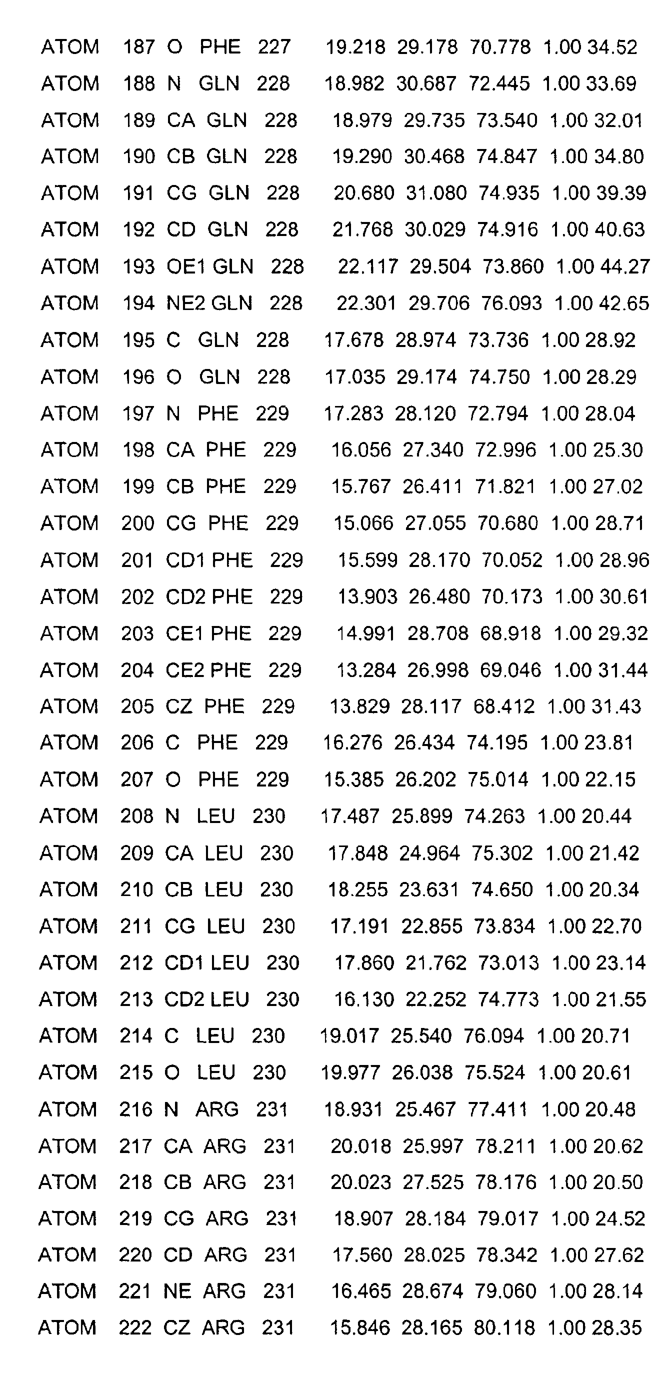

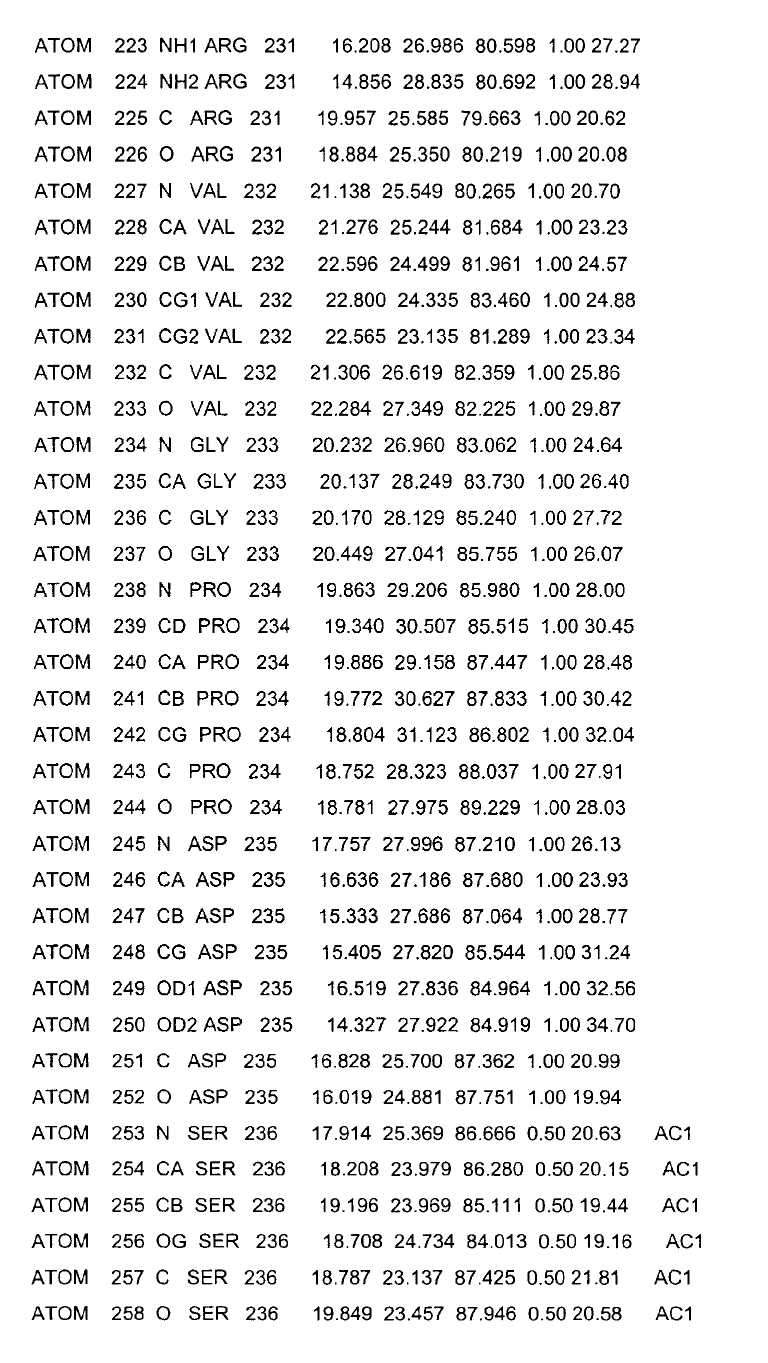

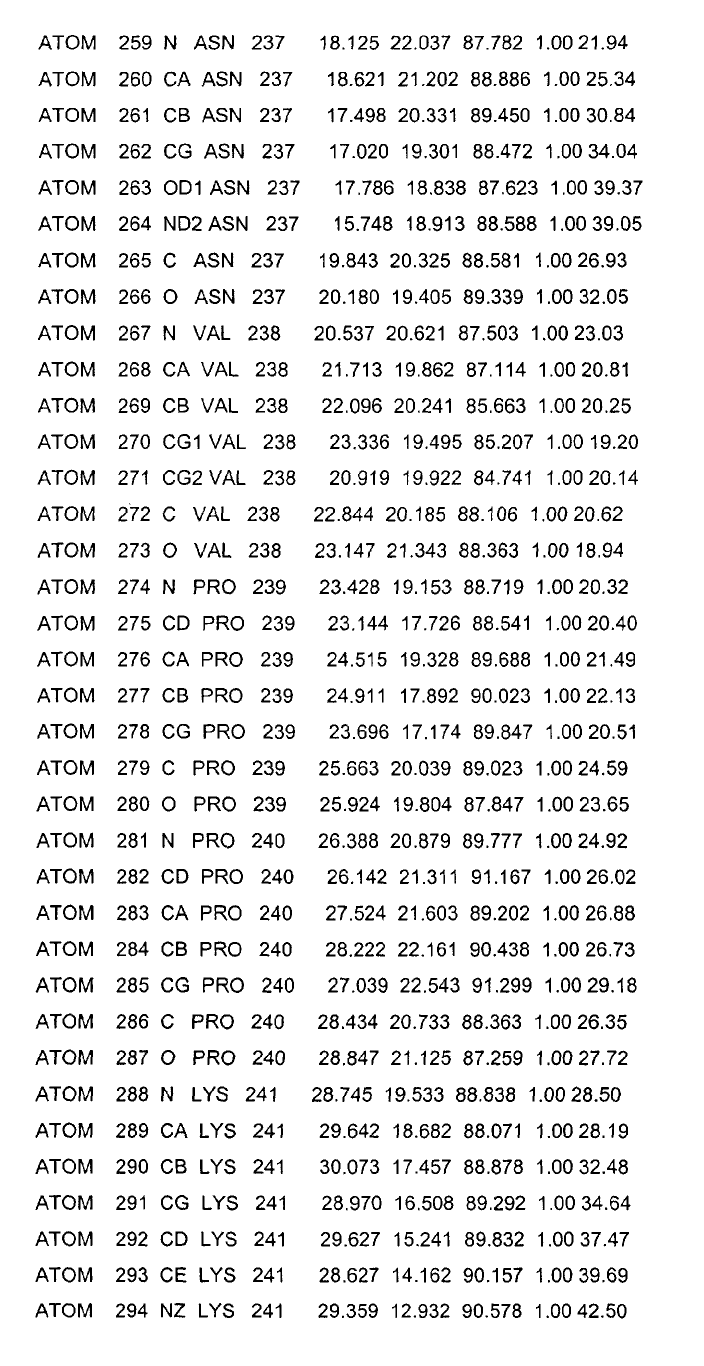

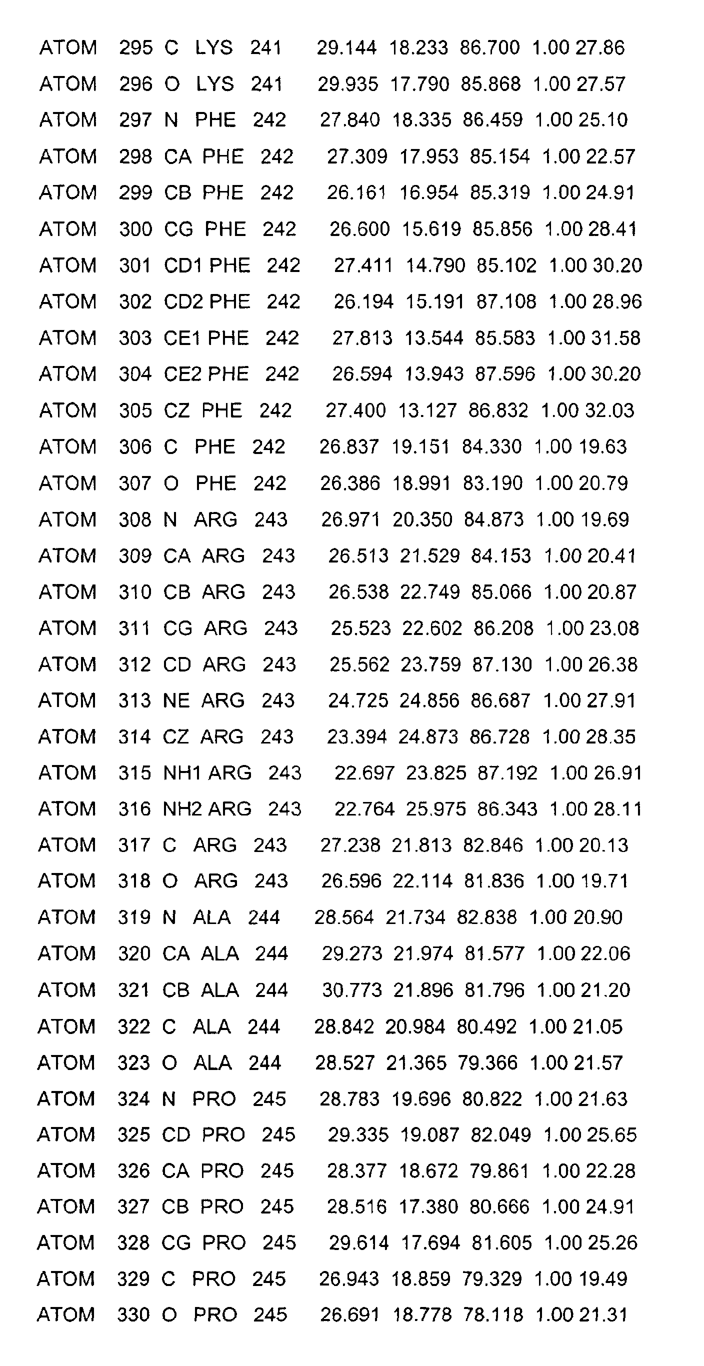

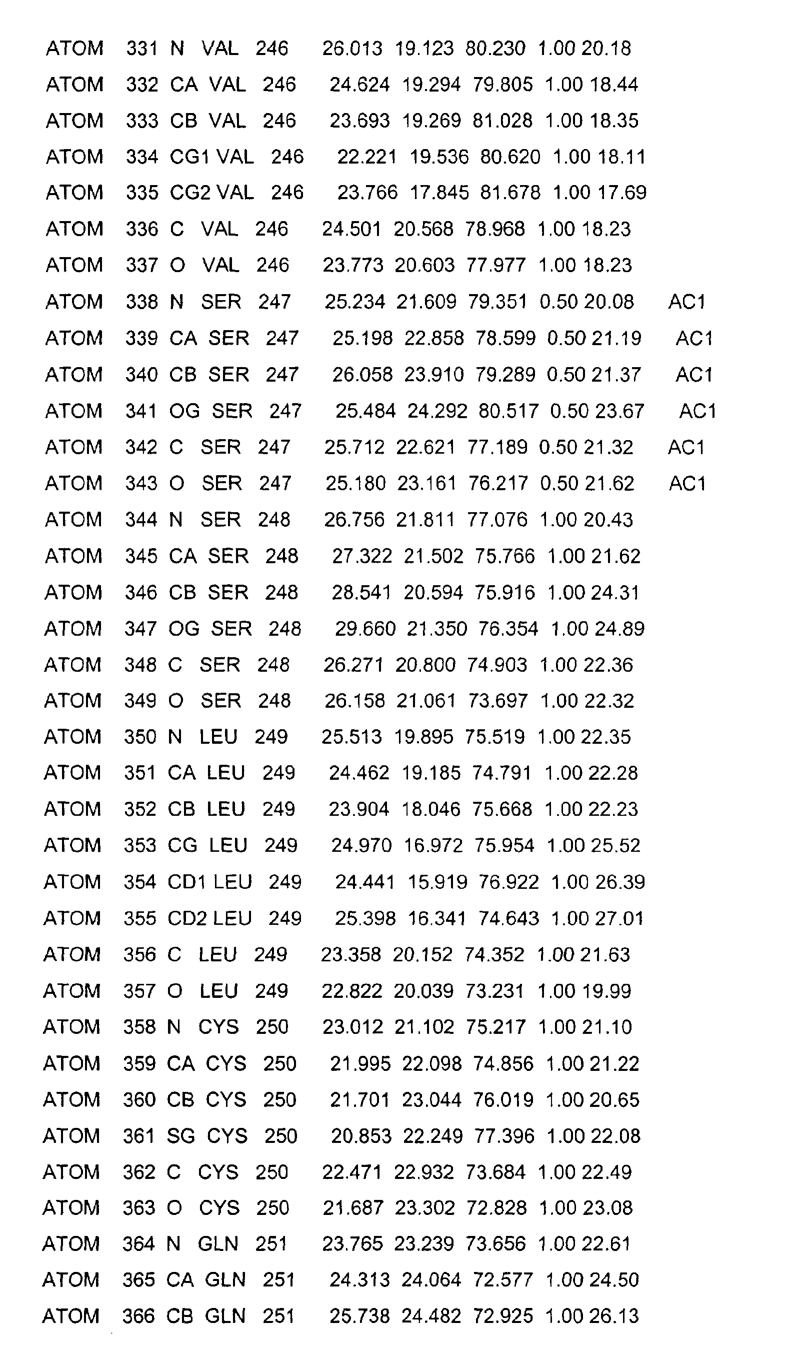

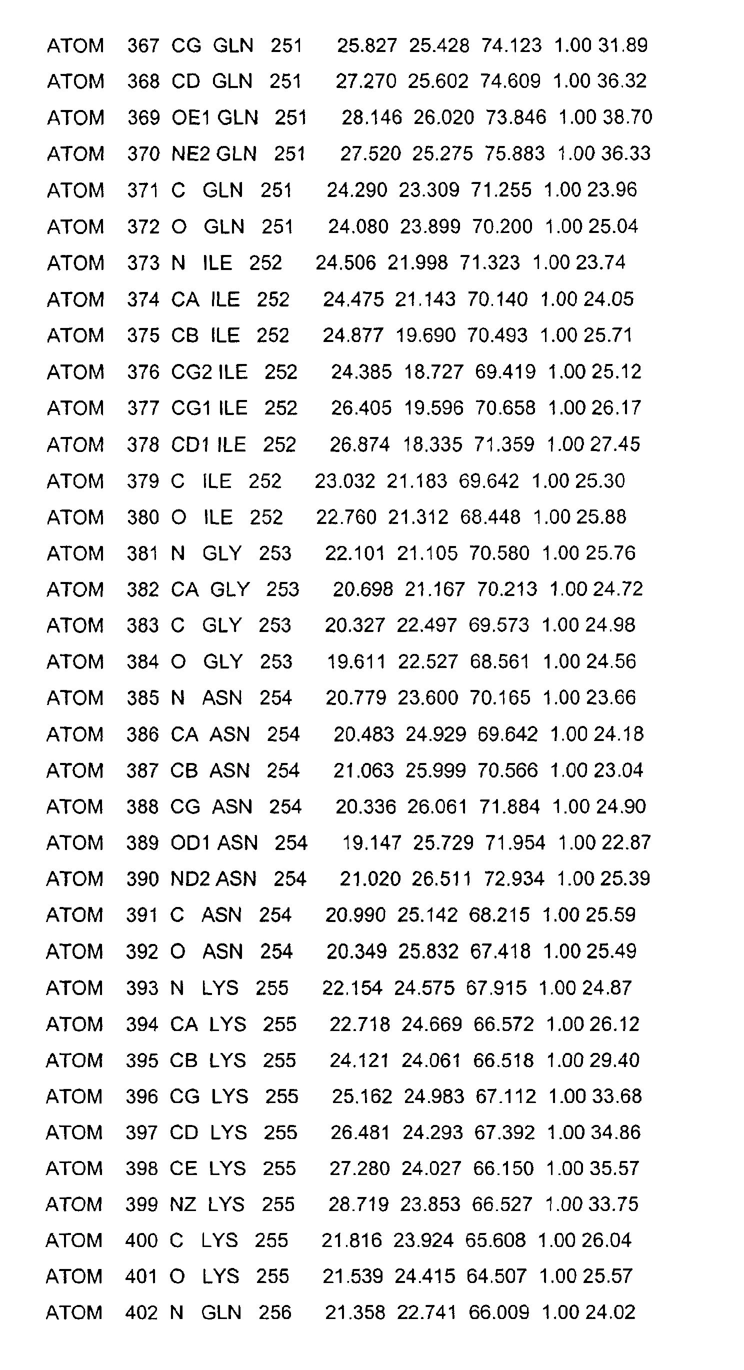

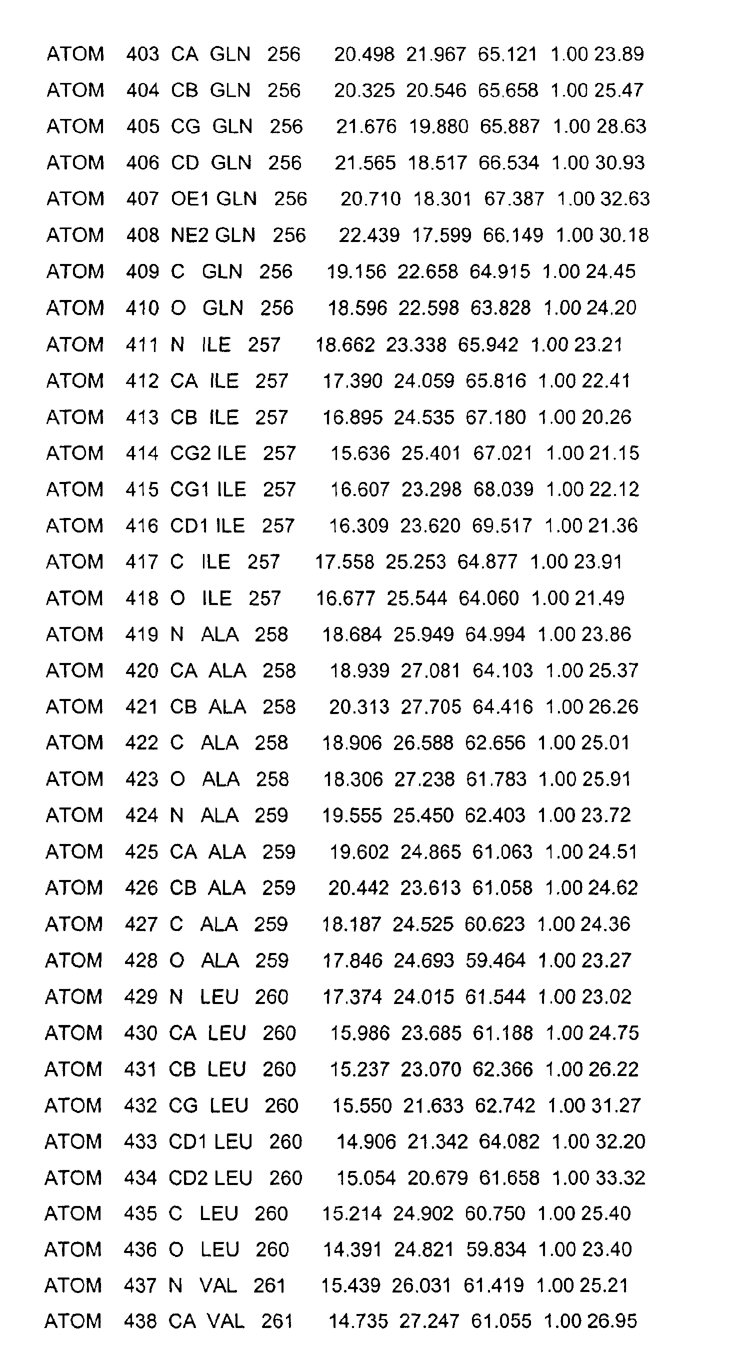

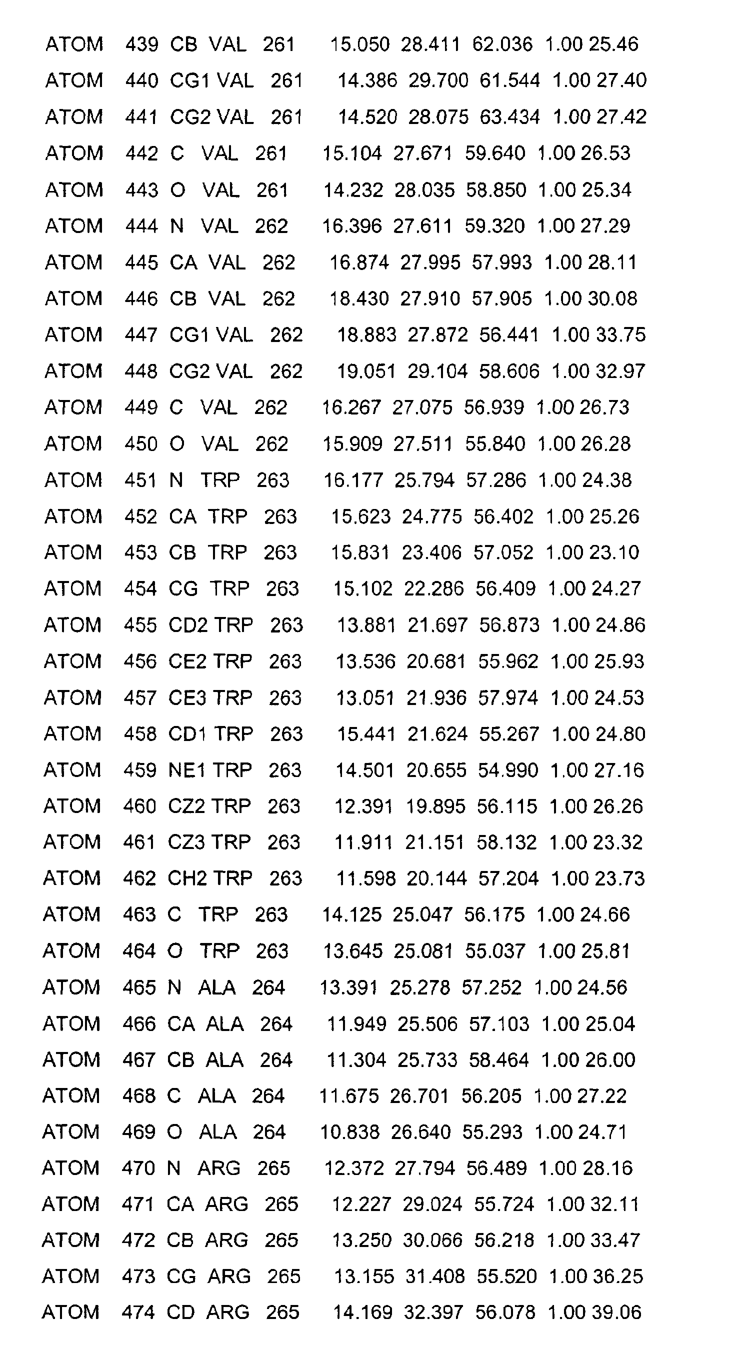

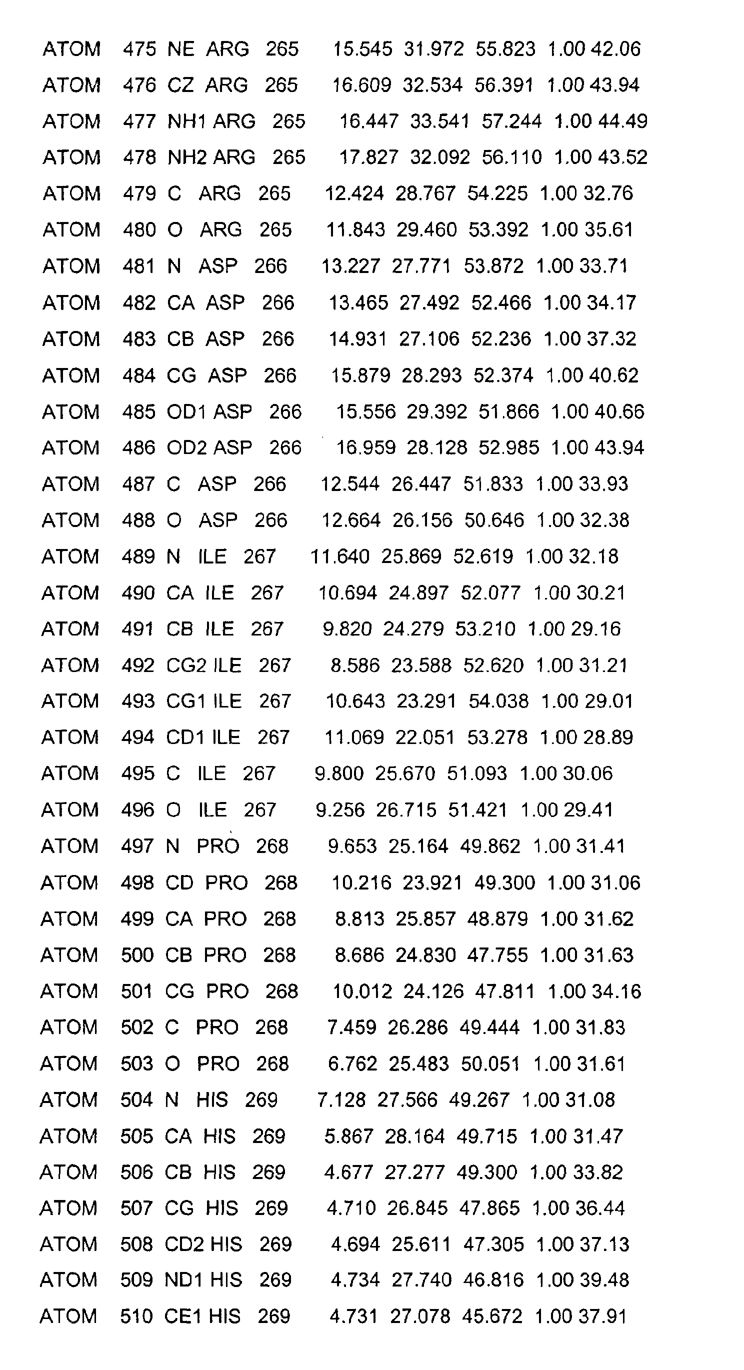

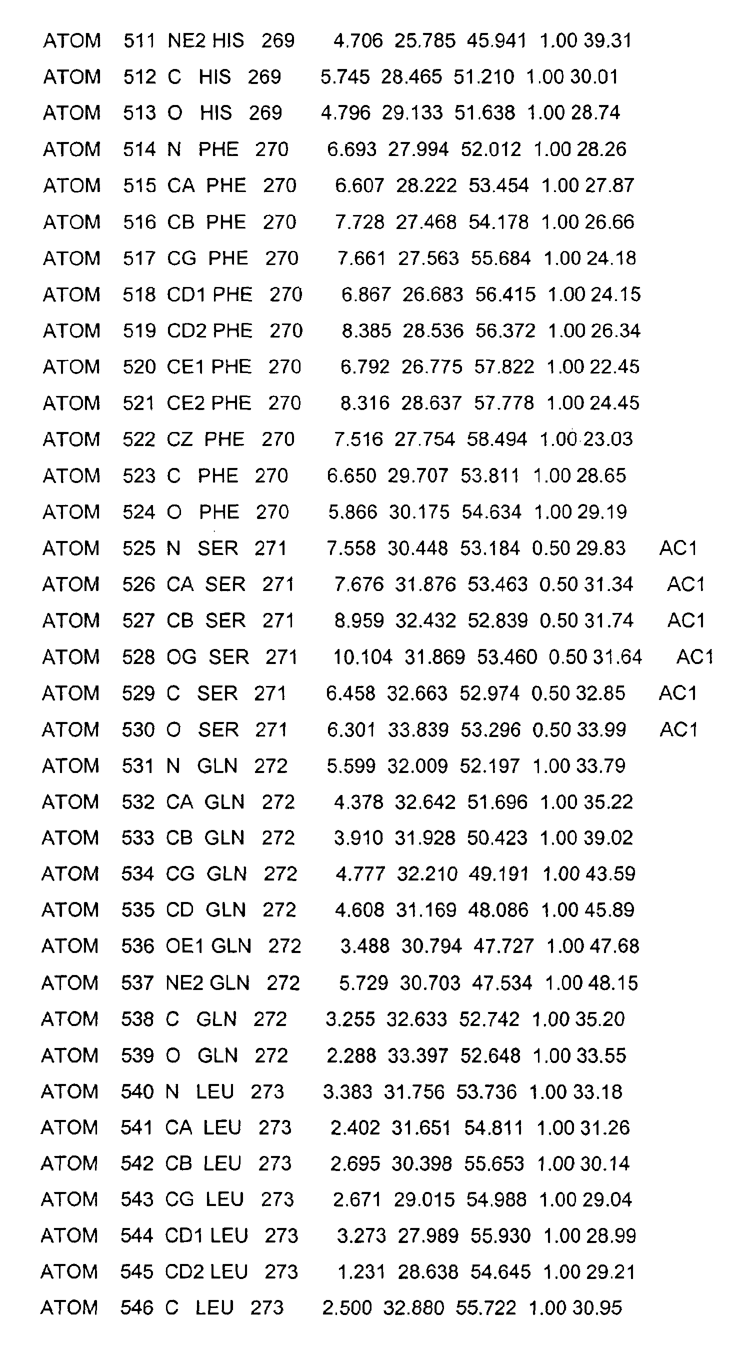

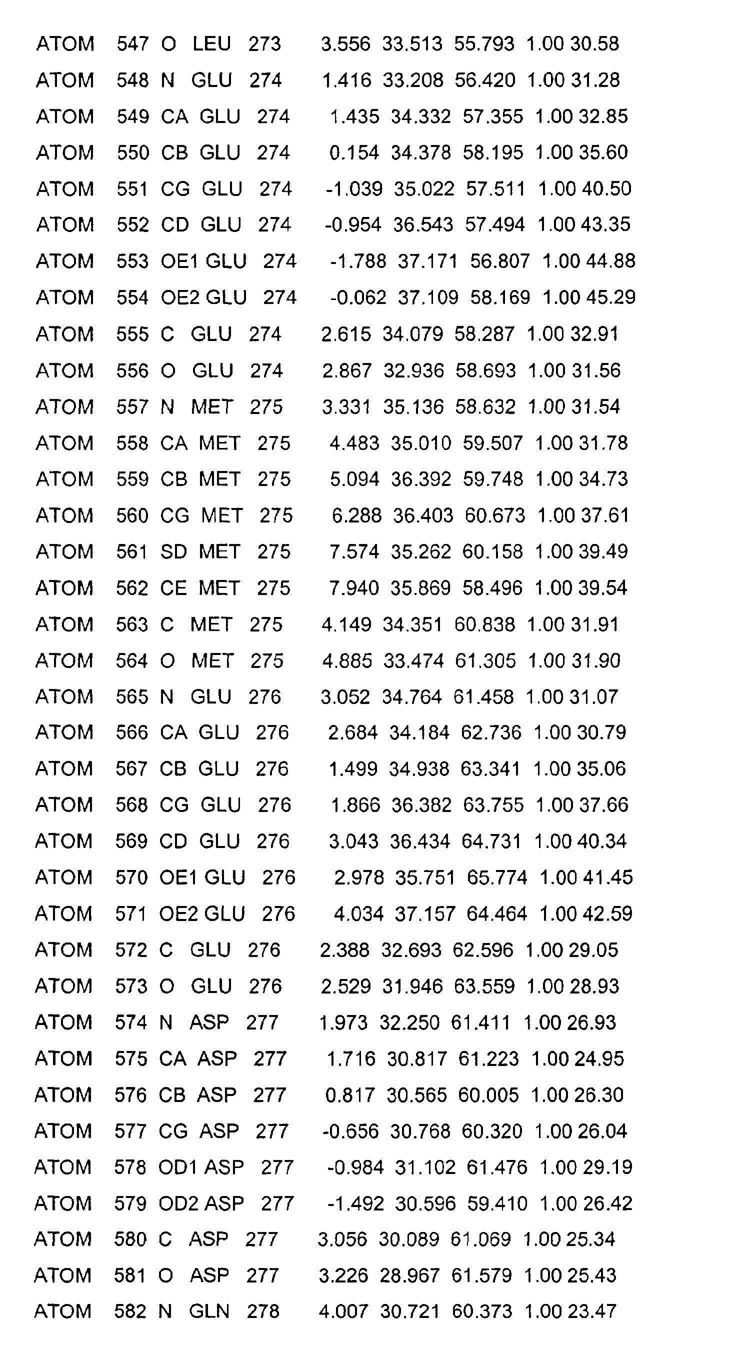

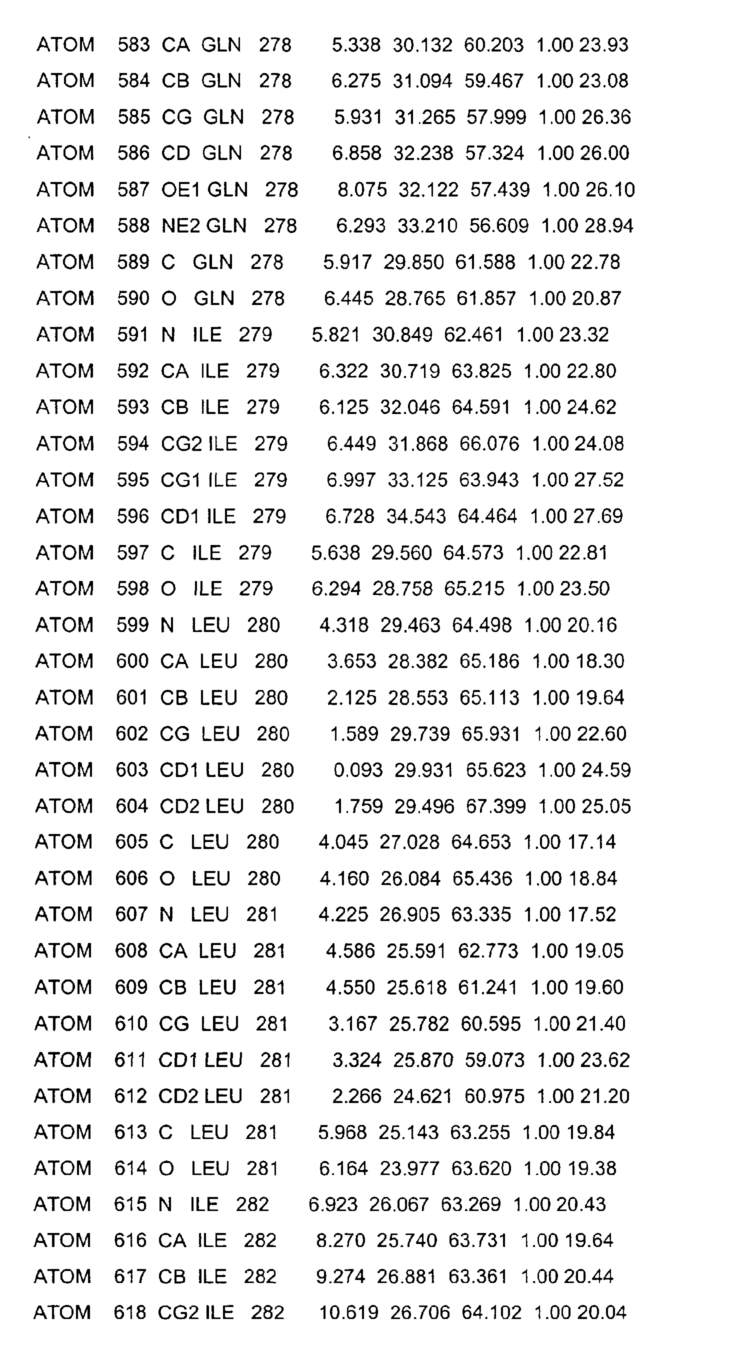

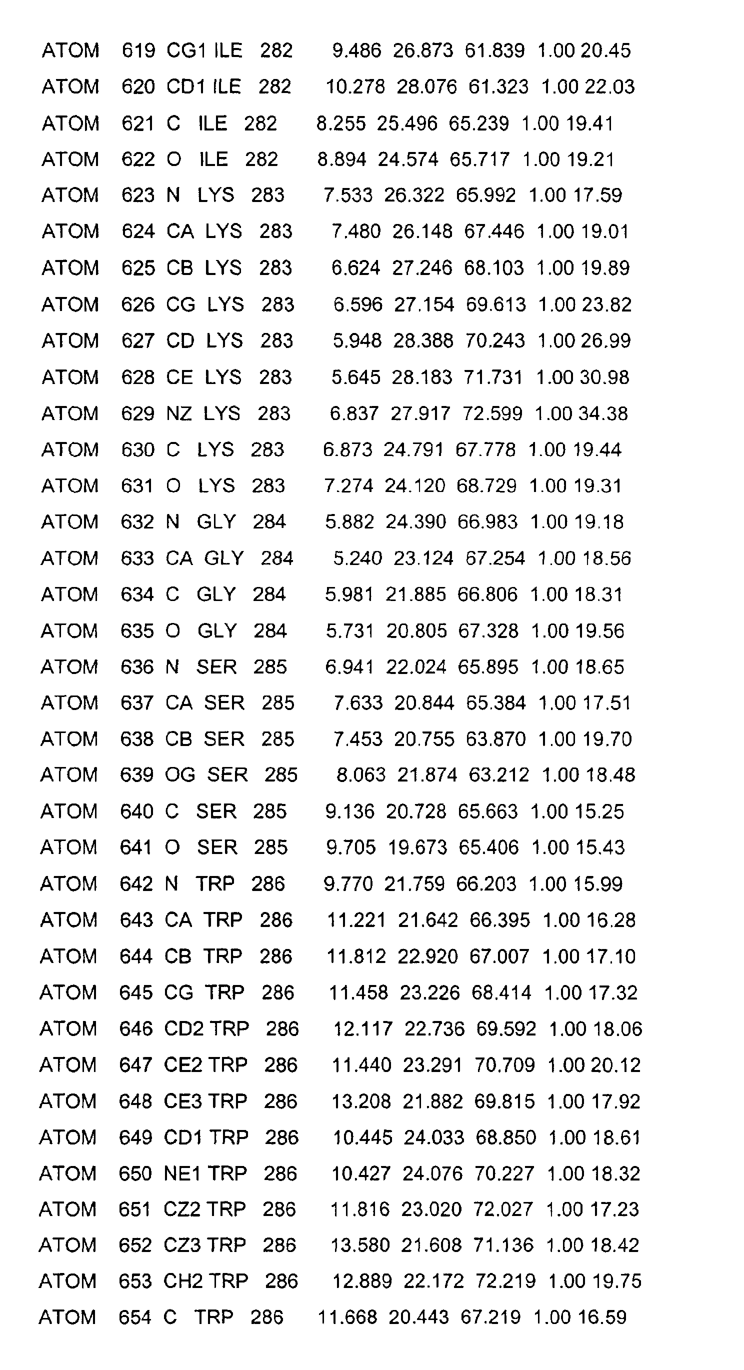

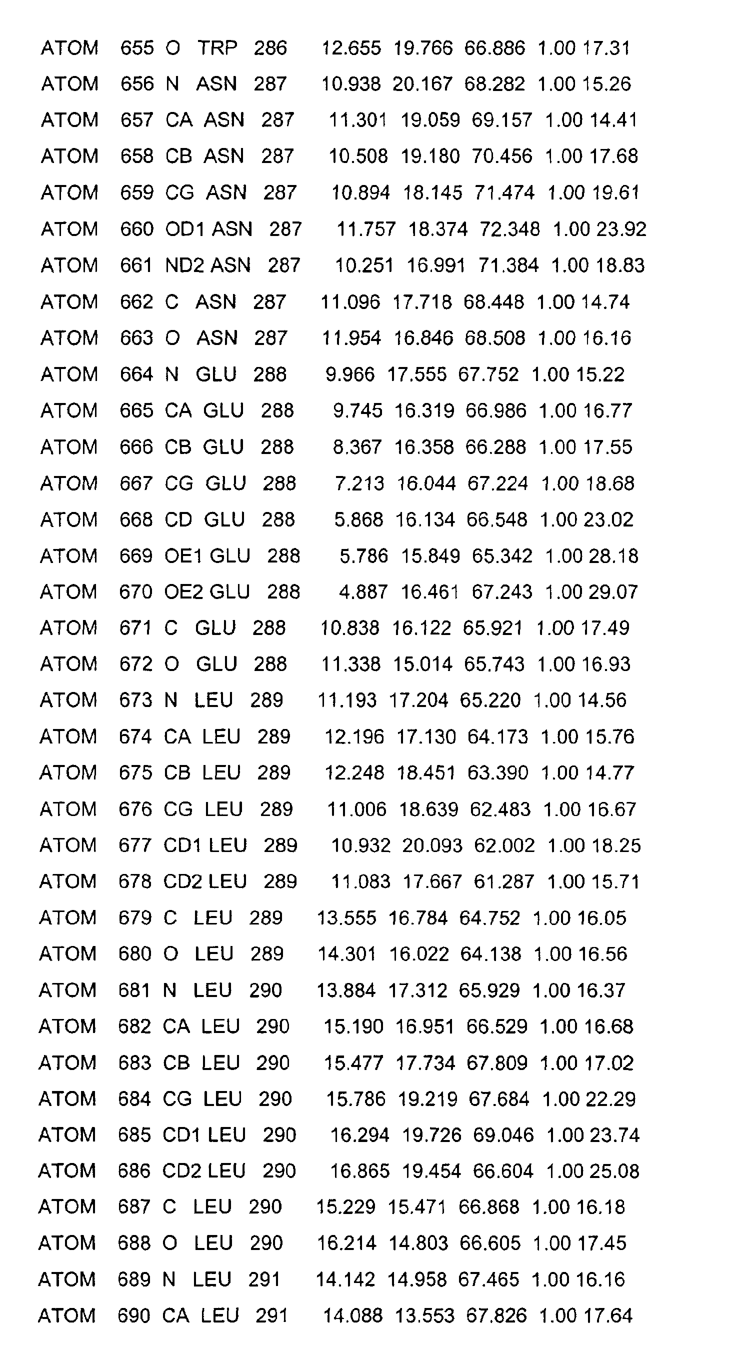

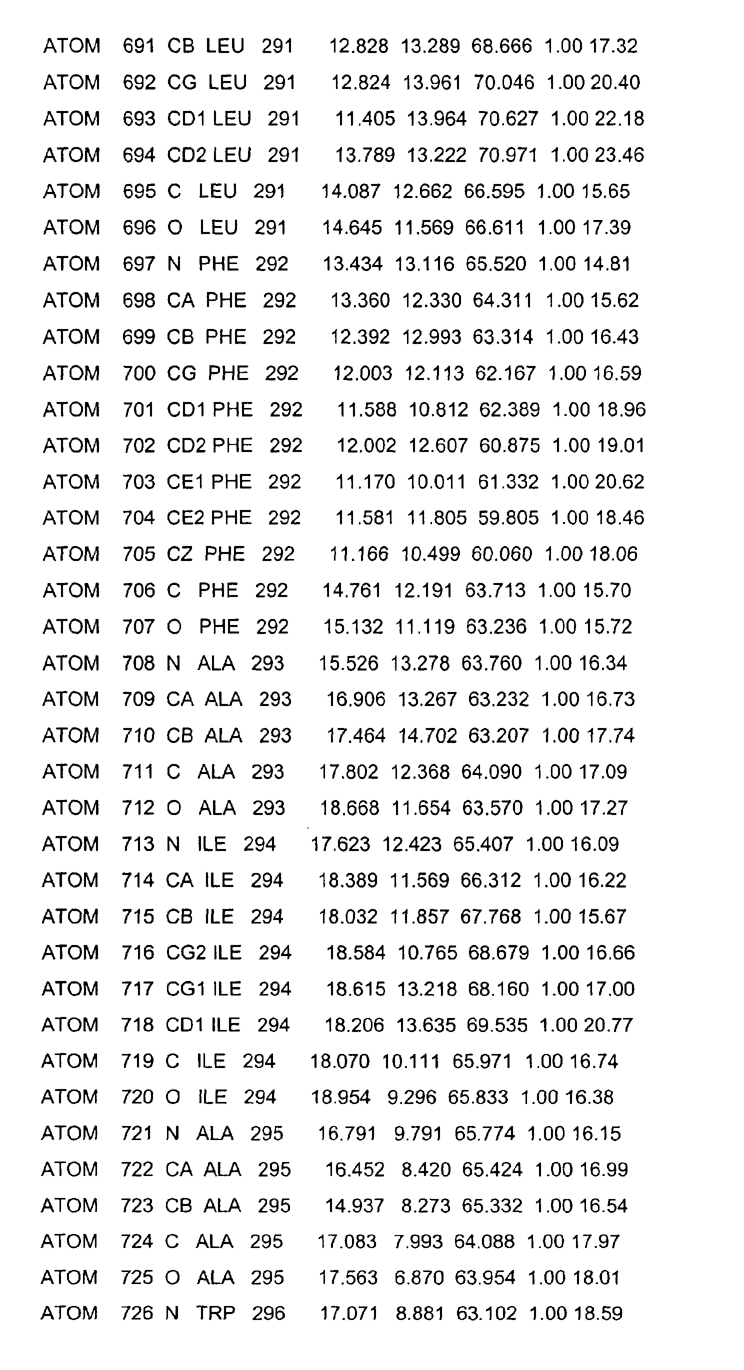

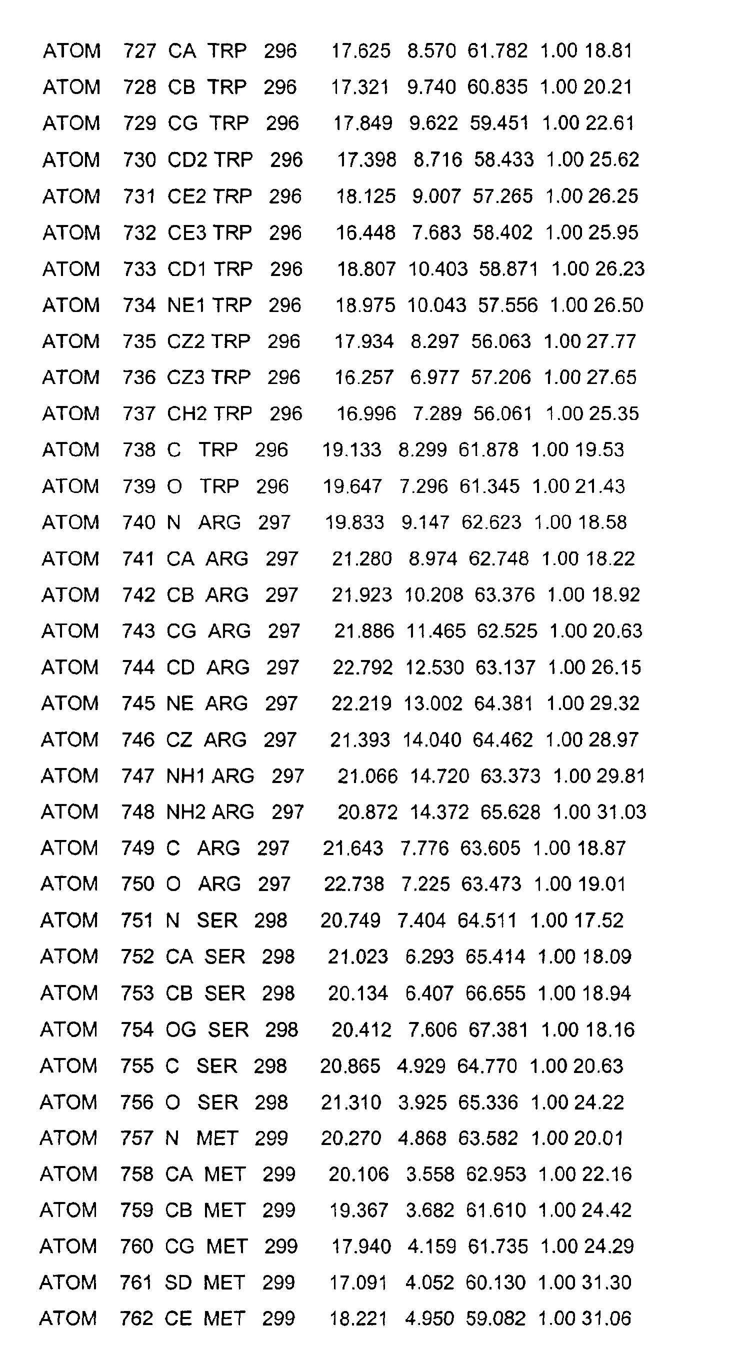

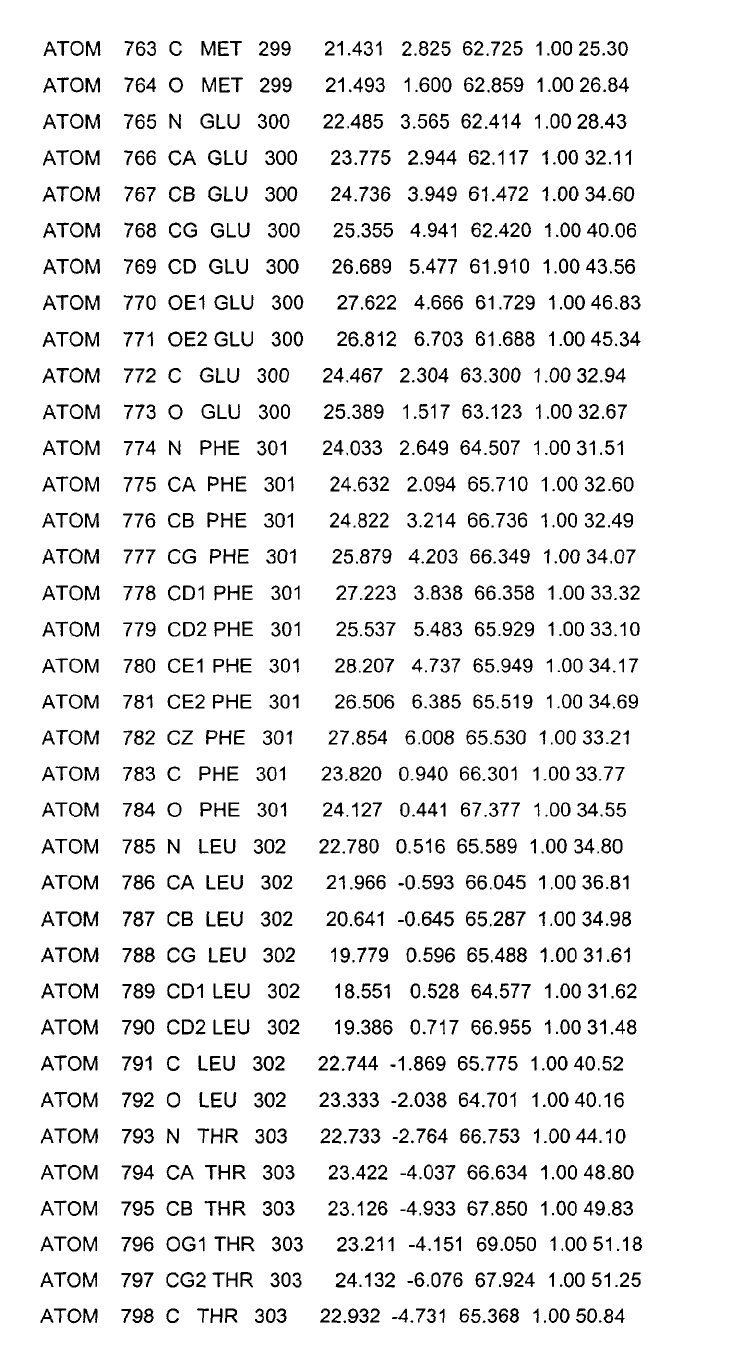

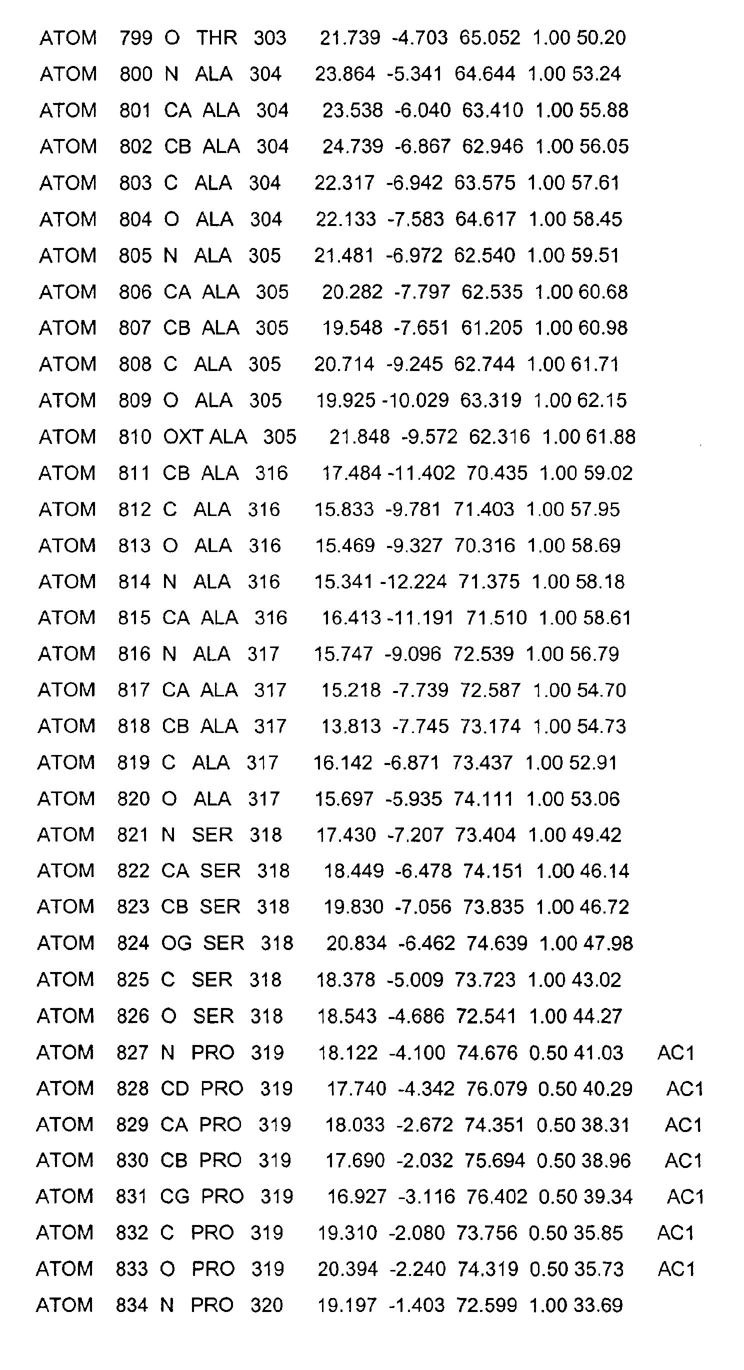

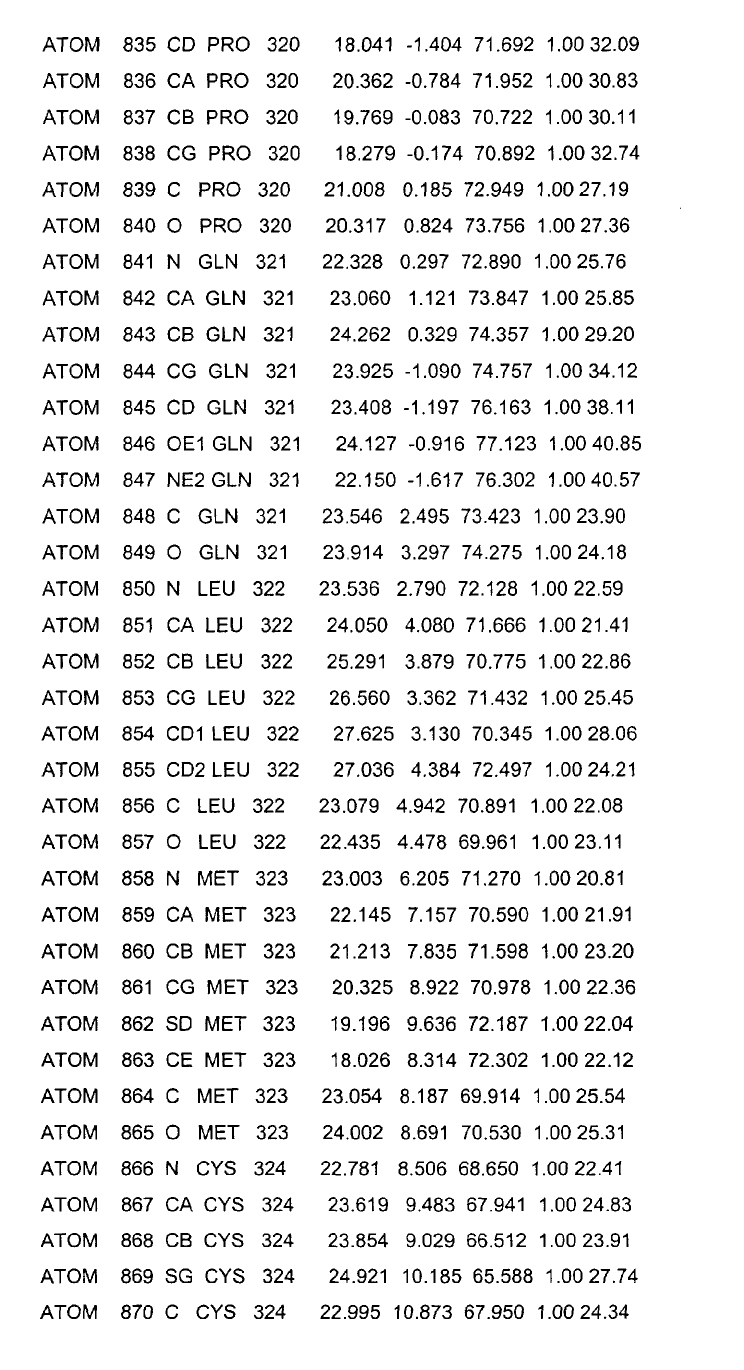

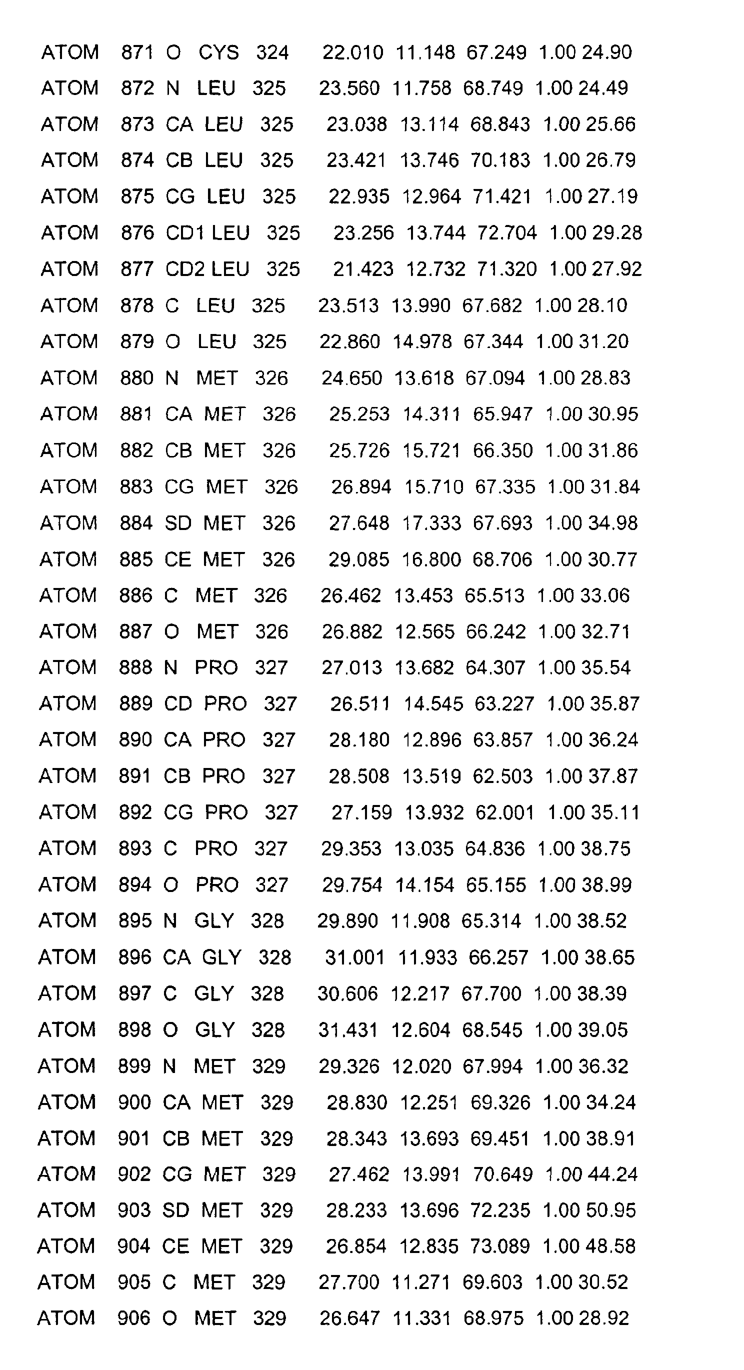

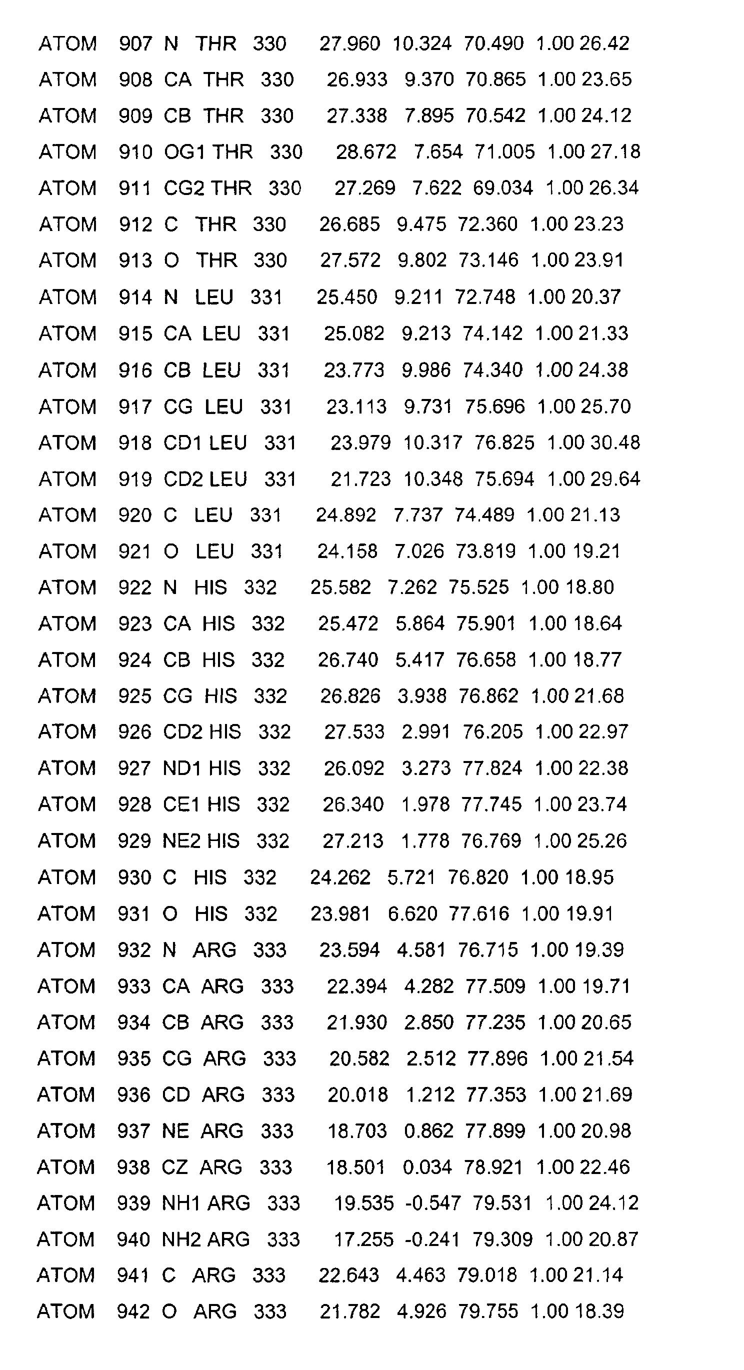

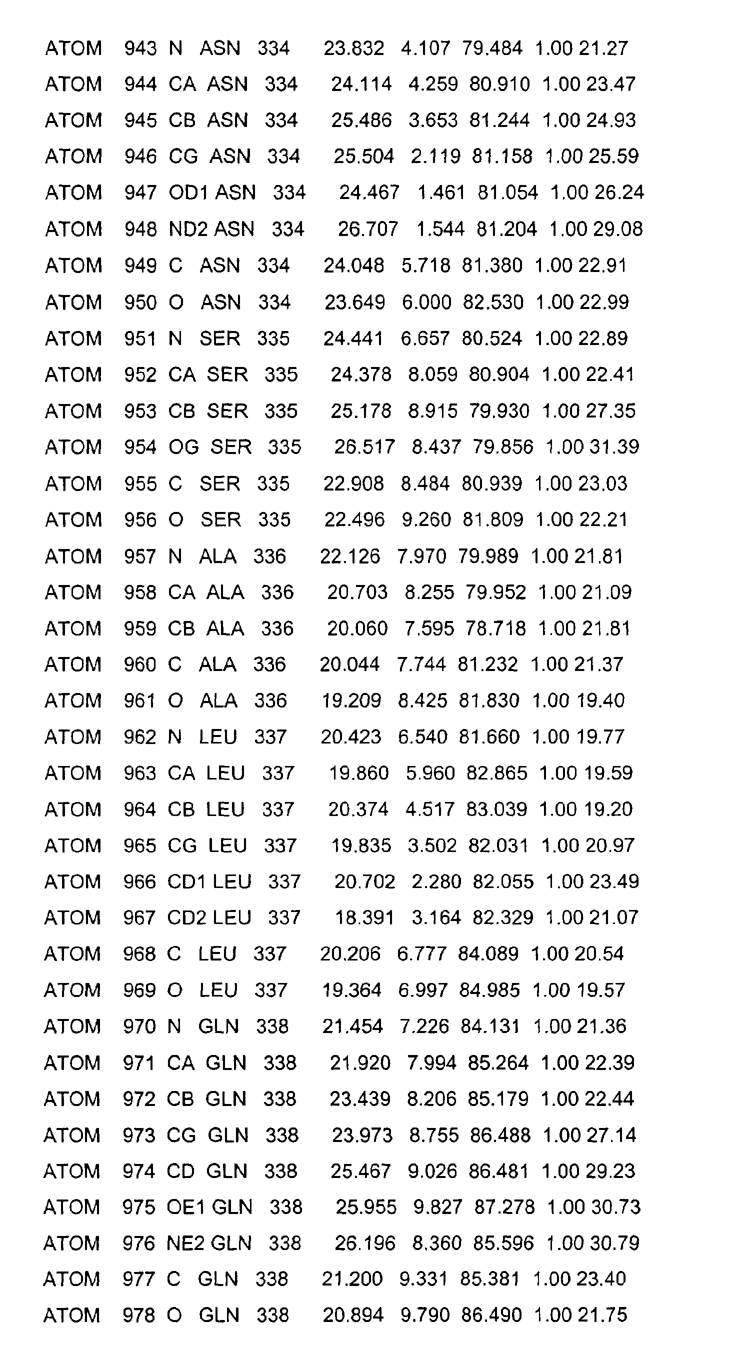

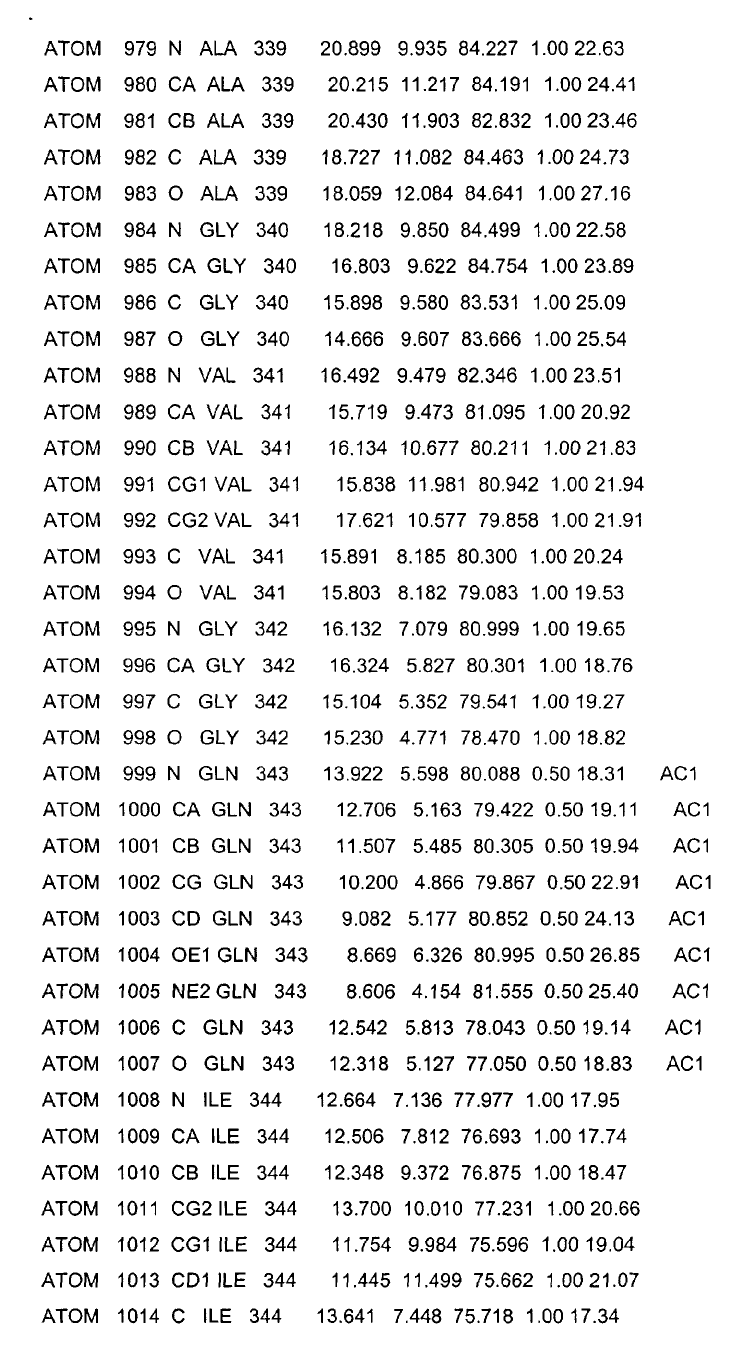

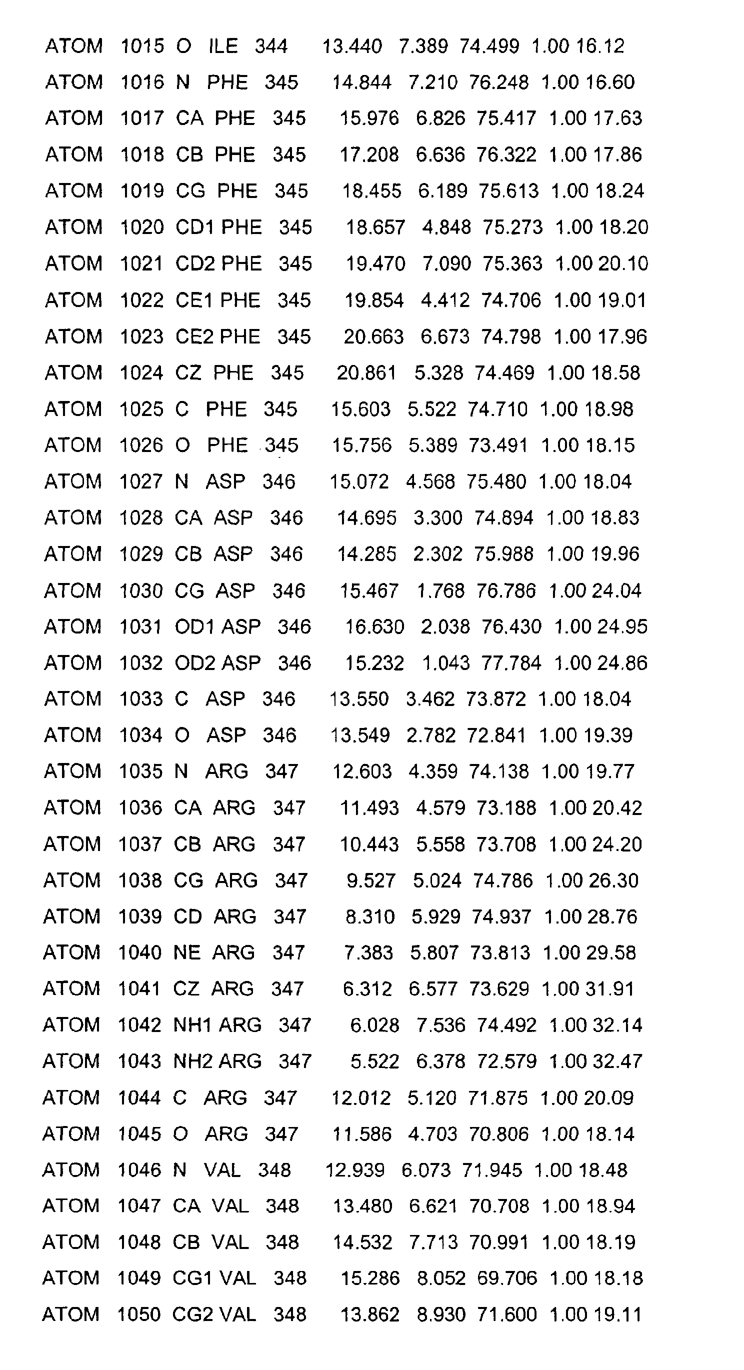

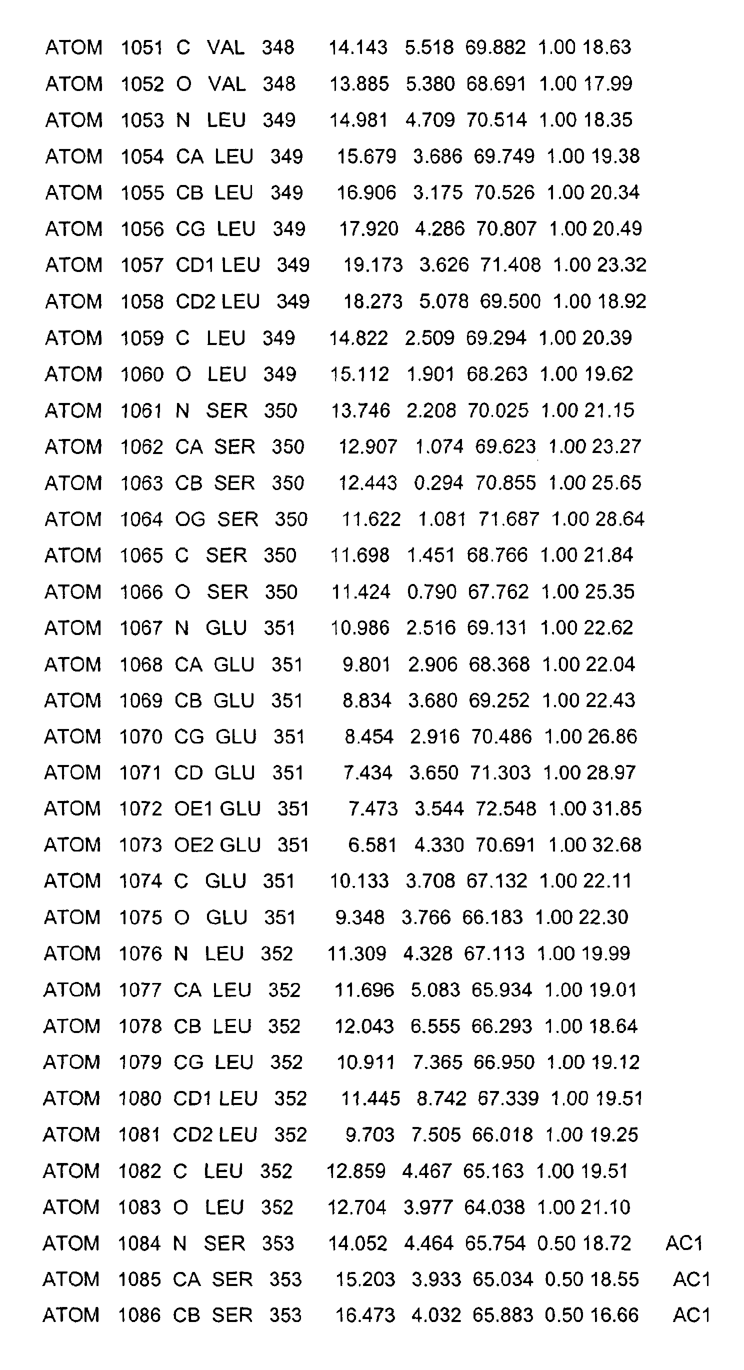

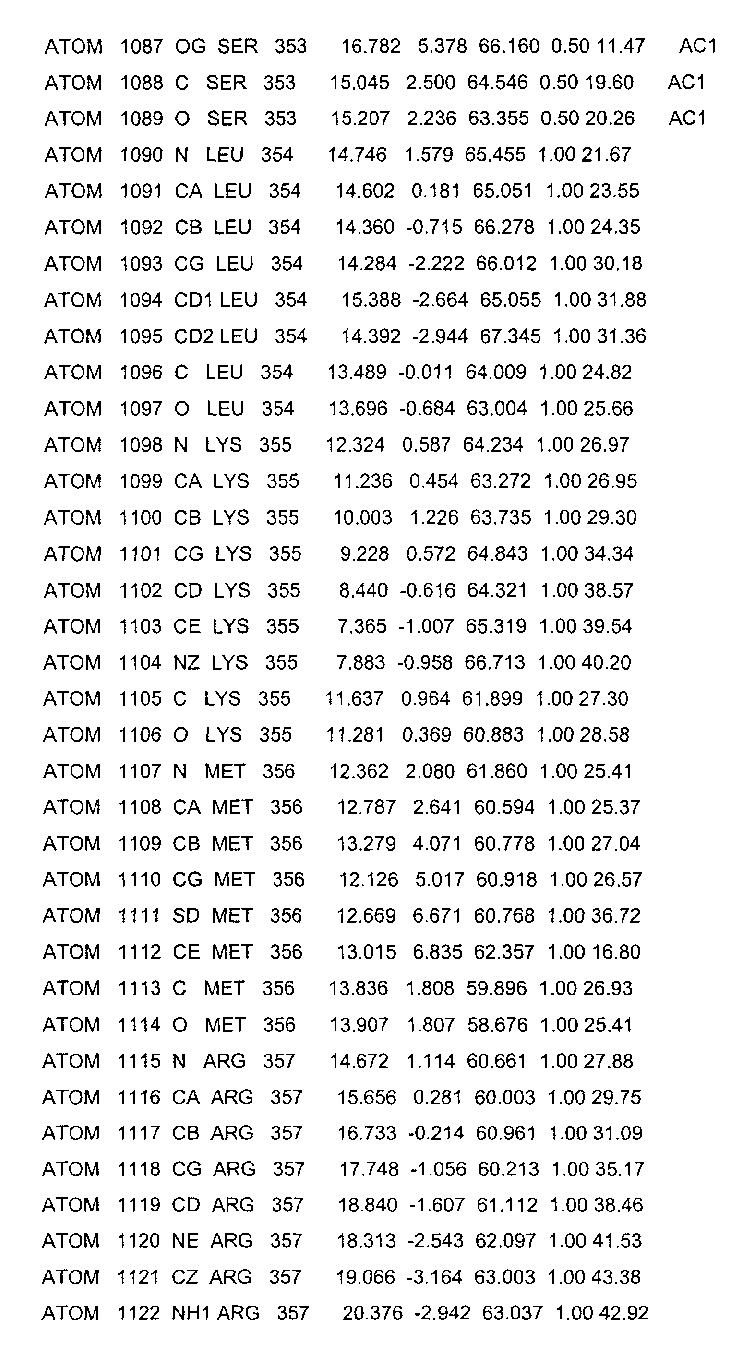

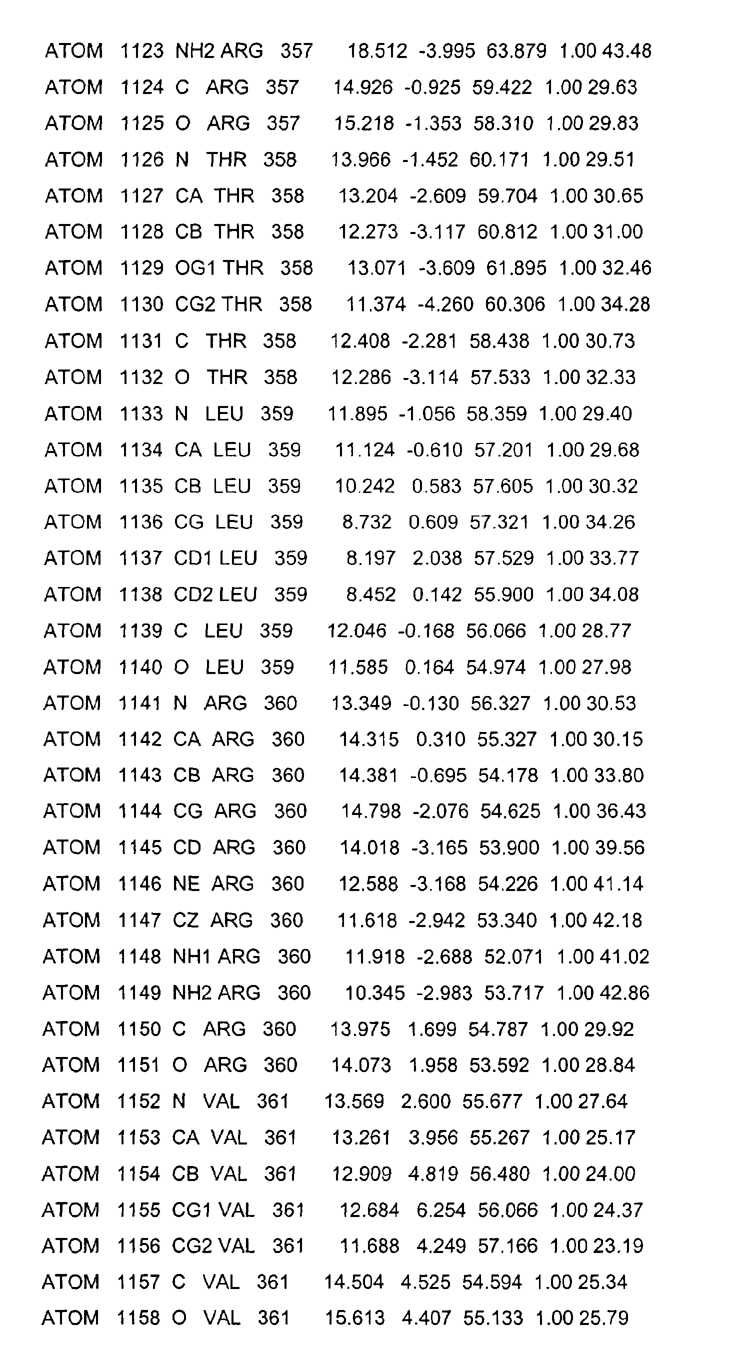

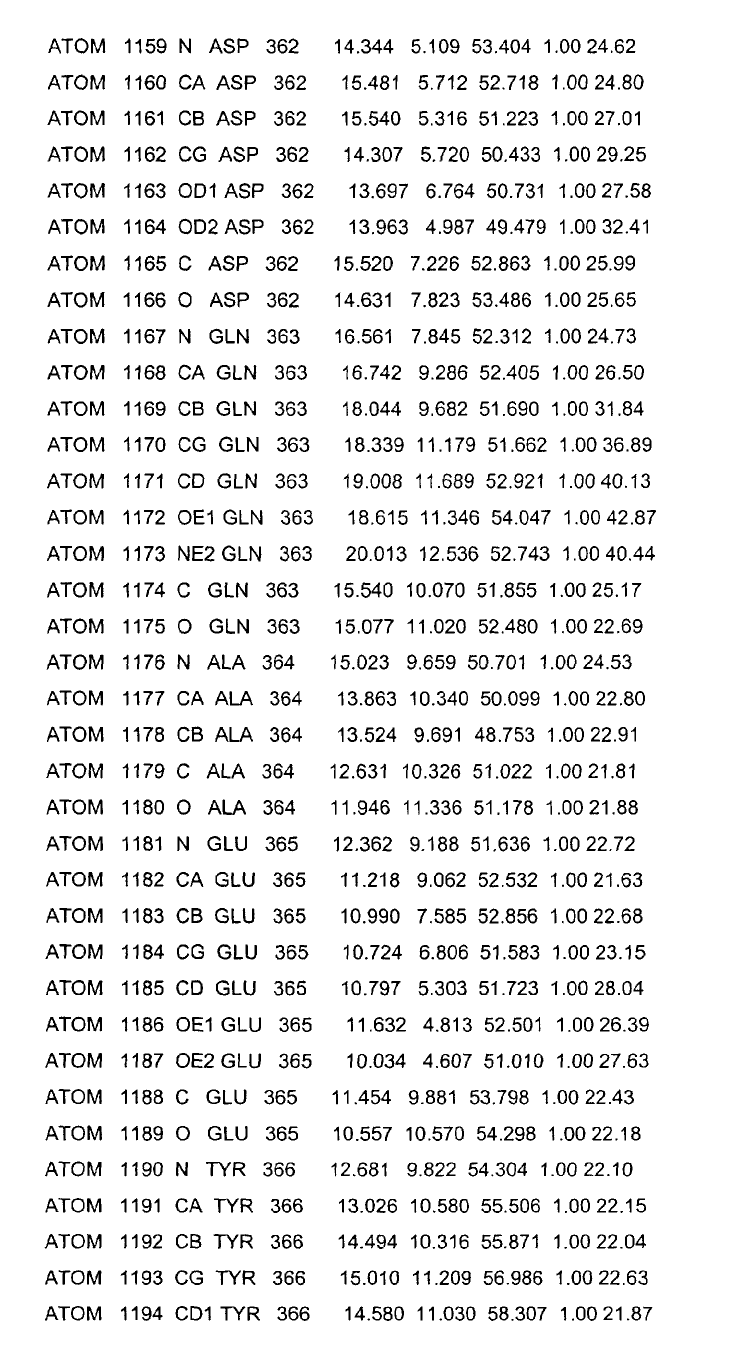

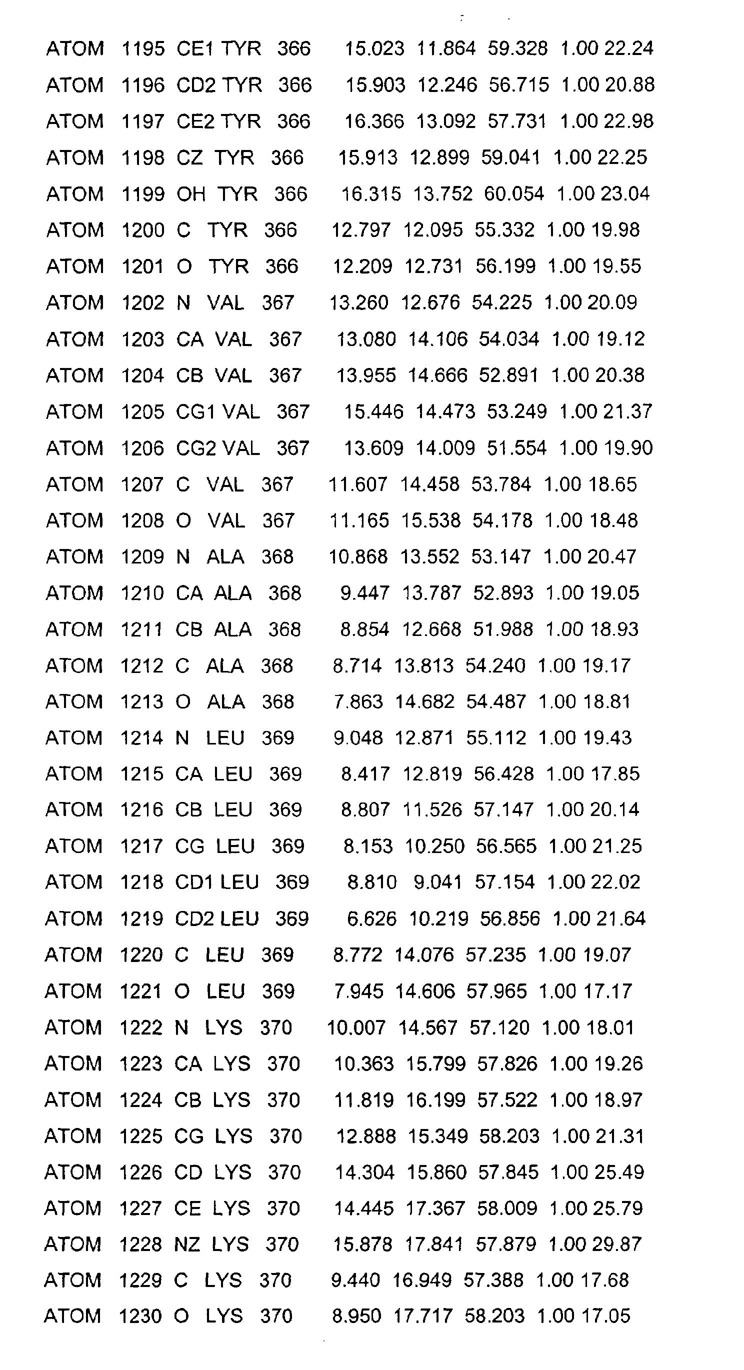

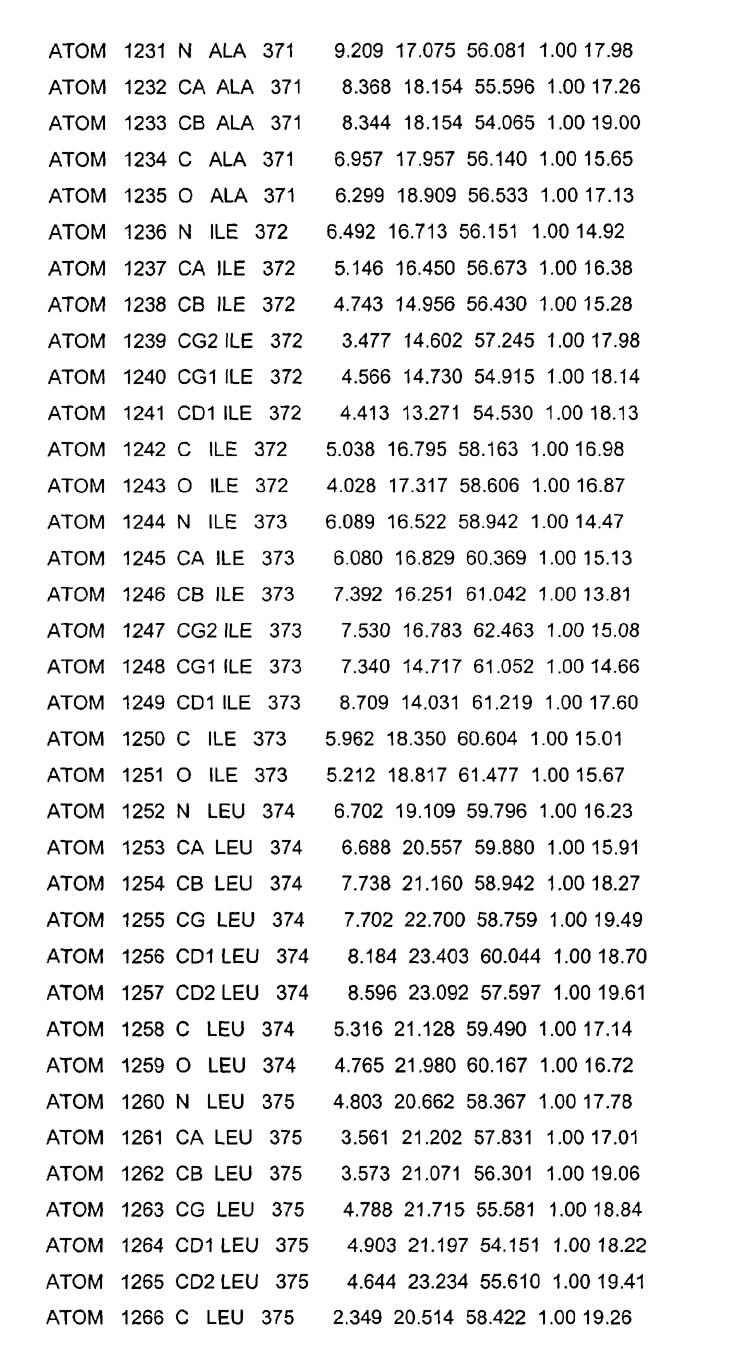

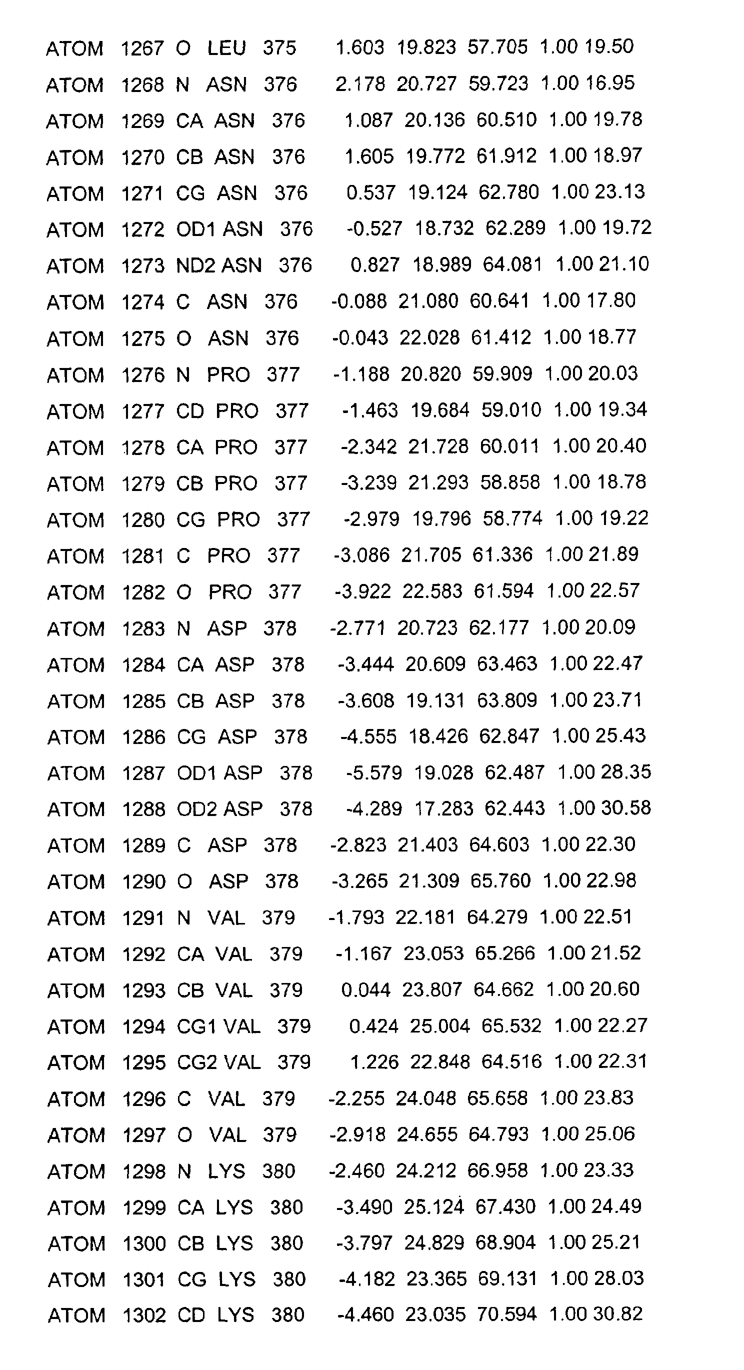

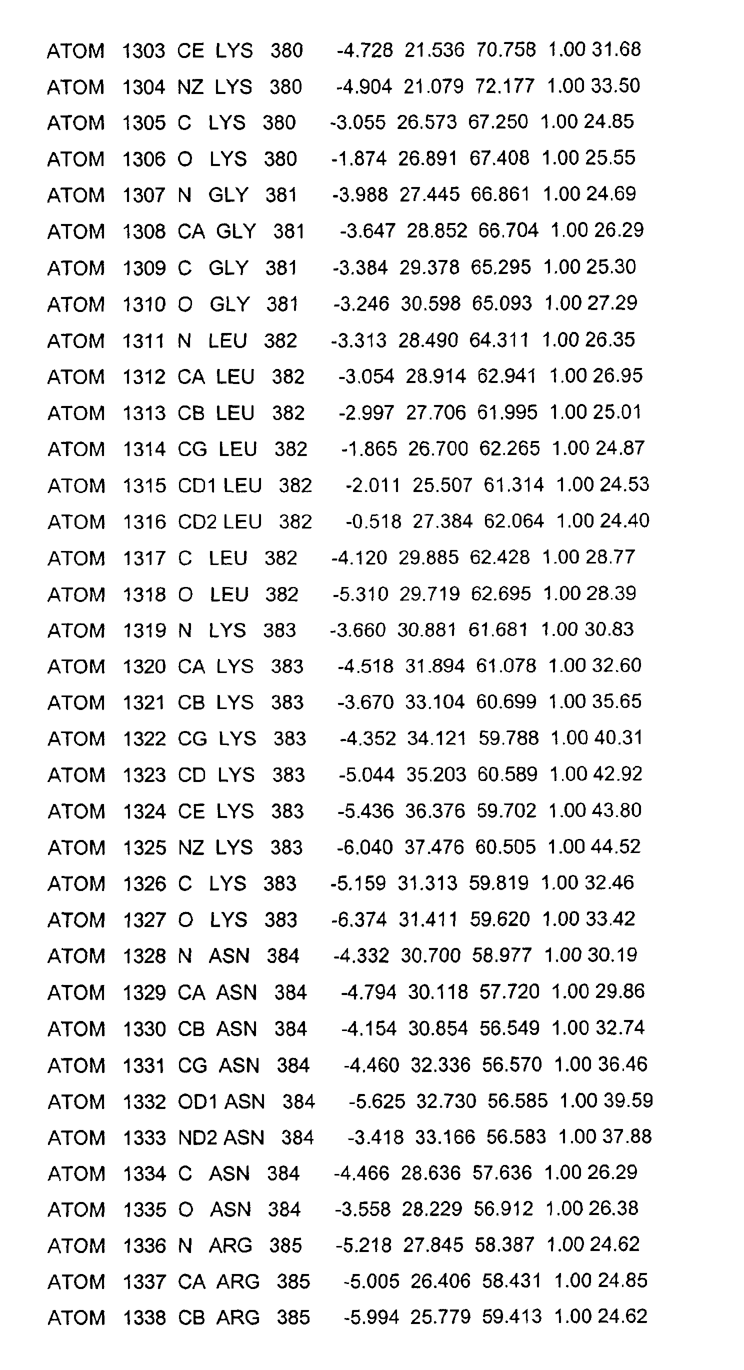

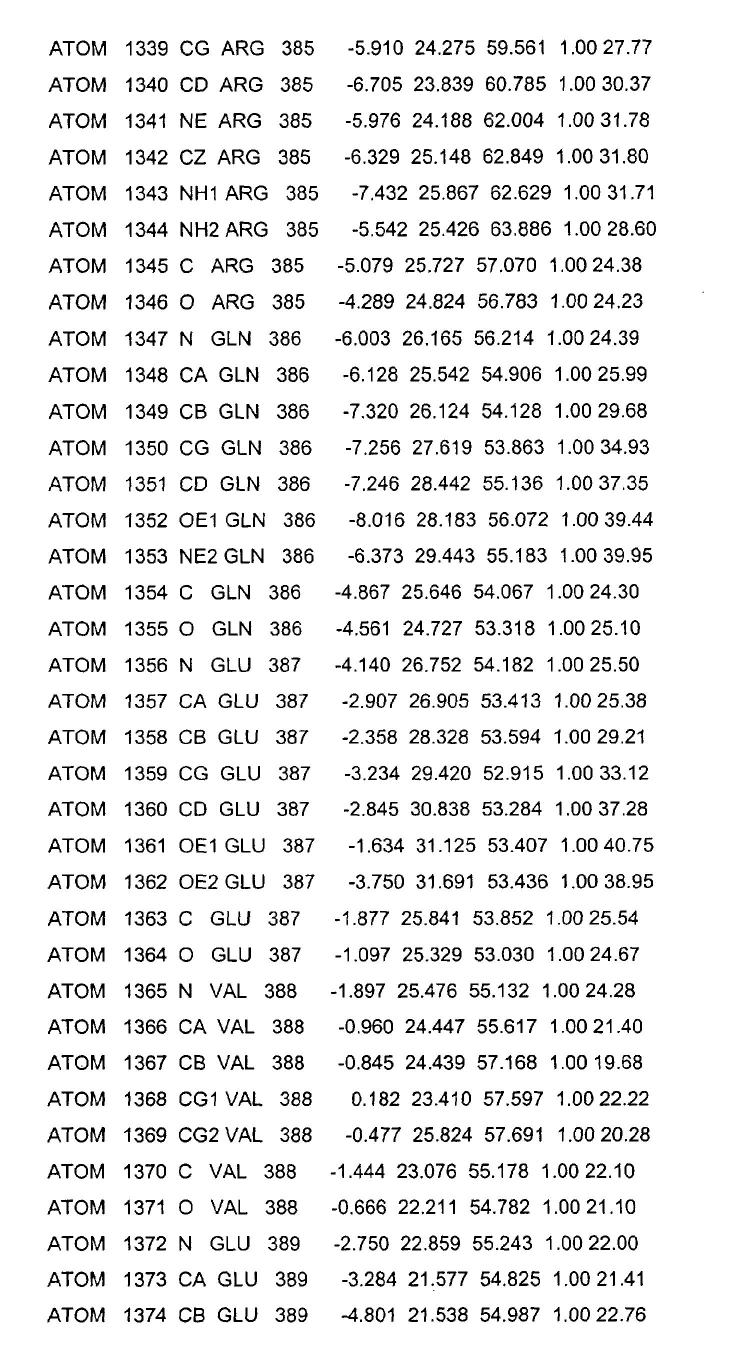

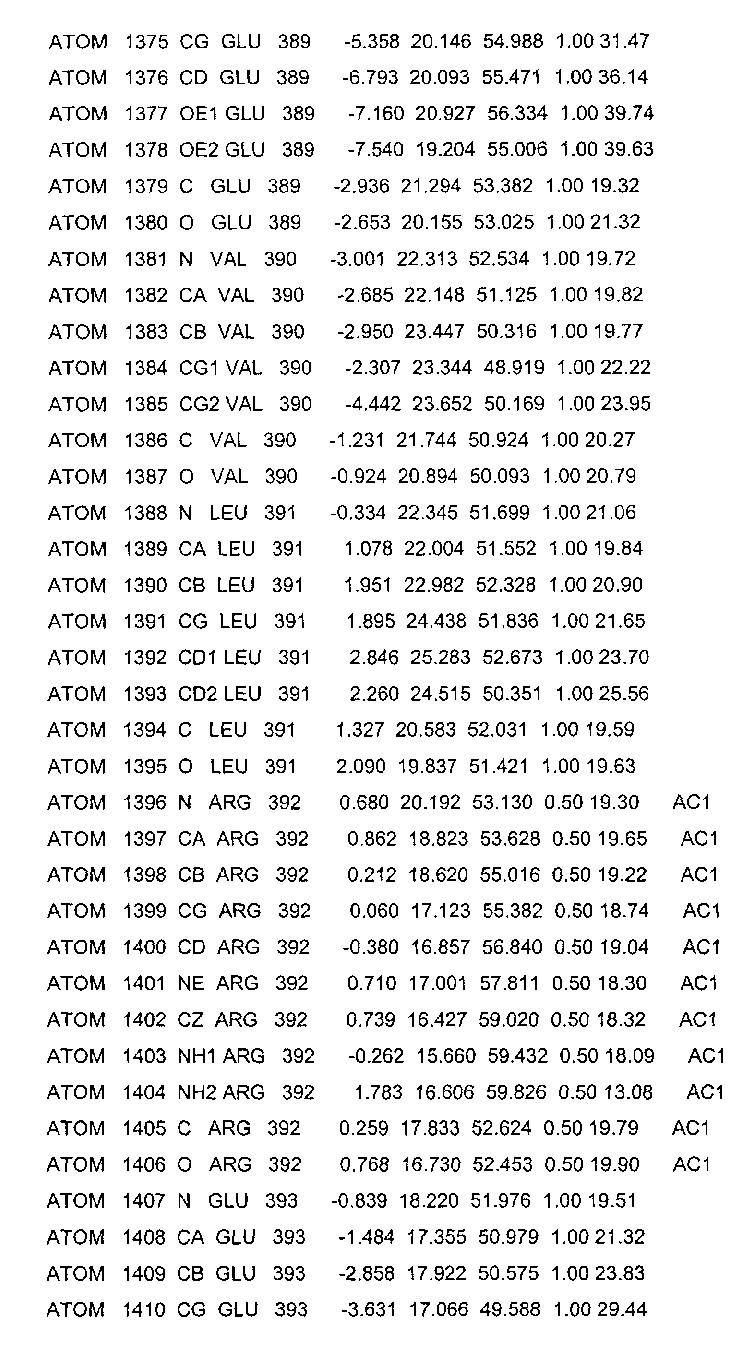

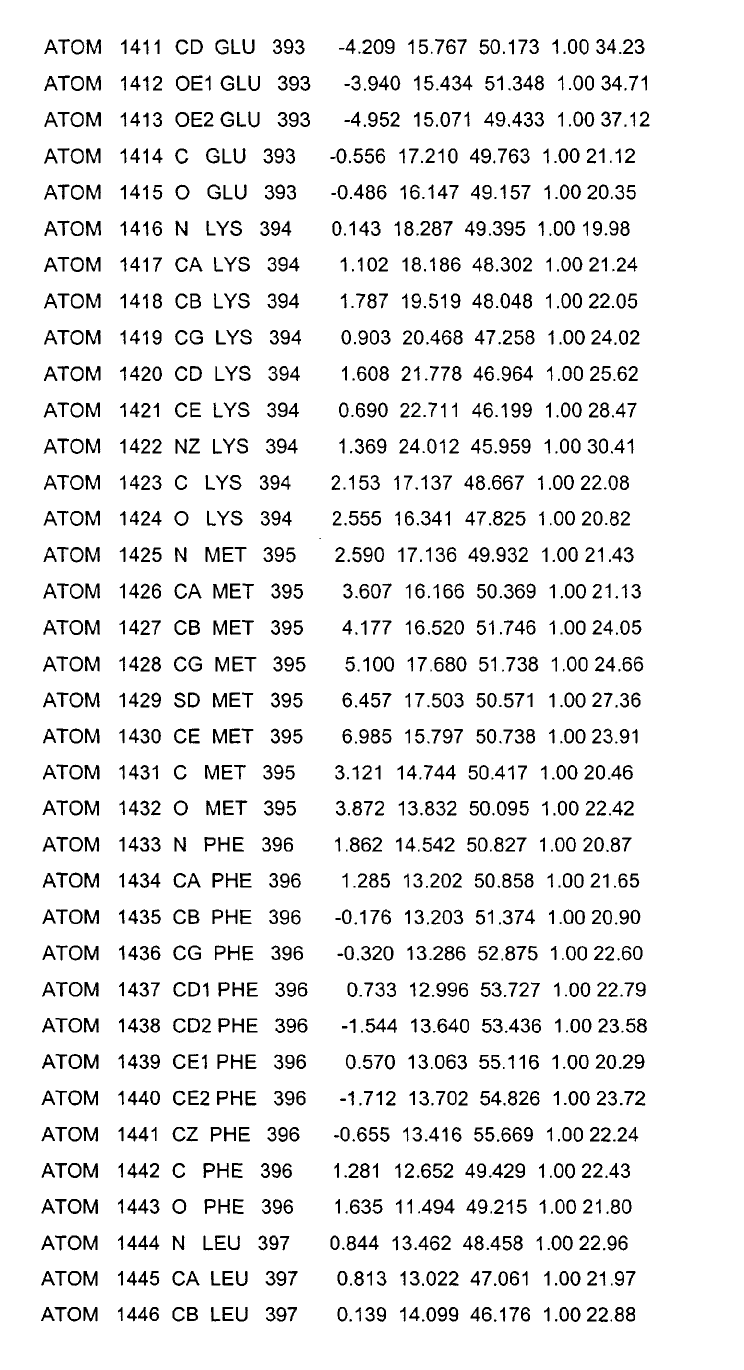

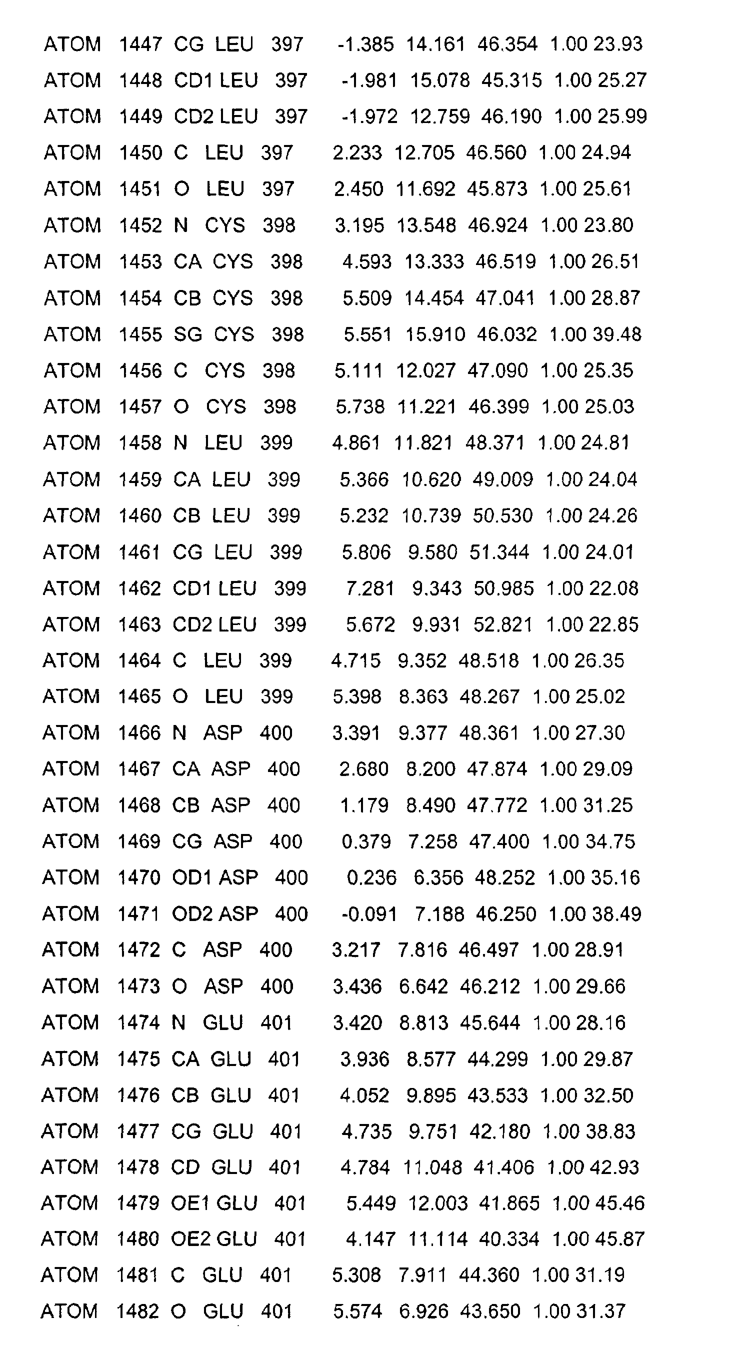

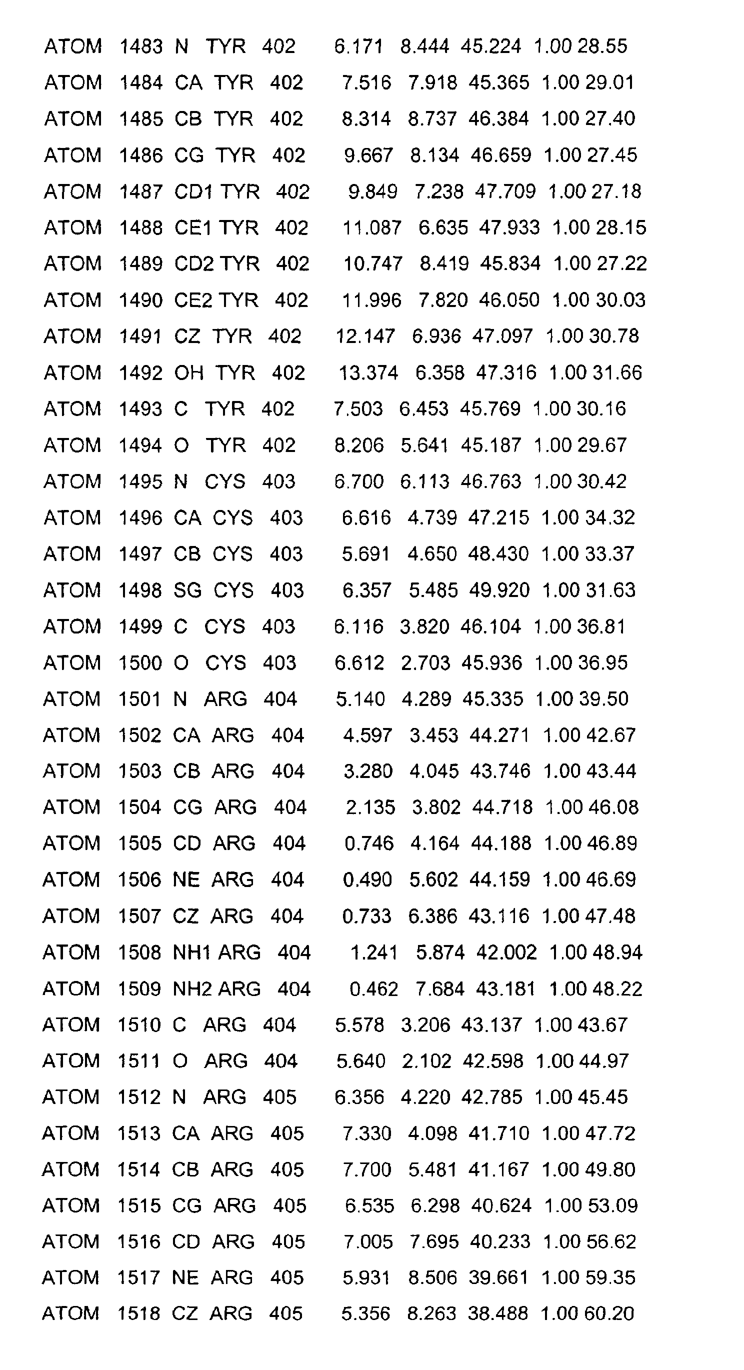

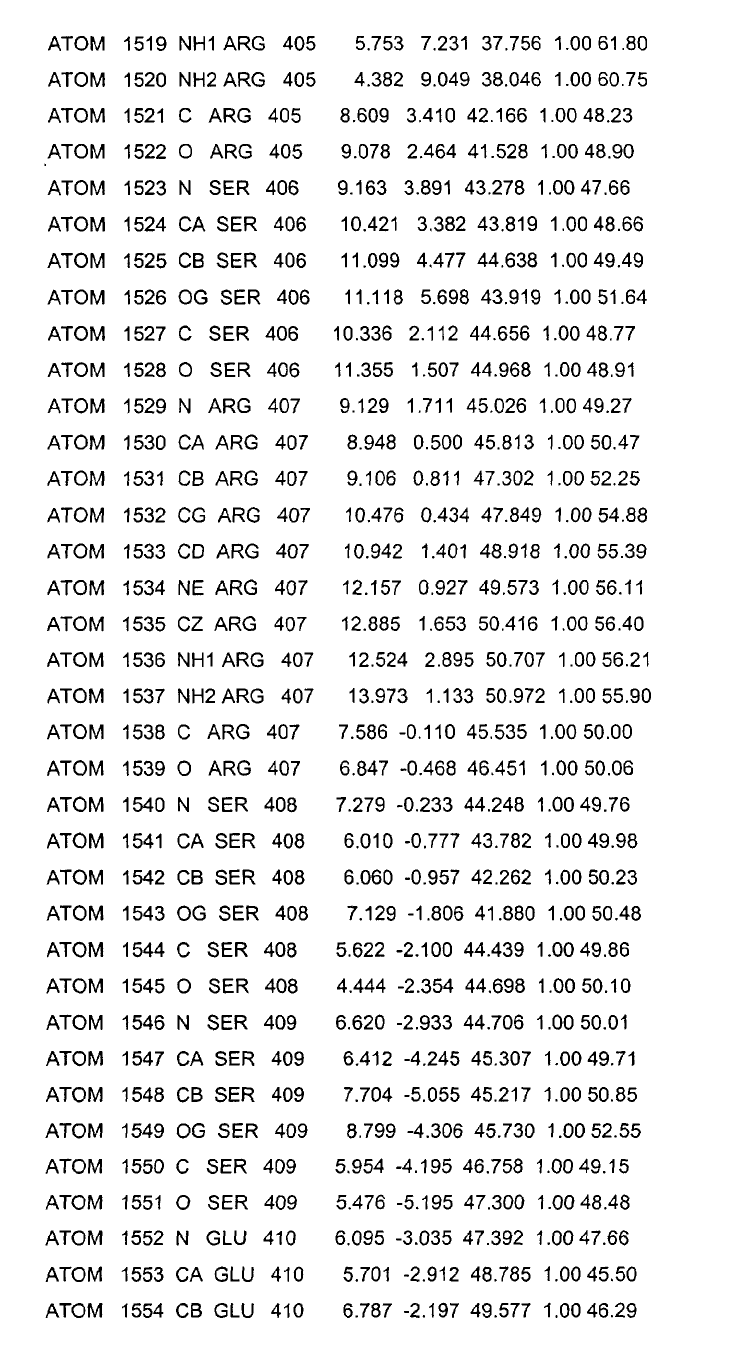

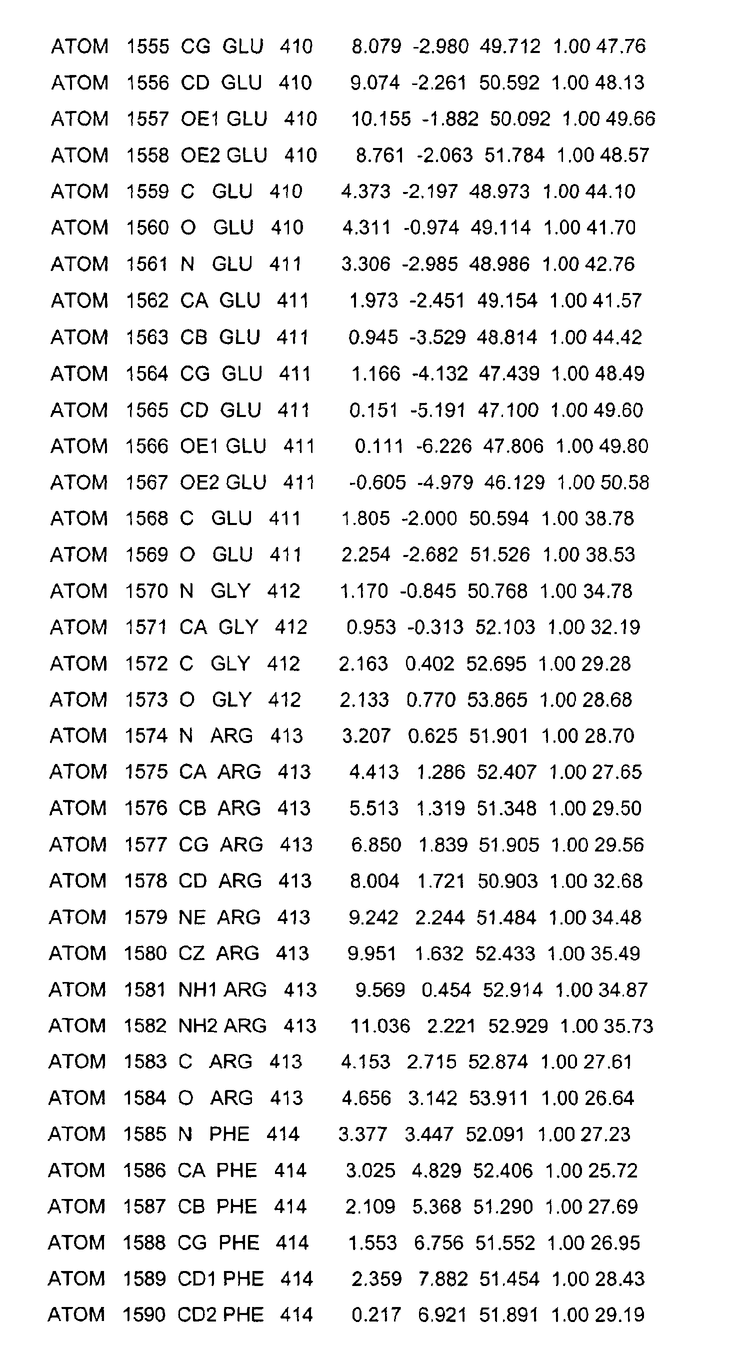

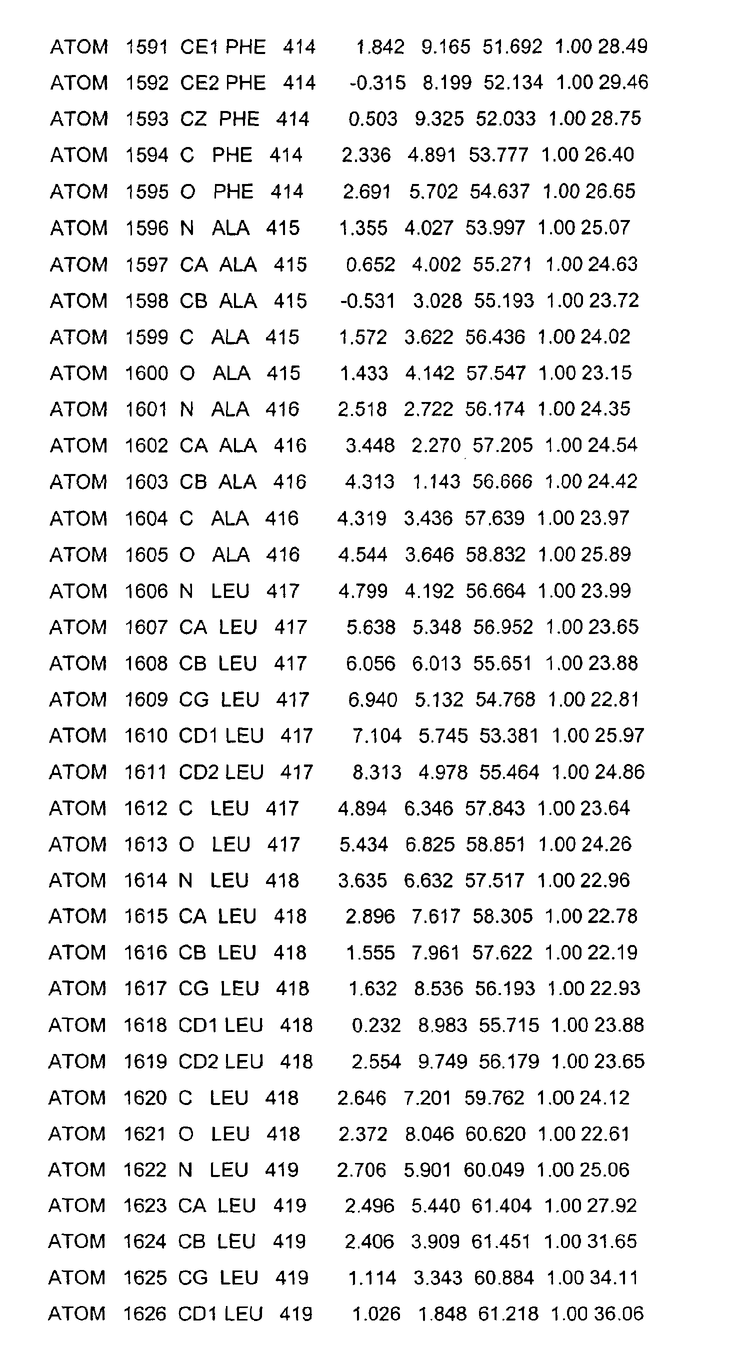

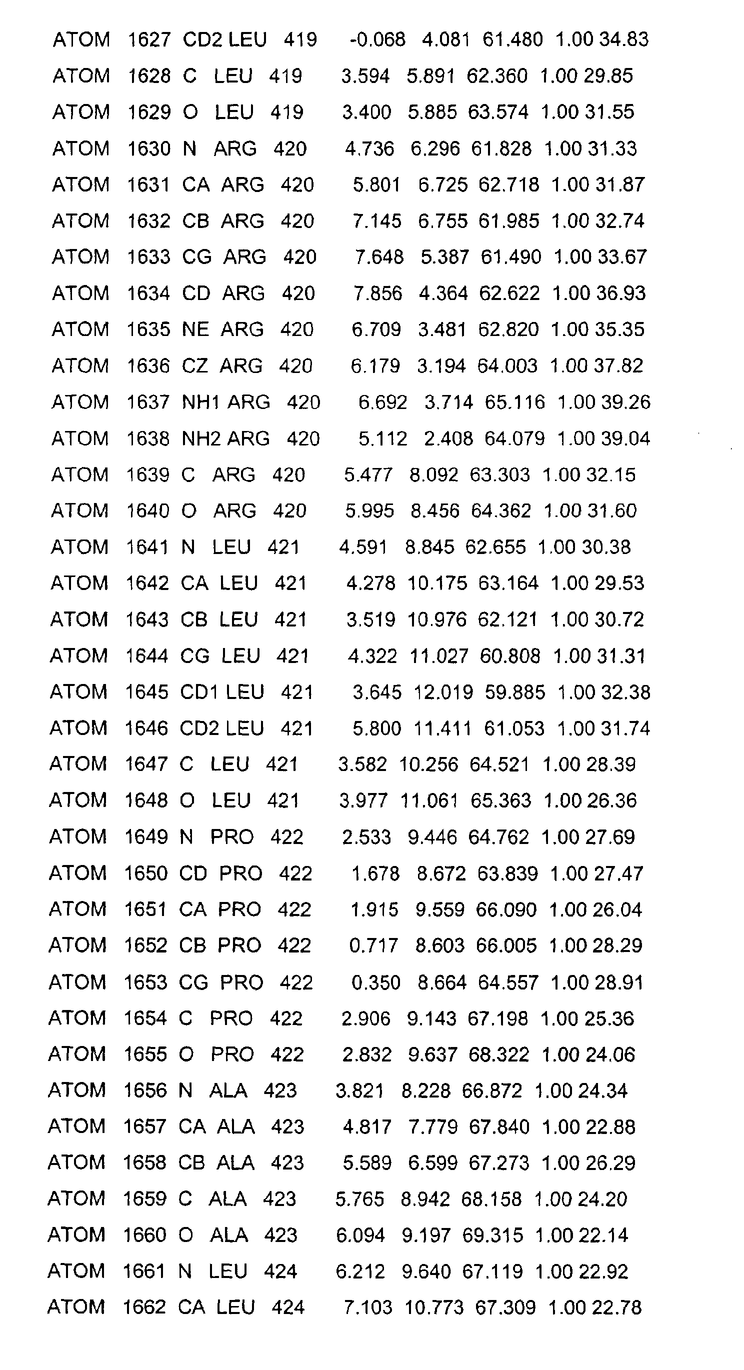

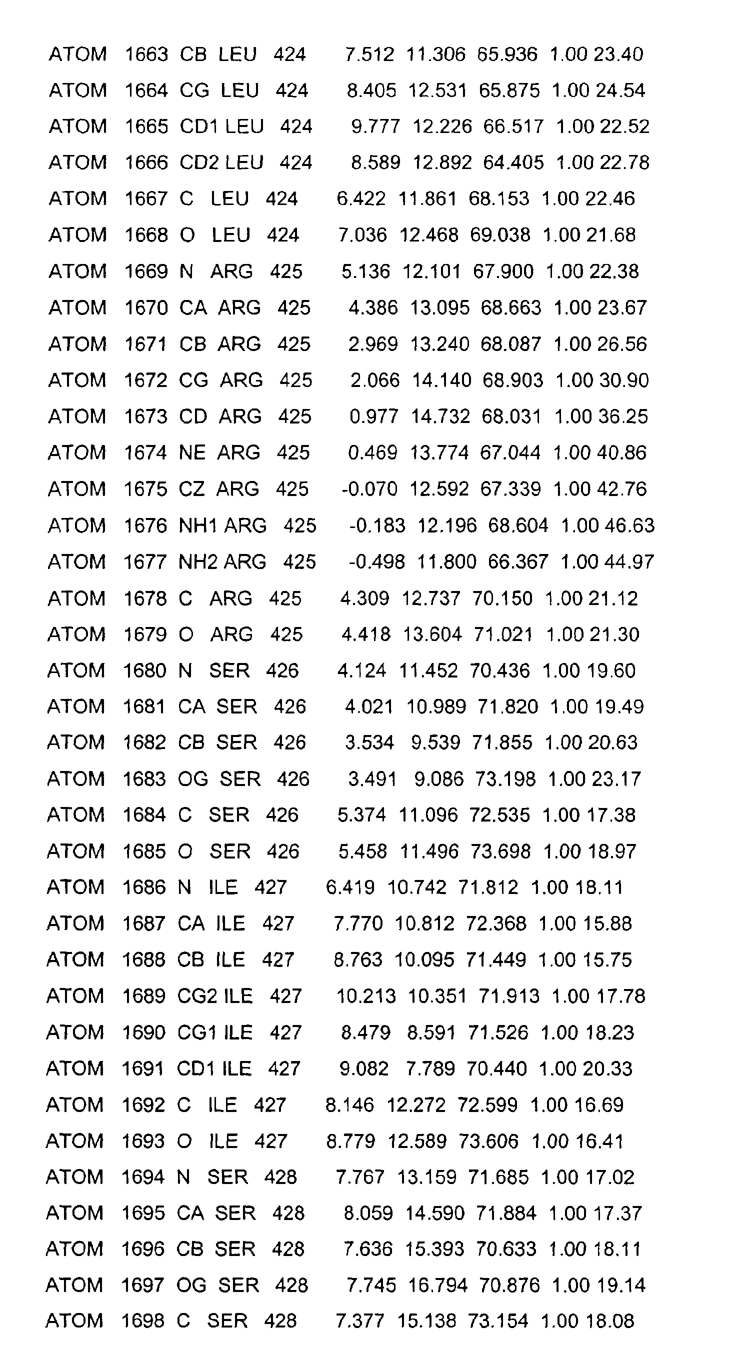

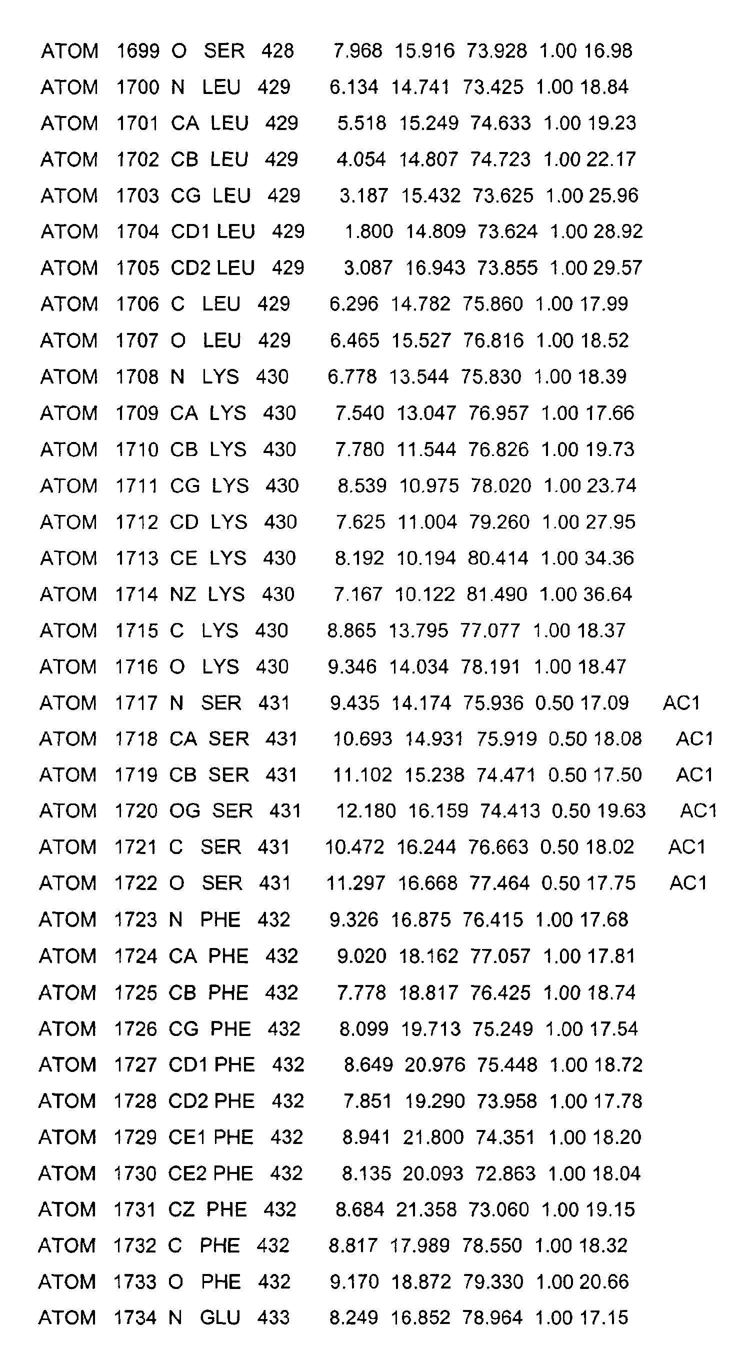

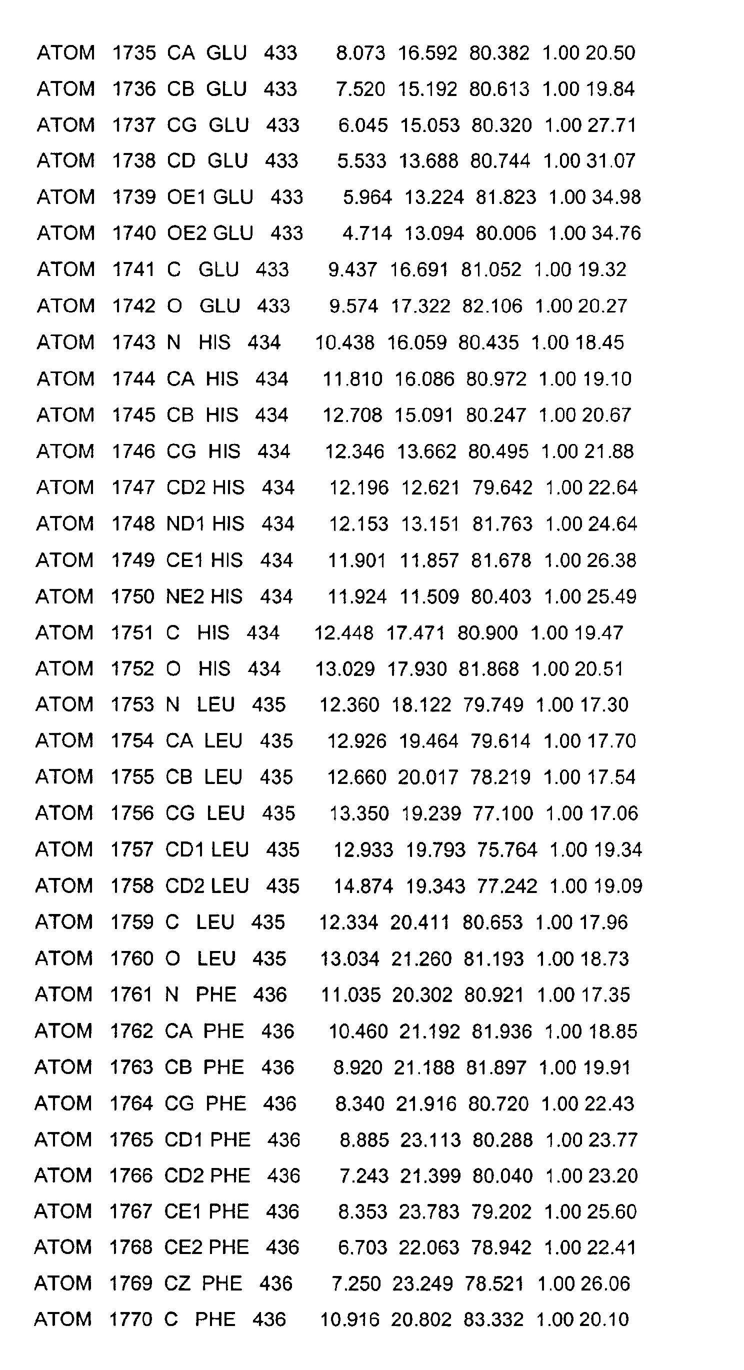

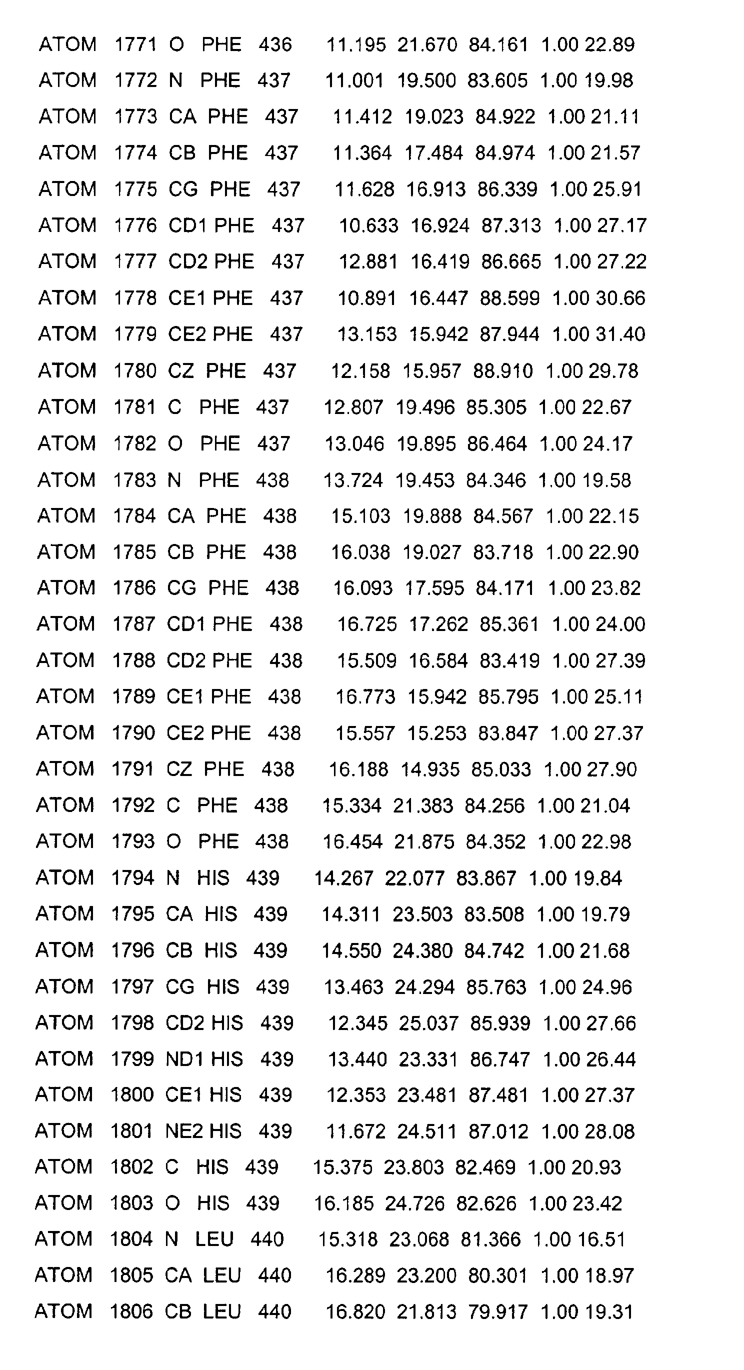

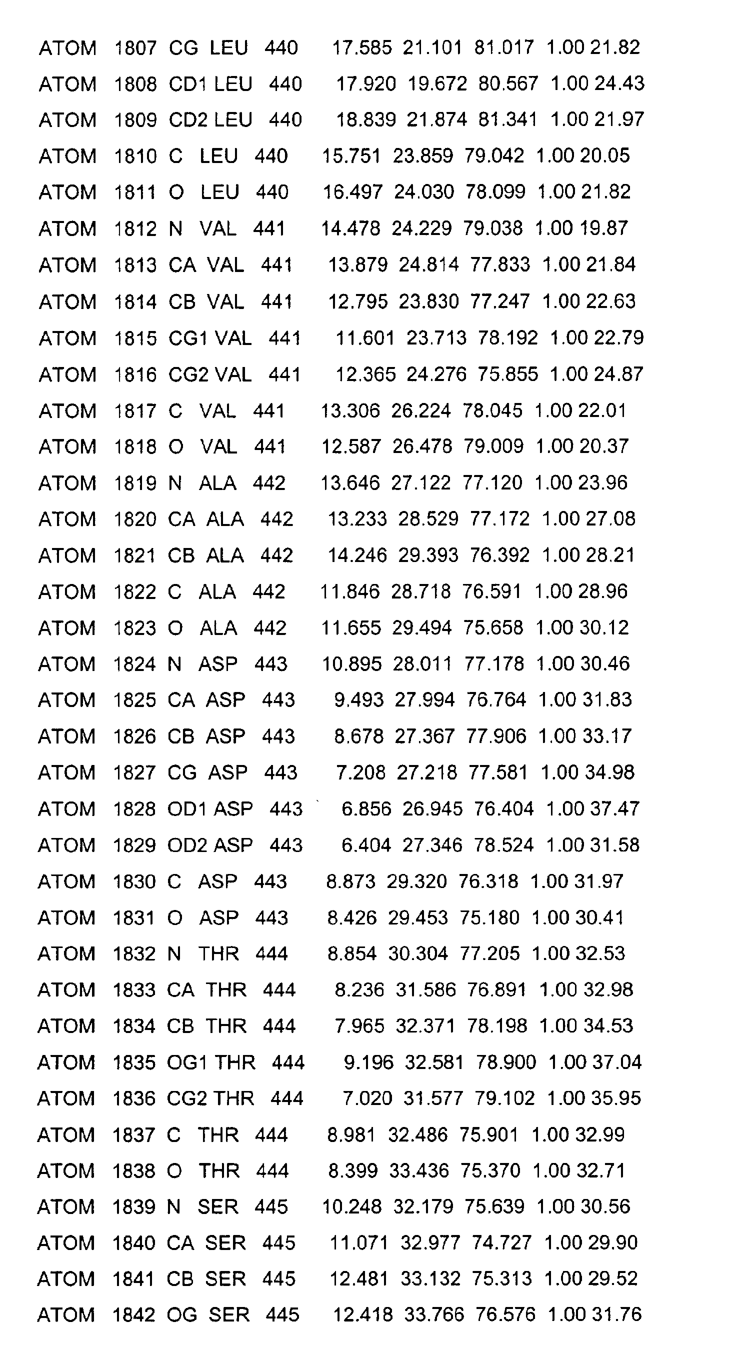

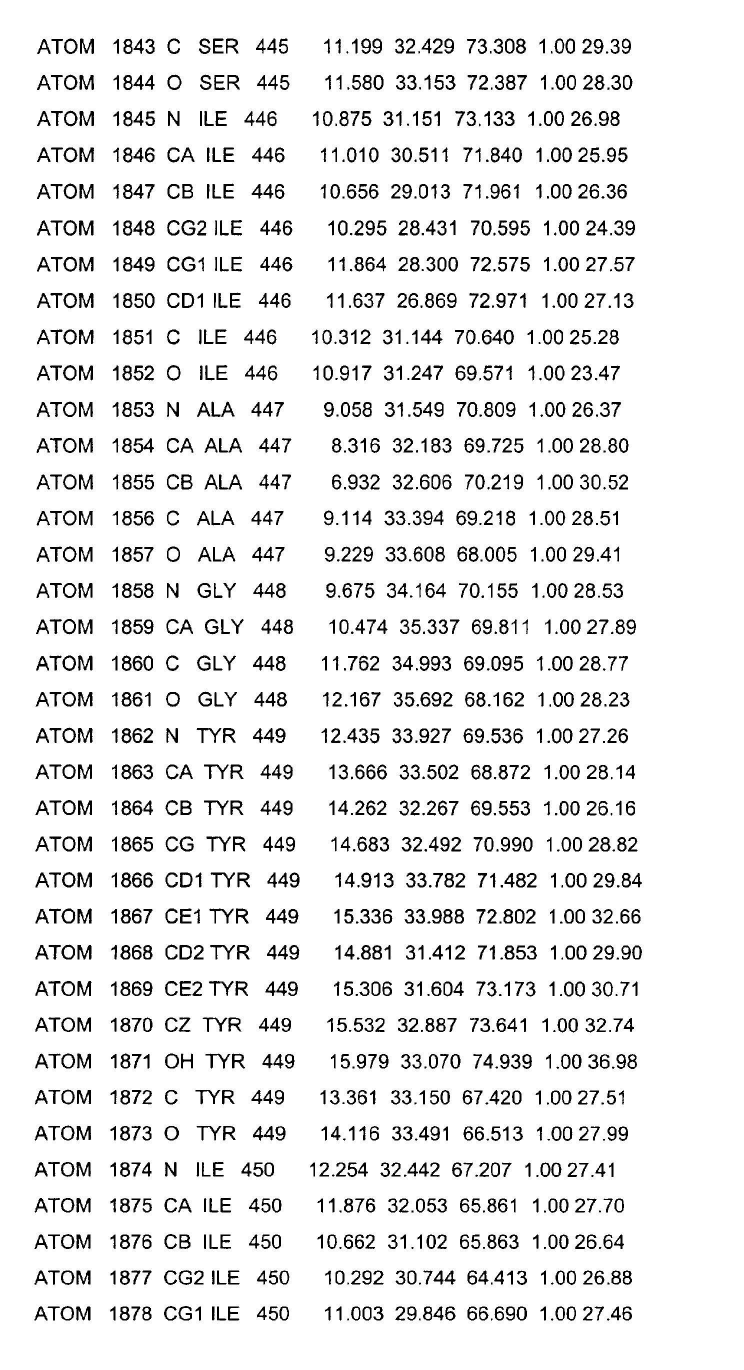

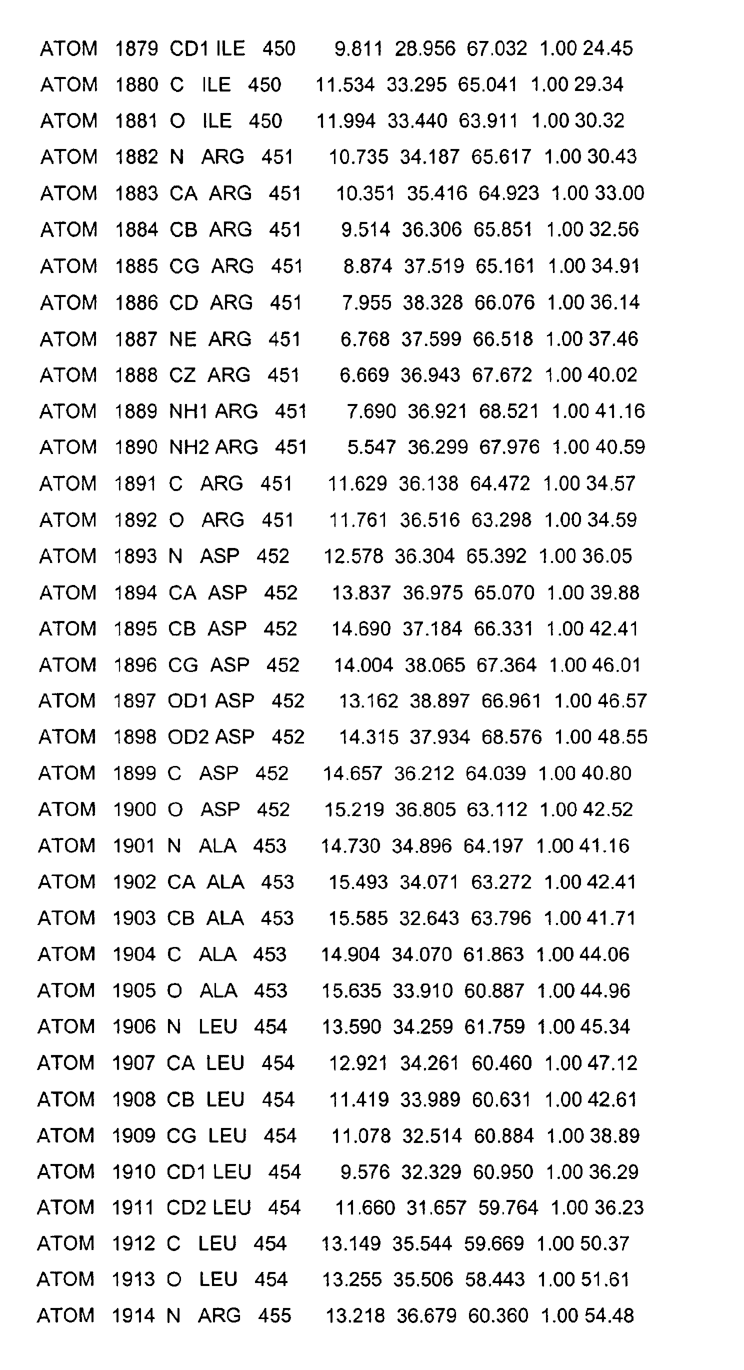

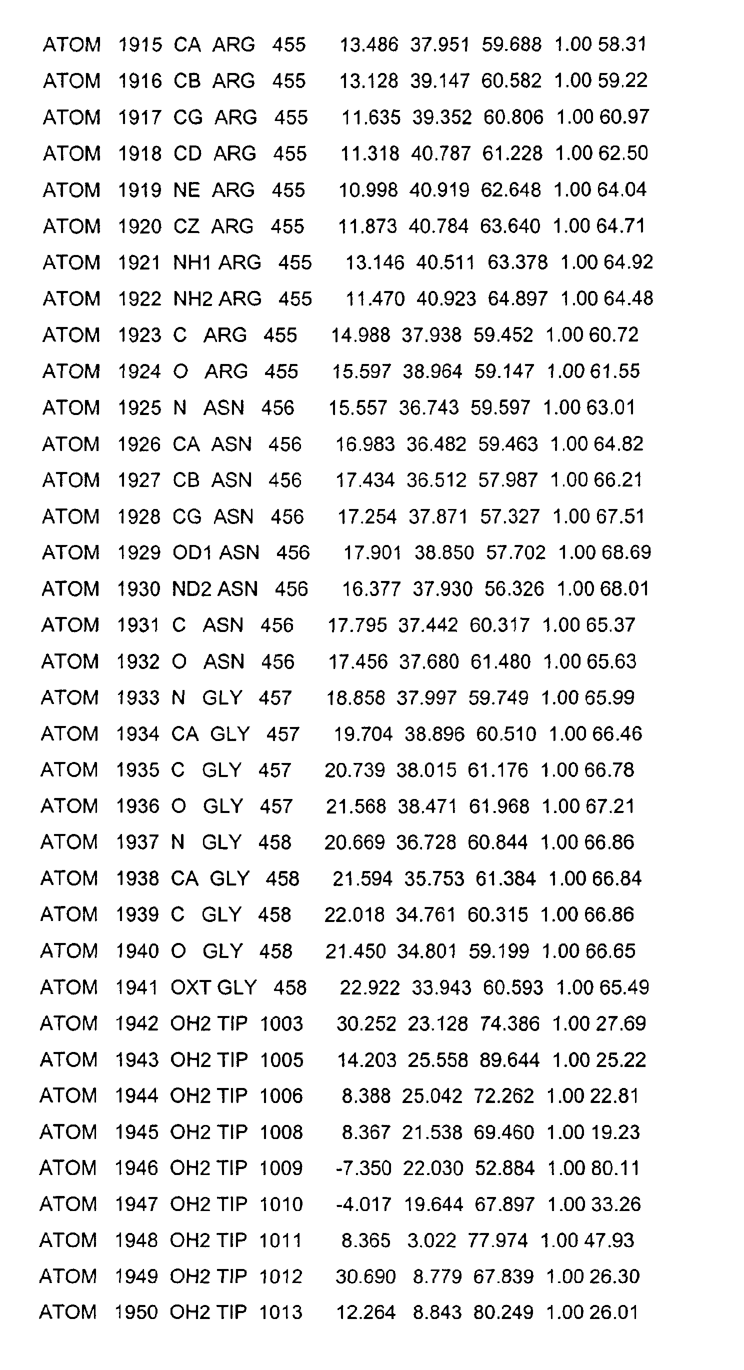

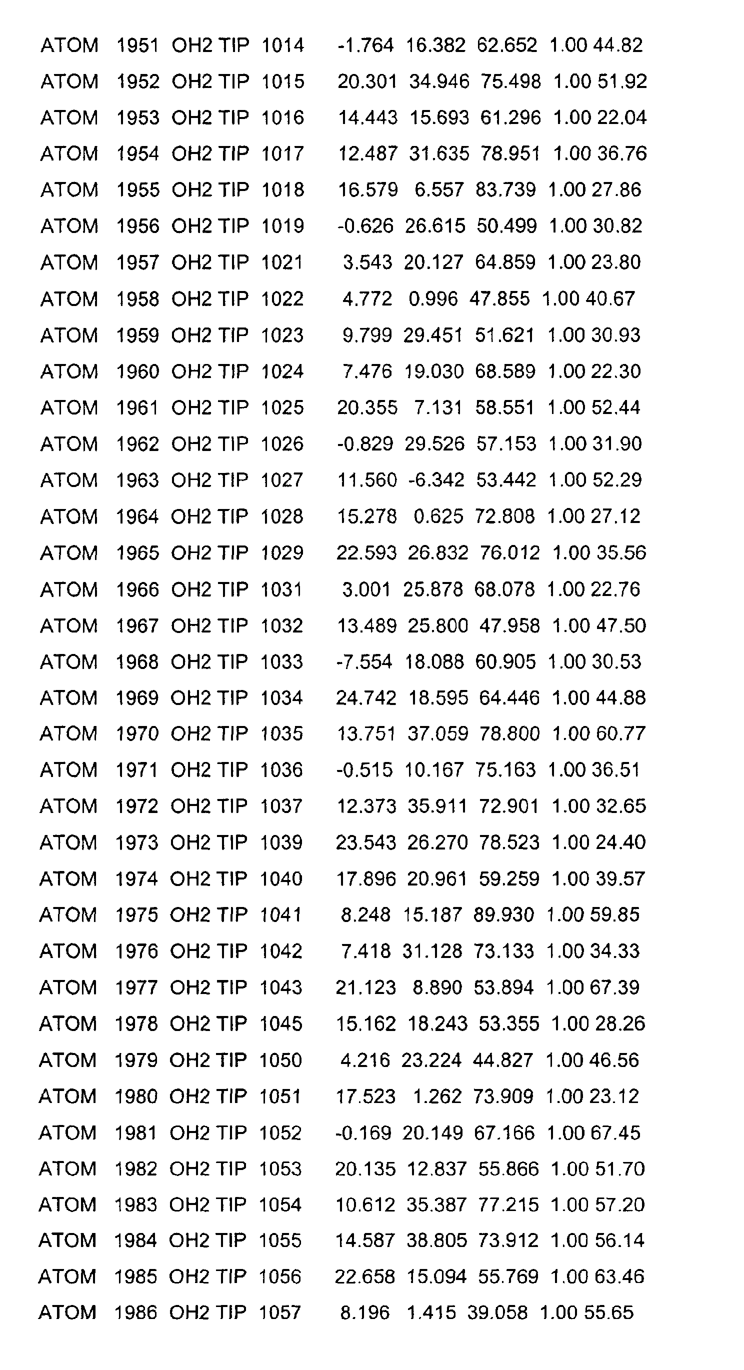

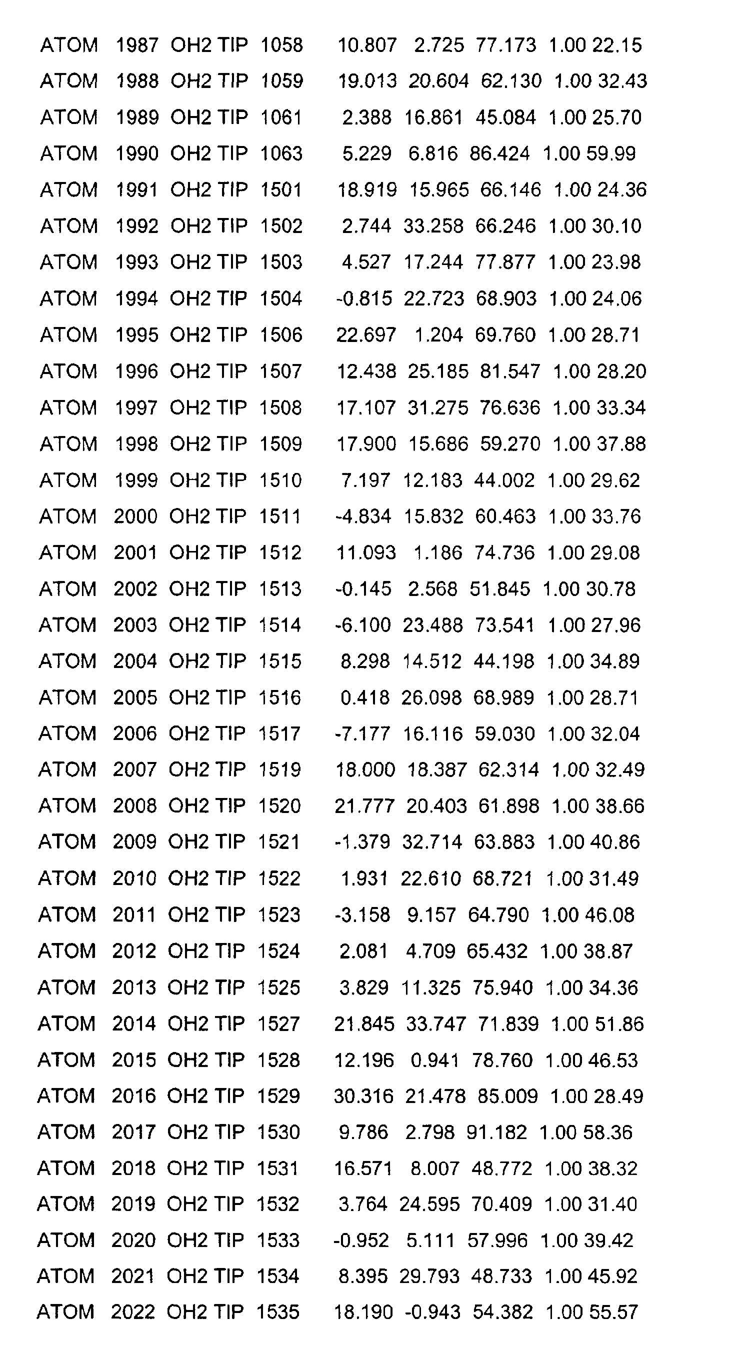

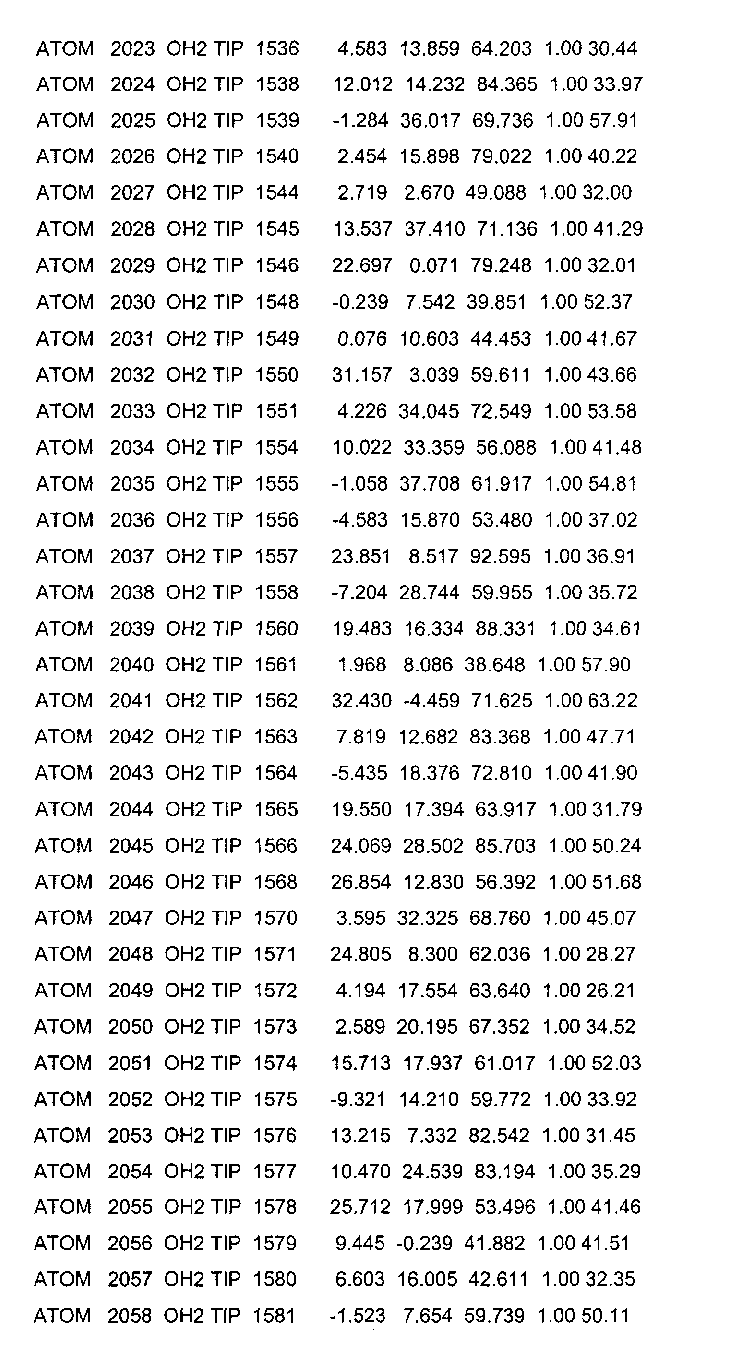

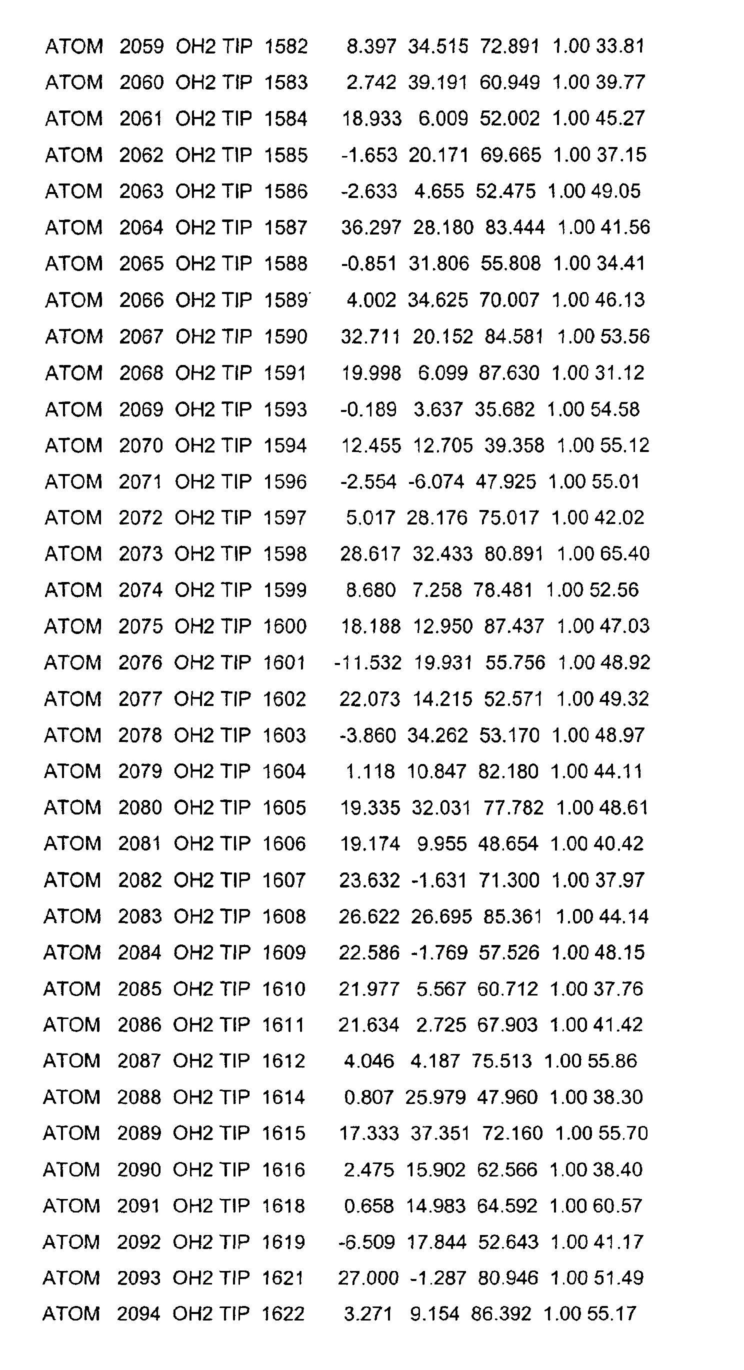

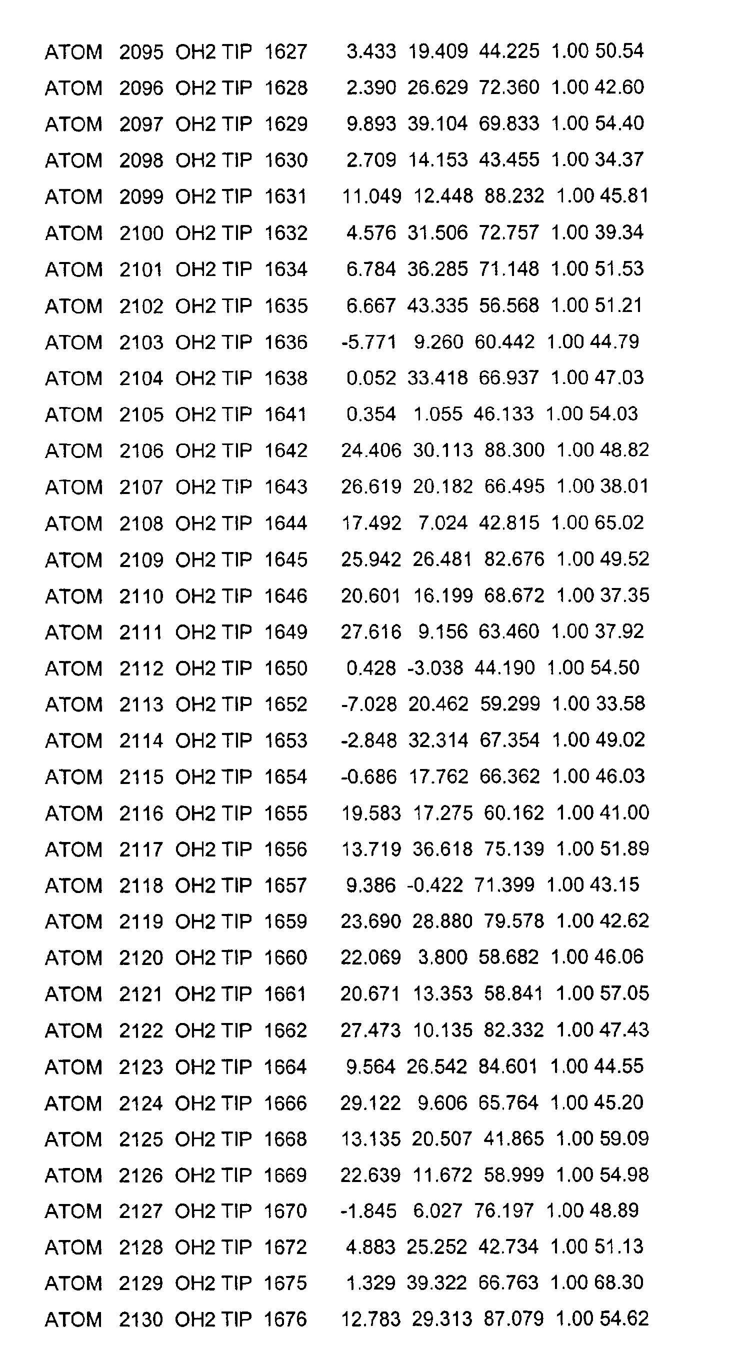

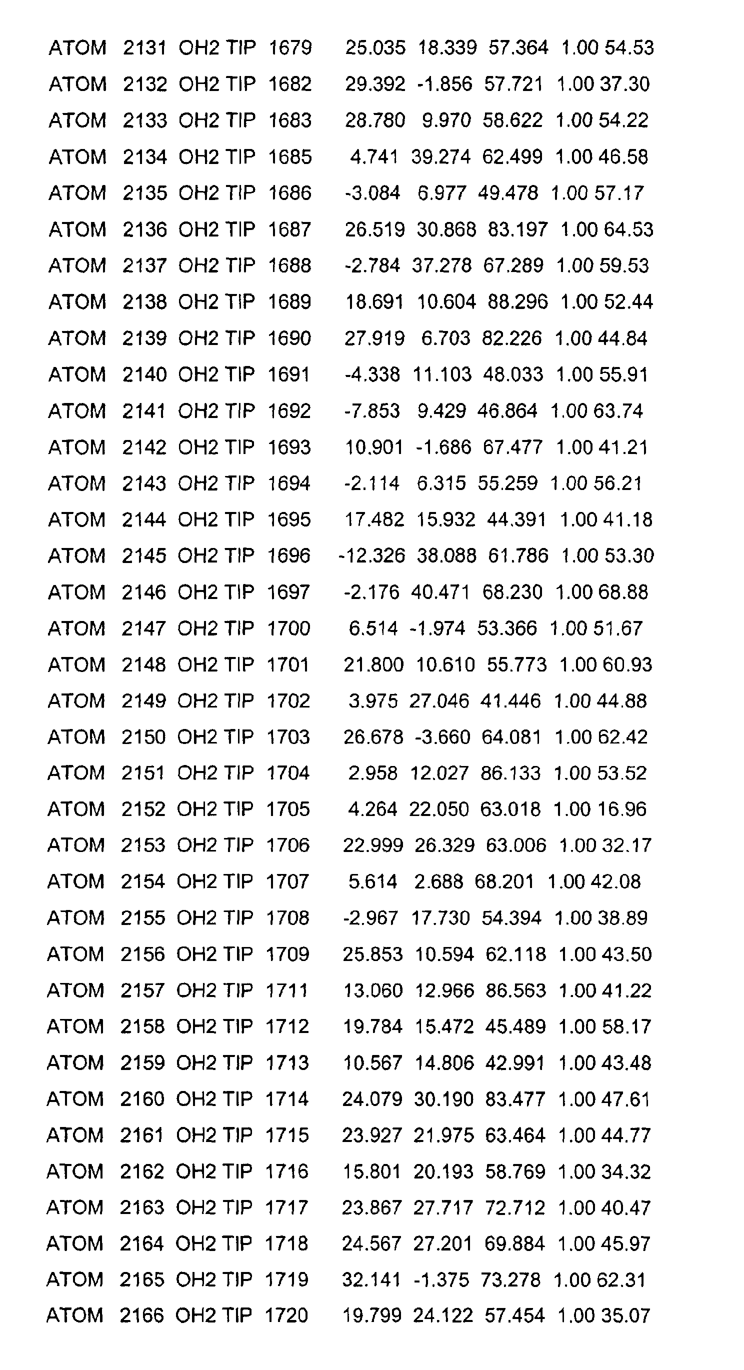

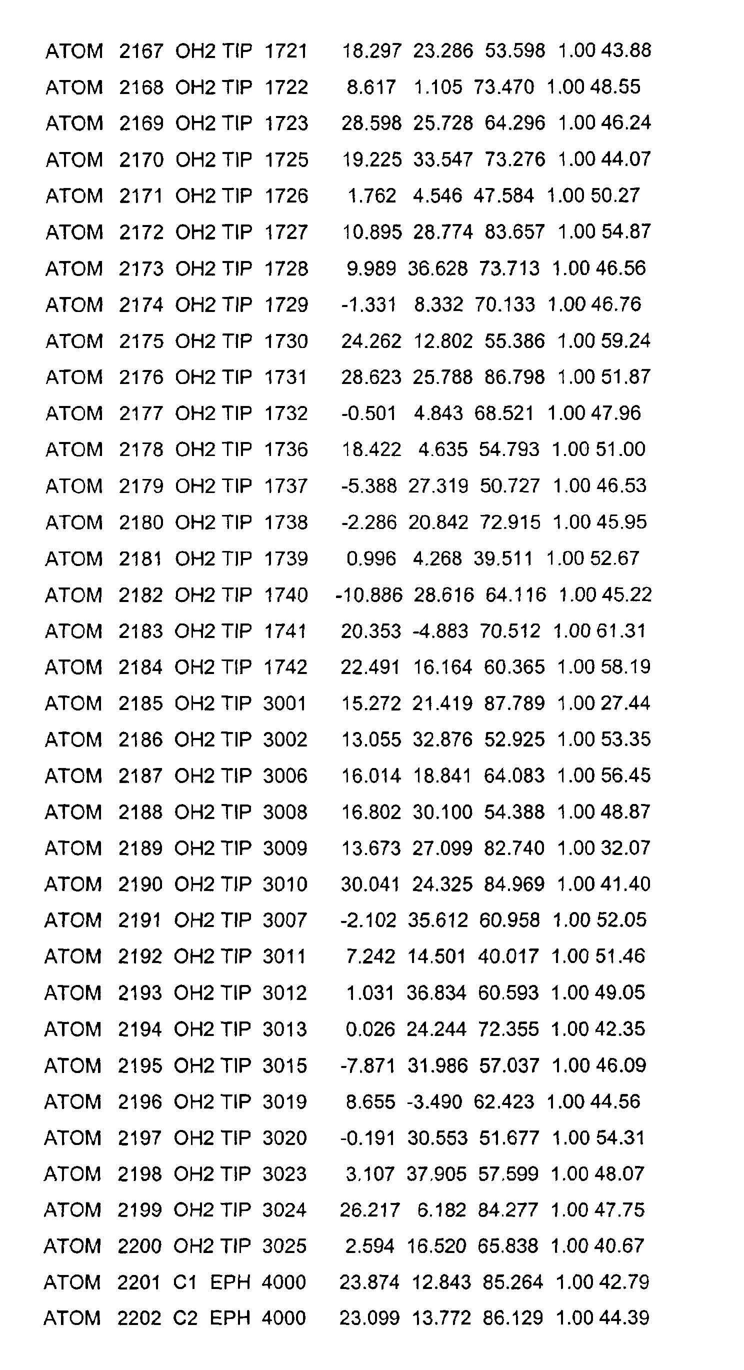

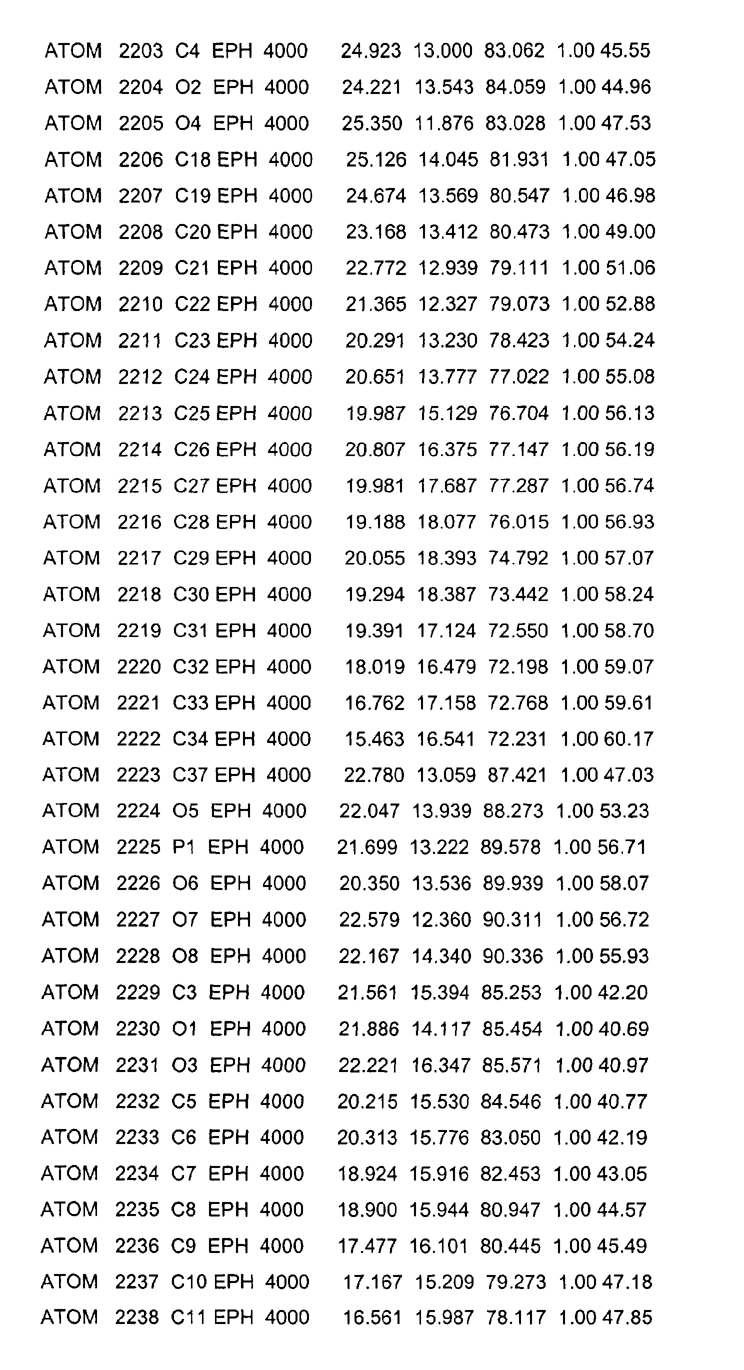

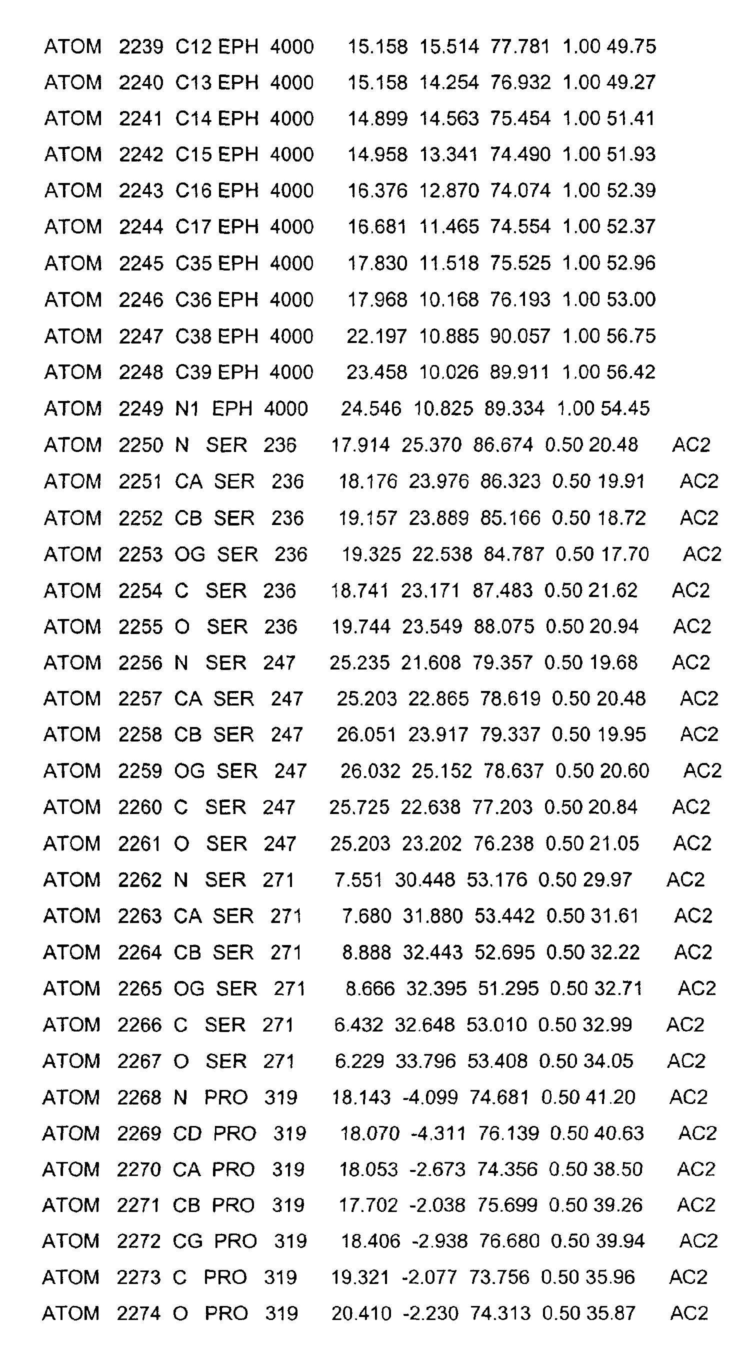

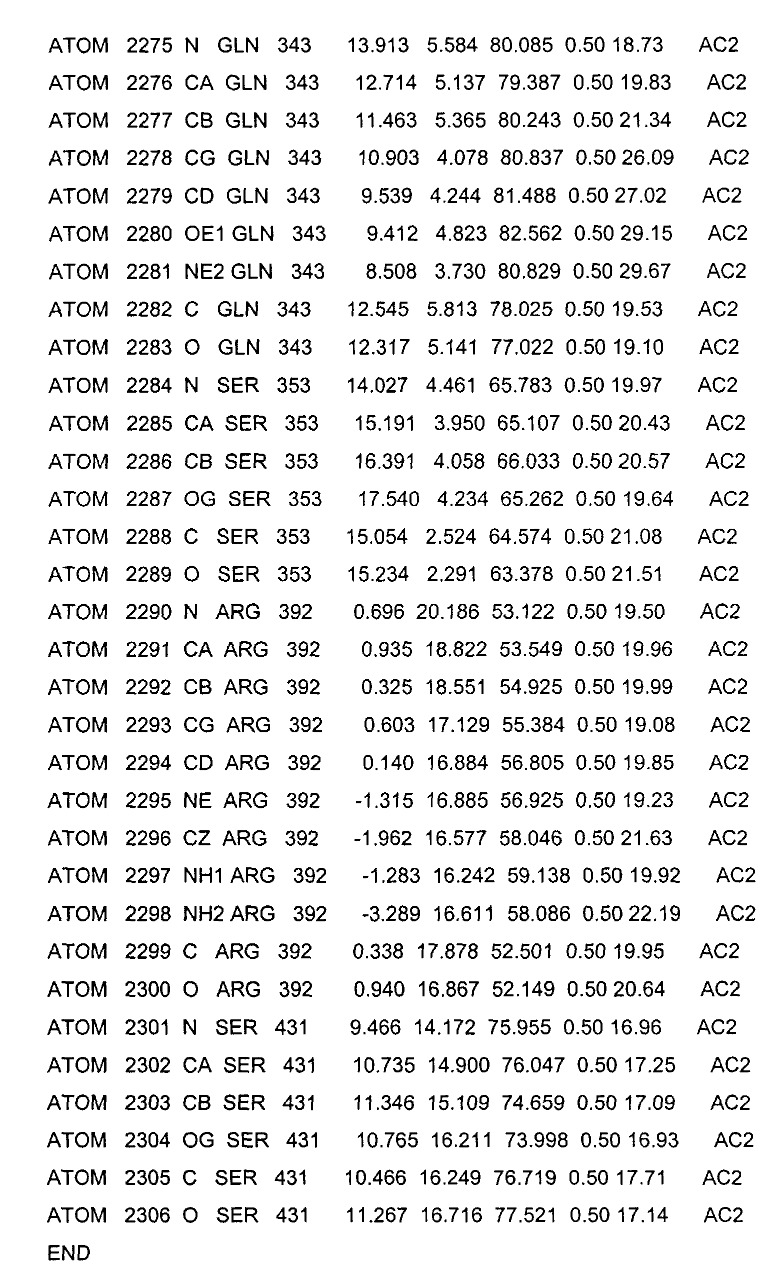

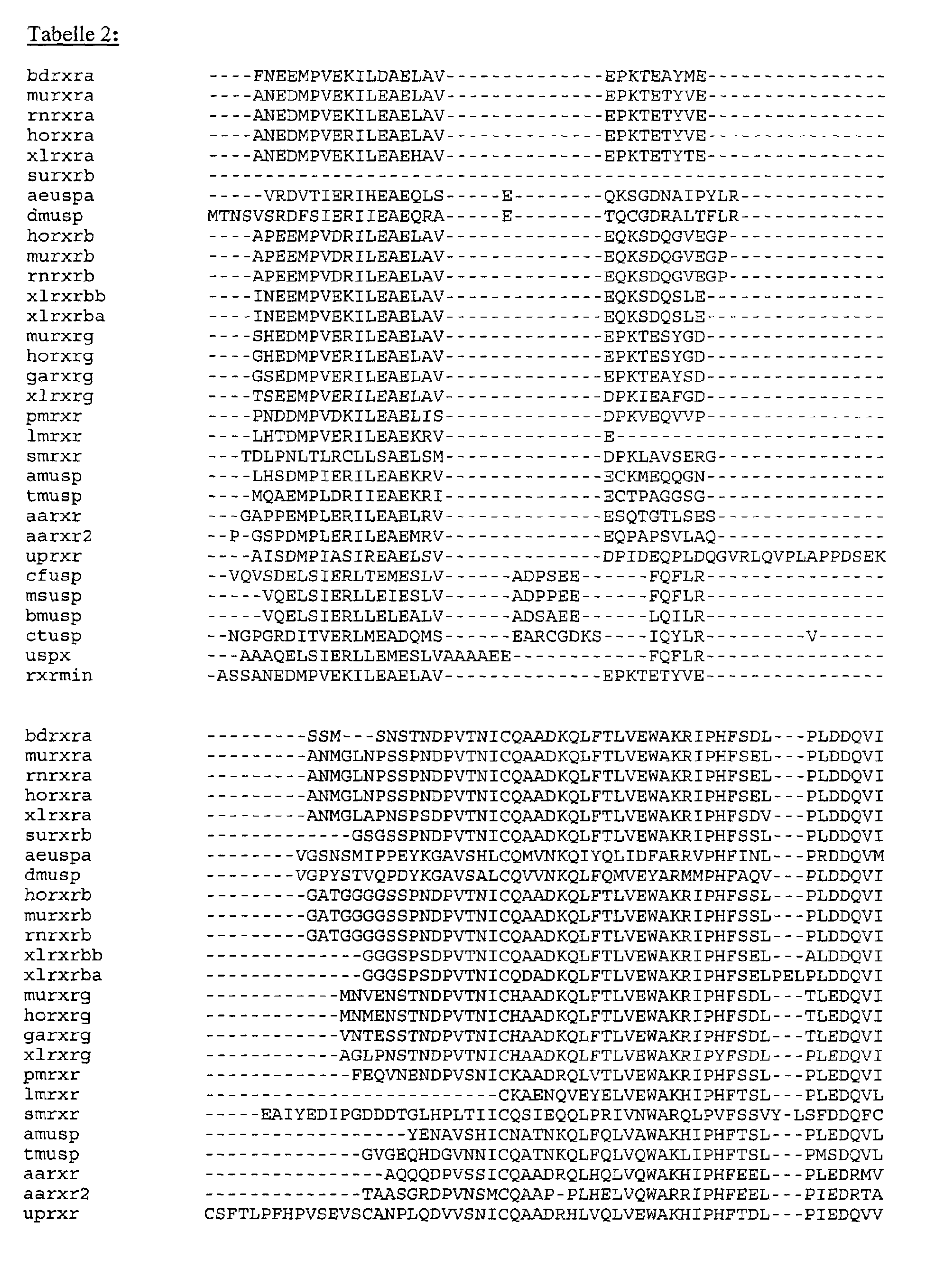

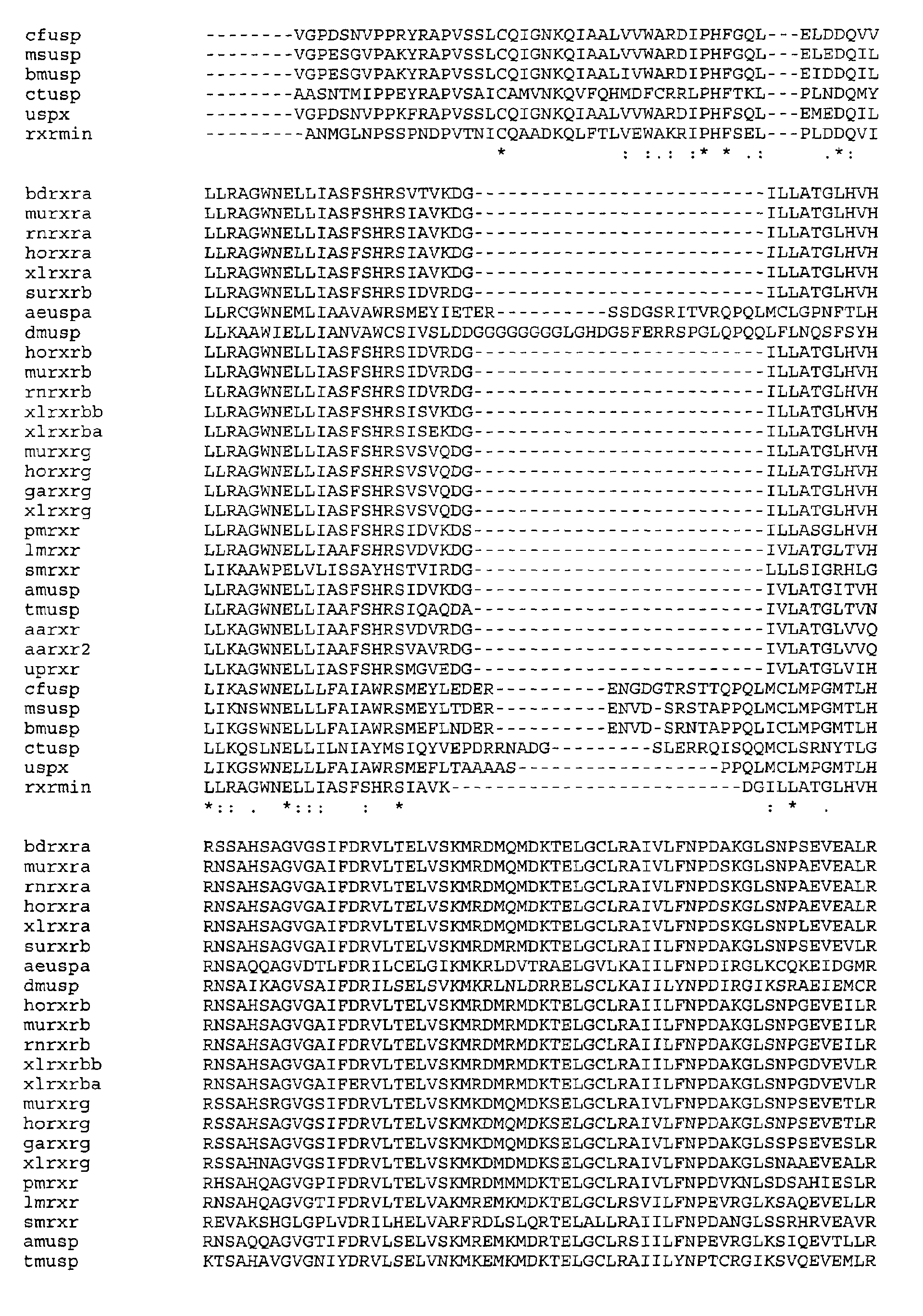

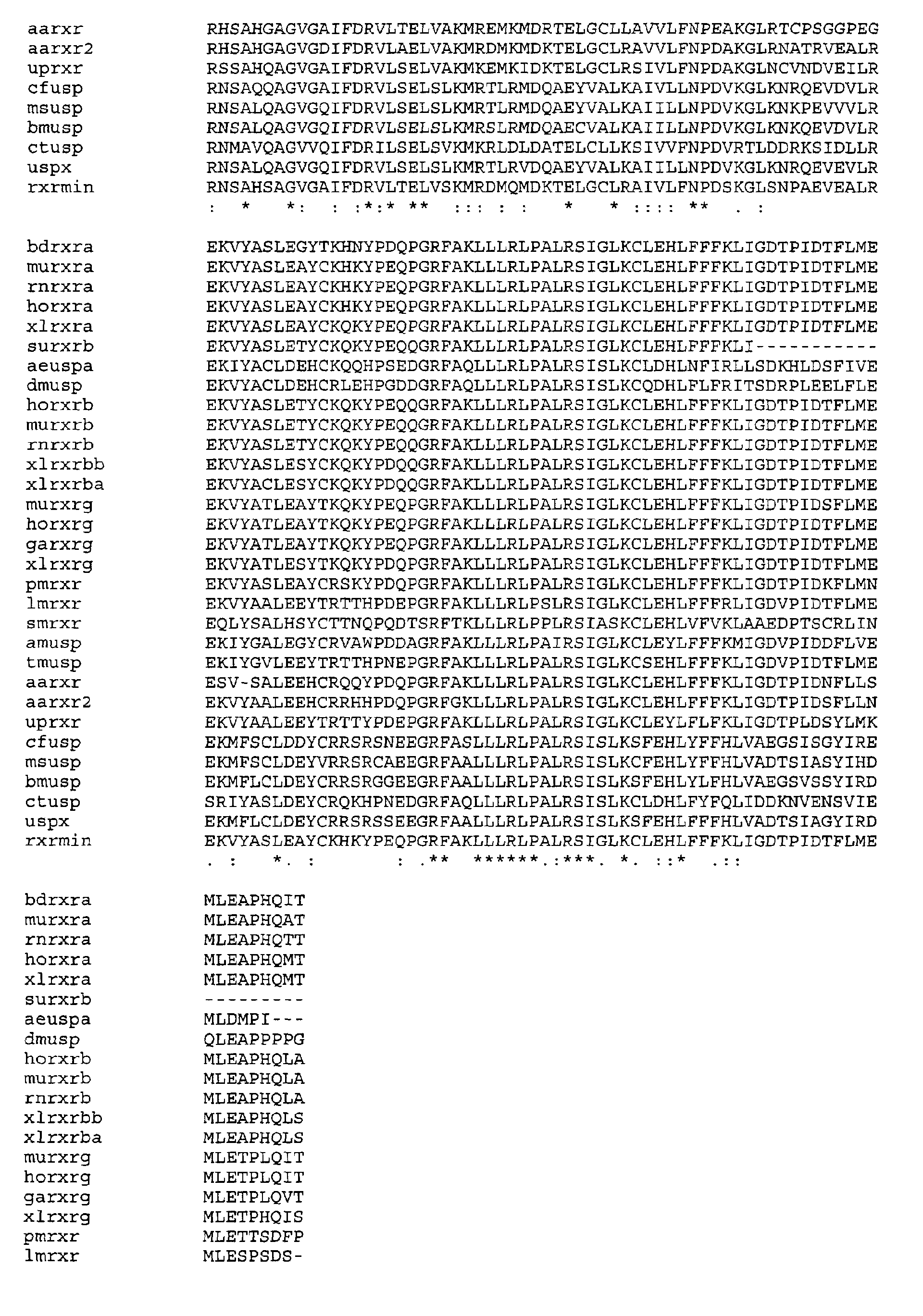

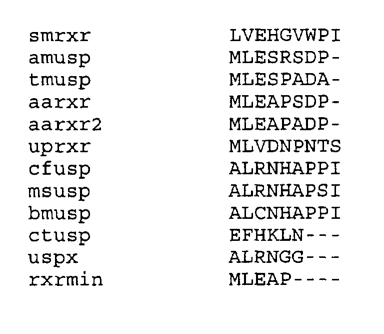

### Literatur

1. Oro A.E. et al. (1990): Relationship between the product of the Drosophila ultraspiracle locus and the vertebrate retinoid X receptor. Nature 347, 298-301
2. Moras D. & Gronemeyer H. (1998): The nuclear receptor ligand-binding domain: structure and fiction. Current Opinion in Cell Biology 10, 384-391
3. Segraves W.A. (1994): Steroid Receptors and Other Transcription Factors in Ecdysone Response. Recent Progress in Hormone Research, 49, 167-195
4. Henrich V.C. & Brown N.E. (1995): Insect Nuclear Receptors: A Developmental and Comparative Perspective. Insect Biochem. Mol. Biol. 25 (8), 881-897
5. Thummel C.S. (1995): From Embryogenesis to Metamorphosis: The Regulation and Function of Drosophila Nuclear Receptor Superfamily Members. Cell 83, 871-877
6. Truman J.W. (1996): Ecdysis Control Sheds Another Layer. Science 271, 40-41
7. Yao T. et al. (1993): Functional ecdysone receptor is the product of EcR and Ultraspiracle genes. Nature 366, 476-479
8. Hall B.L. & Thummel C.S. (1998): The RXR homolog Ultraspiracle is an essential component of the Drosophila ecdysone receptor. Development 125, 4709-4717
9. Lezzi M. et al. (1999): The Ecdysone Receptor Puzzle. Arch. Insect Biochem. Physiol. 41, 99-106
10. Mikitani K. (1996): Ecdysteroid Receptor Binding Activity and Ecdysteroid Agonist Activity at the Level of Gene Expression are Correlated with the Activity of Dibenzoyl Hydrazines in Larvae of Bombyx mori. J. Insect Physiol. 42 (10), 937-941
11. Dhadialla T.S. et al. (1998): New Insecticides with Ecdysteroidal and Juvenile Hormone Activity. Annu. Rev. Entomol. 43, 545-569
12. Sundaram M. et al. (1998): Basis for selective action of a synthetic molting hormone agonist, RH-5992 on lepidopteran insects. Insect Biochem. Mol. Biol. 28, 693-704
13. Rarey M. et al. (1996): Predicting Receptor-Ligand Interactions by an Incremental Construction Algorithm. J. Mol. Biol. 261(3), 470-489
14. Jones G. et al. (1997): Development and Validation of a Genetic Algorithm for Flexible Docking. J. Mol. Biol. 267 (3), 727-748
15. Böhm H.J. (1992): LUDI: Rule-Based Automatic Design of New Substituents for Enzyme Inhibitor Leads. J. Comp.-Aided Mol. Design 6, 593-606
16. Nishibata Y. & Itai A. (1991): Automatic creation of drug candidate structures based on receptor structure. Starting point for artificial lead generation. Tetrahedron 47, 8985-8990
17. Moon J.B. & Howe W.J. (1991): Computer design of bioactive molecules: a method for receptor-based de novo ligand design. Proteins 11 (4), 314-328
18. Otwinowski Z. & Minor W. (1997): Processing of X-ray diffraction data collected in oscillation mode. Methods Enzymol. 276, 307-326
19. Navaza J. (1994): Amore: an automated package for molecular replacement. Acta Cryst. A 50, 157-163
20. Egea P.F. et al. (2000): Crystal structure of the human RXRalpha ligand-binding domain bound to its natural ligand 9-cis retinoic acid. EMBO J. 19, 2592-2601
21. Jones T.A. et al. (1991): Improved methods for building protein models in electron density maps and the location of errors in these models. Acta Cryst. A 47, 110-119
22. Perrakis A. et al (1997): wARP: improvement and extension of crystallographic phases by weighted averaging of multiple-refined dummy atomic models. Acta Cryst. D 53, 448-455
23. Brünger A.T. et al. (1998): Crystallography & NMR System: A New Software Suite for Macromolecular Structure Determination. Acta Cryst. D 54, 905-921
24. Laskowski R.A. et al. (1993): PROCHECK:a programme to check the stereochemical quality of protein structure coordinates. J. Appl. Crystallogr. 26, 283-291

## Patentansprüche

1. Ligandenbindedomäne (LBD) des Ultraspiracle-Proteins (USP) in kristalliner Form.

2. LBD gemäß Anspruch 1, **dadurch gekennzeichnet, dass** es sich um die LBD des USP von Heliothis virescens handelt.

3. LBD gemäß Anspruch 1 oder 2, **dadurch gekennzeichnet, dass** sie die Aminosäuresequenz gemäß SEQ ID NO: 1 umfasst.

4. LBD gemäß einem der Ansprüche 1 bis 3, **dadurch gekennzeichnet, dass** sie im Komplex mit einem Liganden vorliegt.

5. LBD gemäß einem der Ansprüche 1 bis 4, **dadurch gekennzeichnet, dass** sie die in Tabelle 1 definierten Strukturkoordinaten aufweist.

6. LBD gemäß einem der Ansprüche 1 bis 5, umfassend eine Ligandenbindungstasche, die durch die Aminosäuren LEU230, VAL238, PRO239, PHE242, LEU249, LEU291, ILE294, MET323, LEU331, GLN338, ALA339, VAL341, PHE345, SER431, HIS434, LEU435, PHE438 und LEU440 gemäß SEQ ID NO: 2 und Tabelle 1 definiert ist.

7. LBD gemäß einem der Ansprüche 1 bis 5, umfassend eine Ligandenbindungstasche, die durch die Aminosäuren LEU230, VAL238, PRO239, PHE242, PRO245, VAL246, LEU249, CYS250, GLY253, ASN287, LEU290, LEU291, ILE294, MET323, LEU325, LEU331, SER335, ALA336, GLN338, ALA339, VAL341, ILE344, PHE345, VAL348, SER431, HIS434, LEU435, PHE438, LEU440 gemäß SEQ ID NO: 2 und Tabelle 1 definiert ist.

8. Computer-lesbares Datenspeichermedium umfassend ein Datenspeichermaterial, auf welchem die Strukturkoordinaten einer LBD gemäß einem der Ansprüche 1 bis 7 gespeichert sind.

9. Computer-lesbares Datenspeichermedium gemäß Anspruch 8 in einer Form, die es ermöglicht, eine dreidimensionale Abbildung einer LBD gemäß einem der Ansprüche 1 bis 7 auf einem Computer-Bildschirm zu erzeugen.

10. Verfahren zum Erstellen von Proteinmodellen von USP-LBDs, **gekennzeichnet durch** Computer-unterstütztes Erstellen einer dreidimensionalen Abbildung einer LBD gemäß einem der Ansprüche 1 bis 7.

11. Verfahren zum Erstellen von Proteinmodellen von USP-LBDs in einer agonistischen Konformation, **gekennzeichnet durch** Computer-unterstütztes Erstellen einer dreidimensionalen Abbildung einer LBD gemäß einem der Ansprüche 1 bis 7 in einer agonistischen Konformation.

12. Verfahren zum Erstellen von Proteinmodellen von Kernrezeptoren, die Homologien zu USP-LBDs aufweisen, **gekennzeichnet durch** Computer-unterstütztes Erstellen einer dreidimensionalen Abbildung einer LBD gemäß einem der Ansprüche 1 bis 7 mit einer mutierten Aminosäuresequenz.

13. Verfahren zum Erstellen von Proteinmodellen von Kernrezeptoren, die Homologien zu USP-LBDs aufweisen, in einer agonistischen Konformation, **gekennzeichnet durch** Computer-unterstütztes Erstellen einer dreidimensionalen Abbildung einer LBD gemäß einem der Ansprüche 1 bis 7 mit einer mutierten Aminosäuresequenz in einer agonistischen Konformation.

14. Verfahren zum Auffinden von Liganden des USP, **gekennzeichnet durch** die folgenden Schritte:
(c) Computer-unterstütztes Erstellen einer dreidimensionalen Abbildung einer LBD gemäß einem der Ansprüche 1 bis 7, und
(d) Computer-unterstütztes Durchsuchen (virtuelles Screenen) von Datenbanken, die Strukturdaten von chemischen Verbindungen enthalten, nach solchen Strukturen, die die Fähigkeit besitzen, spezifische Wechselwirkungen mit einer LBD gemäß einem der Ansprüche 1 bis 7 einzugehen.

15. Verfahren zum Auffinden von Liganden des USP, **gekennzeichnet durch** die folgenden Schritte:
(c) Computer-unterstütztes Erstellen einer dreidimensionalen Abbildung einer LBD gemäß einem der Ansprüche 1 bis 7, und
(d) Computer-unterstütztes Modellieren von chemischen Verbindungen mit Strukturen, die die Fähigkeit besitzen, spezifische Wechselwirkungen mit einer LBD gemäß einem der Ansprüche 1 bis 7 einzugehen.

16. Verfahren zum Auffinden von Liganden von USP-LBDs in einer agonistischen Konformation, **gekennzeichnet durch** die folgenden Schritte:
(c) Computer-unterstütztes Erstellen einer dreidimensionalen Abbildung einer LBD gemäß einem der Ansprüche 1 bis 7 in einer agonistischen Konformation, und
(d) Computer-unterstütztes Durchsuchen (virtuelles Screenen) von Datenbanken, die Strukturdaten von chemischen Verbindungen enthalten, nach solchen Strukturen, die die Fähigkeit besitzen, spezifische Wechselwirkungen mit einer LBD in einer agonistischen Konformation einzugehen.

17. Verfahren zum Auffinden von Liganden von USP-LBDs in einer agonistischen Konformation, **gekennzeichnet durch** die folgenden Schritte:
(c) Computer-unterstütztes Erstellen einer dreidimensionalen Abbildung einer LBD gemäß einem der Ansprüche 1 bis 7 in einer agonistischen Konformation, und
(d) Computer-unterstütztes Modellieren von chemischen Verbindungen mit Strukturen, die die Fähigkeit besitzen, spezifische Wechselwirkungen mit einer LBD in einer agonistischen Konformation einzugehen.

18. Verfahren zum Auffinden von Wirkstoffen für den Pflanzenschutz, insbesondere von chemischen Verbindungen, welche durch Bindung an eine LBD gemäß einem der Ansprüche 1 bis 7 zur Aktivierung oder Hemmung von USP führen, umfassend die folgenden Schritte:
(d) Durchführen des Verfahrens gemäß Anspruch 14 oder 15,
(e) Synthetisieren der als Liganden identifizierten Verbindung(en), und
(f) Detektieren der biologischen Aktivität der im Schritt (b) synthetisierten Verbindung durch Transaktivierungstests, Verdrängungstests oder Biotests.

19. Verfahren zum Auffinden von Wirkstoffen für den Pflanzenschutz, insbesondere von chemischen Verbindungen, welche durch Bindung an eine LBD gemäß einem der Ansprüche 1 bis 7 in einer agonistischen Konformation zur Aktivierung oder Hemmung von USP führen, umfassend die folgenden Schritte:
(d) Durchführen des Verfahrens gemäß Anspruch 16 oder 17,
(e) Synthetisieren der als Liganden identifizierten Verbindung(en), und
(f) Detektieren der biologischen Aktivität der im Schritt (b) synthetisierten Verbindung durch Transaktivierungstests, Verdrängungstests oder Biotests.

20. Verfahren zum Auffinden von Effektoren für Systeme zur induzierbaren Expression von Zielgenen mittels USP, umfassend die folgenden Schritte:
(e) Durchführen des Verfahrens gemäß Anspruch 14 oder 15,
(f) Synthetisieren der als Liganden identifizierten Verbindung(en),
(g) Applizieren einer im Schritt (b) synthetisierten Verbindung auf Wirtszellen oder Wirtsorganismen, welche ein auf USP basierendes Expressionssystem enthalten, und
(h) Detektieren einer Induktion oder Hemmung des Expressionssystems.

21. Verwendung einer LBD gemäß einem der Ansprüche 1 bis 7 oder eines Computer-lesbares Datenspeichermediums gemäß Anspruch 8 oder 9 zum Auffinden von Wirkstoffen für den Pflanzenschutz oder von Effektoren zur kontrollierten Expression von Zielgenen in Wirtszellen oder ganzen Wirtsorganismen.


SEQUENZPROTOKOLL

<110> Bayer Aktiengesellschaft

<120> Ligandenbindedomäne des Ultraspiracle (USP)-Proteins

<130> Le A 34 772

<140>
<141>

<150> DE 100 36 461.6
<151> 2000-07-25

<160> 2

<170> PatentIn Ver. 2.1

<210> 1
<211> 262
<212> PRT
<213> Heliothis virescens

<400> 1
Val Gln Glu Leu Ser Ile Glu Arg Leu Leu Glu Met Glu Ser Leu Val
  1               5                  10                  15

Ala Asp Pro Ser Glu Glu Phe Gln Phe Leu Arg Val Gly Pro Asp Ser
             20                  25                  30

Asn Val Pro Pro Lys Phe Arg Ala Pro Val Ser Ser Leu Cys Gln Ile
         35                  40                  45

Gly Asn Lys Gln Ile Ala Ala Leu Val Val Trp Ala Arg Asp Ile Pro
     50                  55                  60

His Phe Ser Gln Leu Glu Met Glu Asp Gln Ile Leu Leu Ile Lys Gly
 65                  70                  75                  80

Ser Trp Asn Glu Leu Leu Leu Phe Ala Ile Ala Trp Arg Ser Met Glu
                 85                  90                  95

Phe Leu Thr Glu Glu Arg Asp Gly Val Asp Gly Thr Gly Asn Arg Thr
            100                 105                 110

Thr Ser Pro Pro Gln Leu Met Cys Leu Met Pro Gly Met Thr Leu His
        115                 120                 125

Arg Asn Ser Ala Leu Gln Ala Gly Val Gly Gln Ile Phe Asp Arg Val
    130                 135                 140

Leu Ser Glu Leu Ser Leu Lys Met Arg Thr Leu Arg Val Asp Gln Ala
145                 150                 155                 160

Glu Tyr Val Ala Leu Lys Ala Ile Ile Leu Leu Asn Pro Asp Val Lys
                165                 170                 175

Gly Leu Lys Asn Arg Gln Glu Val Glu Val Leu Arg Glu Lys Met Phe
            180                 185                 190

Leu Cys Leu Asp Glu Tyr Cys Arg Arg Ser Arg Ser Ser Glu Glu Gly
        195                 200                 205

Arg Phe Ala Ala Leu Leu Leu Arg Leu Pro Ala Leu Arg Ser Ile Ser
    210                 215                 220

Leu Lys Ser Phe Glu His Leu Phe Phe Phe His Leu Val Ala Asp Thr
225                 230                 235                 240

Ser Ile Ala Gly Tyr Ile Arg Asp Ala Leu Arg Asn His Ala Pro Pro
                245                 250                 255

Ile Asp Thr Asn Met Met
            260

<210> 2
<211> 466
<212> PRT
<213> Heliothis virescens

<400> 2
Met Ser Val Ala Lys Lys Asp Lys Pro Thr Met Ser Val Thr Ala Leu
  1               5                  10                  15

Ile Asn Trp Ala Arg Pro Leu Pro Pro Gly Gln Gln Gln Gln Pro Met
             20                  25                  30

Thr Pro Thr Ser Pro Gly Asn Met Leu Gln Pro Met Ala Thr Pro Ser
         35                  40                  45

Asn Leu Pro Thr Val Asp Cys Ser Leu Asp Ile Gln Trp Leu Asn Leu
     50                  55                  60

Glu Gly Gly Phe Met Ser Pro Met Ser Pro Pro Glu Met Lys Pro Asp

65                  70                  75                  80

Thr Ala Met Leu Asp Gly Leu Arg Asp Asp Ser Thr Pro Pro Pro Ala
                85                  90                  95

Phe Lys Asn Tyr Pro Pro Asn His Pro Leu Ser Gly Ser Lys His Leu
            100                 105                 110

Cys Ser Ile Cys Gly Asp Arg Ala Ser Gly Lys His Tyr Gly Val Tyr
        115                 120                 125

Ser Cys Glu Gly Cys Lys Gly Phe Phe Lys Arg Thr Val Arg Lys Asp
    130                 135                 140

Leu Thr Tyr Ala Cys Arg Glu Glu Arg Asn Cys Ile Ile Asp Lys Arg
145                 150                 155                 160

Gln Arg Asn Arg Cys Gln Tyr Cys Arg Tyr Gln Lys Cys Leu Ala Cys
                165                 170                 175

Gly Met Lys Arg Glu Ala Val Gln Glu Glu Arg Gln Arg Ala Ala Arg
            180                 185                 190

Gly Thr Glu Asp Ala His Pro Ser Ser Ser Val Gln Val Gln Glu Leu
        195                 200                 205

Ser Ile Glu Arg Leu Leu Glu Met Glu Ser Leu Val Ala Asp Pro Ser
    210                 215                 220

Glu Glu Phe Gln Phe Leu Arg Val Gly Pro Asp Ser Asn Val Pro Pro
225                 230                 235                 240

Lys Phe Arg Ala Pro Val Ser Ser Leu Cys Gln Ile Gly Asn Lys Gln
                245                 250                 255

Ile Ala Ala Leu Val Val Trp Ala Arg Asp Ile Pro His Phe Ser Gln
            260                 265                 270

Leu Glu Met Glu Asp Gln Ile Leu Leu Ile Lys Gly Ser Trp Asn Glu
        275                 280                 285

Leu Leu Leu Phe Ala Ile Ala Trp Arg Ser Met Glu Phe Leu Thr Glu
    290                 295                 300

Glu Arg Asp Gly Val Asp Gly Thr Gly Asn Arg Thr Thr Ser Pro Pro
305                 310                 315                 320

Gln Leu Met Cys Leu Met Pro Gly Met Thr Leu His Arg Asn Ser Ala

                325                 330                 335

Leu Gln Ala Gly Val Gly Gln Ile Phe Asp Arg Val Leu Ser Glu Leu
            340                 345                 350

Ser Leu Lys Met Arg Thr Leu Arg Val Asp Gln Ala Glu Tyr Val Ala
        355                 360                 365

Leu Lys Ala Ile Ile Leu Leu Asn Pro Asp Val Lys Gly Leu Lys Asn
    370                 375                 380

Arg Gln Glu Val Glu Val Leu Arg Glu Lys Met Phe Leu Cys Leu Asp
385                 390                 395                 400

Glu Tyr Cys Arg Arg Ser Arg Ser Ser Glu Glu Gly Arg Phe Ala Ala
                405                 410                 415

Leu Leu Leu Arg Leu Pro Ala Leu Arg Ser Ile Ser Leu Lys Ser Phe
            420                 425                 430

Glu His Leu Phe Phe Phe His Leu Val Ala Asp Thr Ser Ile Ala Gly
        435                 440                 445

Tyr Ile Arg Asp Ala Leu Arg Asn His Ala Pro Pro Ile Asp Thr Asn
    450                 455                 460

Met Met
465